(19) Europäisches Patentamt
European Patent Office
Office européen des brevets

(11) Veröffentlichungsnummer: **0 310 676 A1**

(12) **EUROPÄISCHE PATENT ANMELDUNG**
Veröffentlicht nach Art. 158 Abs. 3 EPÜ

(21) Anmeldenummer: **88903125.8**

(22) Anmeldetag: **30.12.87**

Daten der zugrundeliegenden internationalen Anmeldung:

(86) Internationale Anmeldenummer:
**PCT/SU87/00161**

(87) Internationale Veröffentlichungsnummer:
**WO88/07992 (20.10.88 8/23)**

(51) Int.Cl.³: **C 07 D 213/57**
C 07 D 213/16, C 07 D 213/24
C 09 K 19/34, C 09 K 19/42

(30) Priorität: **07.04.87 SU 4234347**
**07.04.87 SU 4234348**
**07.07.87 SU 4280285**

(43) Veröffentlichungstag der Anmeldung:
**12.04.89 Patentblatt 89.15**

(84) Benannte Vertragsstaaten:
**DE FR GB IT NL**

(71) Anmelder: **PAVLJUCHENKO, Assya Iosifovna**
**pr. Marshala Zhukova, 62-38**
**Moscow, 123448(SU)**

(71) Anmelder: **PURVANYATSKAS, Gediminas Vladovich**
**ul. Partinazu, 24**
**Vilnjus, 232006(SU)**

(71) Anmelder: **SMIRNOVA, Natalya Ivanovna**
**Likhachevskoe shosse, 13-1-89 Moskovskaya obl.**
**Dolgoprudny, 142700(SU)**

(71) Anmelder: **GREBENKIN, Mikhail Fedorovich**
**ul. Linia Oktyabrskoi zheleznoi dorogi, 10-79**
**Moscow, 127238(SU)**

(71) Anmelder: **PETROV, Vladimir Fedorovich**
**ul. Vasilievskaya, 3-66**
**Moscow, 123056(SU)**

(71) Anmelder: **BARNIK, Mikhail Ivanovich**
**Likhachevskoe shosse, 20-212 Moskovskaya obl.**
**Dolgoprudny, 141700(SU)**

(71) Anmelder: **IVASCHENKO, Alexandr Vasilievich**
**ul. Pervomaiskaya, 44a-57 Moskovskaya obl.**
**Dolgoprudny, 141700(SU)**

(71) Anmelder: **KOROTKOVA, Natalya Ivanovna**
**Likhachevskoe shosse, 20-3-16 Moskovskaya obl.**
**Dolgoprudny, 141700(SU)**

(54) **FLUSSIGKRISTALLDERIVATE VON 2,5-DISUBSTITUIERTEN PYRIDINEN ALS KOMPONENTEN VON FLUSSIGKRISTALLMATERIALEN UND FLUSSIGKRISTALLMATERIAL**

(57) Flüssigkristalline Derivate von 2,5-disubstituiertem Pyridin weisen die allgemeine Formel

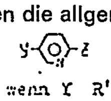

auf, worin wenn Y R' 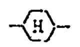 ist, Z für CN,

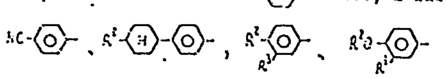

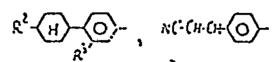

steht, wenn Y $R^1$ ist, Z für 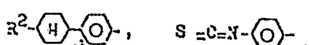

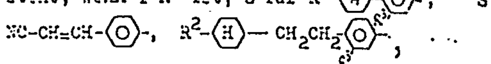

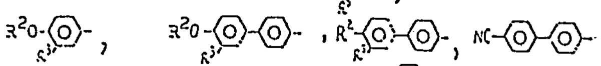

steht, wenn Y CH ist, Z für R' 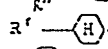 steht wenn Y 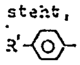 ist, Z für 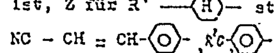

steht, wenn y 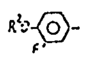 ist Z für 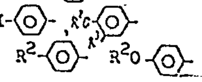 steht,

$R^1$, $R^2$ eine Alkylgruppe mit 1 bis 12 Kohlenstoffatomen darstellen, $R^3$ F, $NO_2$, $NH_2$, CN bedeutet.

Flüssigkristallines Material, enthaltend zumindest zwei flüssigkristalline Komponenten, von denen mindestens die eine ein flüssigkristallines Derivat von 2,5-disubstituiertem Pyridin der genannten allgemeinen Formel darstellt, das in einer Menge von 1 bis 75 Masse% genommen wird.

./...

EP 0 310 676 A1

(71) Anmelder: KOVSHEV, Evgeny Ivanovich
ul. Kirova, 12-15 Moskovskaya obl.
Khimki, 141400(SU)

(71) Anmelder: RABINOVICH, Arnold Zinovievich
ul. Gastello, 8-9
Moscow, 107014(SU)

(71) Anmelder: BUMAGIN, Nikolai Alexandrovich
ul. Tomilinskaya, 22-113 Moskovskaya obl.
Dzerzhinsky, 140056(SU)

(71) Anmelder: ANDRJUKHOVA, Nonna Petrovna
Leninskie gory, MGU, zona 'V'-1108
Moscow, 117234(SU)

(71) Anmelder: BELETSKAYA, Irina Petrovna
ul. Profsojuznaya, 43-1-212
Moscow, 117420(SU)

(71) Anmelder: MALTSEV, Sergei Dimitrievich
ul. Patsaeva, 164-308 Moskovskaya obl.
Dolgoprudny, 141700(SU)

(71) Anmelder: TITOV, Viktor Vasilievich
ul. Festivalnaya, 12-95
Moscow, 125581(SU)

(72) Erfinder: PAVLJUCHENKO, Assya Iosifovna
pr. Marshala Zhukova, 62-38
Moscow, 123448(SU)

(72) Erfinder: PURVANYATSKAS, Gediminas Vladovich
ul. Partinazu, 24
Vilnjus, 232006(SU)

(72) Erfinder: SMIRNOVA, Natalya Ivanovna
Likhachevskoe shosse, 13-1-89 Moskovskaya obl.
Dolgoprudny, 142700(SU)

(72) Erfinder: GREBENKIN, Mikhail Fedorovich
ul. Linia Oktyabrskoi zheleznoi dorogi, 10-79
Moscow, 127238(SU)

(72) Erfinder: PETROV, Vladimir Fedorovich
ul. Vasilievskaya, 3-66
Moscow, 123056(SU)

(72) Erfinder: BARNIK, Mikhail Ivanovich
Likhachevskoe shosse, 20-212 Moskovskaya obl.
Dolgoprudny, 141700(SU)

(72) Erfinder: IVASCHENKO, Alexandr Vasilievich
ul. Pervomaiskaya, 44a-57 Moskovskaya obl.
Dolgoprudny, 141700(SU)

(72) Erfinder: KOROTKOVA, Natalya Ivanovna
Likhachevskoe shosse, 20-3-16 Moskovskaya obl.
Dolgoprudny, 141700(SU)

(72) Erfinder: KOVSHEV, Evgeny Ivanovich
ul. Kirova, 12-15 Moskovskaya obl.
Khimki, 141400(SU)

(72) Erfinder: RABINOVICH, Arnold Zinovievich
ul. Gastello, 8-9
Moscow, 107014(SU)

(72) Erfinder: BUMAGIN, Nikolai Alexandrovich
ul. Tomilinskaya, 22-113 Moskovskaya obl.
Dzerzhinsky, 140056(SU)

(72) Erfinder: ANDRJUKHOVA, Nonna Petrovna
Leninskie gory, MGU, zona 'V'-1108
Moscow, 117234(SU)

(72) Erfinder: BELETSKAYA, Irina Petrovna
ul. Profsojuznaya, 43-1-212
Moscow, 117420(SU)

(72) Erfinder: MALTSEV, Sergei Dimitrievich
ul. Patsaeva, 164-308 Moskovskaya obl.
Dolgoprudny, 141700(SU)

(72) Erfinder: TITOV, Viktor Vasilievich
ul. Festivalnaya, 12-95
Moscow, 125581(SU)

(74) Vertreter: von Füner, Alexander, Dr.
Patentanwälte v. Füner, Ebbinghaus, Finck Mariahilfplatz 2&3
D-8000 München 90(DE)

# FLÜSSIGKRISTALLINE DERIVATE VON 2,5-DISUBSTITUIERTEM PYRIDIN ALS KOMPONENTEN EINES FLÜSSIGKRISTALLINEN MATERIALS UND FLÜSSIGKRISTALLINES MATERIAL

## Gebiet der Technik

Die vorliegende Erfindung bezieht sich auf neue organische Verbindungen, die flüssigkristalline Eigenschaften aufweisen, und betrifft sie insbesondere flüssigkristalline Derivate von 2,5-disubstituiertem Pyridin als Komponente für ein flüssigkristallines Material sowie ein in elektrooptischen Einrichtungen verwendbares flüssigkristallines Material.

Die vorgeschlagene Erfindung wird in der Elektronik zur alphanumerischen Informationsdarstellung zum Beispiel in elektronischen Uhren, Mikrokalkulatoren weitgehend Verwendung finden.

## Zugrundeliegender Stand der Technik

Zur Zeit bekannt sind flüssigkristalline Verbindungen -trans-4-Alkyl-(4-zyanophenyl)-zyklohexane der Formel

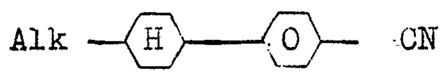

worin der Alkylteil 3 bis 8 Kohlenstoffatome enthält, die als Komponenten der flüssigkristallinen Materialien eingesetzt werden,(DE-C 2636684).

Sie zeichnen sich durch einen recht breiten Bereich der nematischen Phase aus, jedoch weisen sie geringe Anisotropien-Werte der Dielektrizitätskonstante $\Delta\varepsilon$ was zu hohen Werten der Schwellspannung und der Sättigungsspannung beim Twist-Effekt des flüssigkristallinen Materials, das diese Verbindungen aufweist führt.

Bekannt sind flüssigkristalline Verbindungen 2-(4-Zyanophenyl)-5-(4-alkylphenyl)-pyridine der Formel

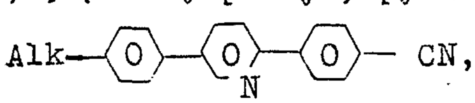

worin der Alkylteil 4 bis 8 Kohlenstoffatome enthält (SU, A, 713153), und 6-(4-Alkylphenyl)-3-zyanopyridine der Formel

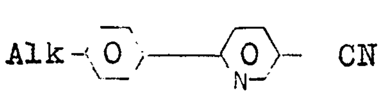

darstellen, worin der Alkylteil 4 bis 8 Kohlenstoffatome enthält (SU, A, 666797) als Komponenten der flüssigkristallinen Materialien.

Diese Verbindungen besitzen hohe Koeffizienten der Rotationsviskosität innerhalb eines breiten Bereichs der nematischen Phase. Wegen solcher Viskosität ist die Verwendung der diese Verbindungen einschließenden Materialien in elektrooptischen Einrichtungen von hoher Schnellwirkung beschränkt.

Bekannt sind flüssigkristalline Verbindungen, 5-Alkyl-2-(4-zyanophenyl)-pyridine der Formel

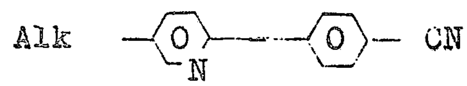

worin der Alkylteil 2 bis 7 Kohlenstoffatome enthält, (SU, A, 675800) als Komponenten der flüssigkristallinen Materialen Diese Verbindungen haben neben hohen Anisotropien der Dielektrizitätskonstante niedrige Klärpunkte und schmale Bereiche der nematischen Phase. Die genannten Eigenschaften derselben verhindern die Verwendung der diese Verbindungen enthaltenden flüssigkristallinen Materialien im breiten Arbeitstemperaturbereich.

Bekannt sind ebenfalls Verbindungen 4-(trans-4-Alkylzyklohexyl)-4'-(trans-4-alkylzyklohexyl)biphenyle der Formel

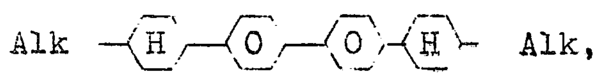

worin der Alkylteil 1 bis 6 Kohlenstoffatome enthält (SU, A, 1055336), und 2- [4-(trans-4-Alkylzyklohexyl)-phenyl]-5-alkylpyridine der Formel

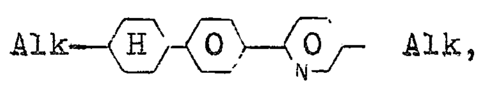

worin der Alkylteil 2 bis 6 Kohlenstoffatome (SU, A, 1302684) enthält, als Komponenten der flüssigkristallinen Materialien.

Diese Verbindungen weisen hohe Klärpunkte auf.

Diese Verbindungen zeigen hohe Temperaturwerte beim Übergang "smektisch-nematisch", wodurch die Verwendung der flüssigkristallinen Materialien, die diese Verbindungen einschließen, im Bereich negativer Temperaturen beschränkt wird.

Offenbarung der Erfindung

Der Erfindung liegt die Aufgabe zugrunde, neue flüssigkristalline Derivate von 2,5-disubstituiertem Pyridin als Komponente eines flüssigkristallinen Materials zu entwickeln, die geringe Werte der Rotationsviskosität, der Temperatur des Übergangs "smektisch-nematisch", hohe Werte der Anisotropie der Dielektrizitätskonstante des Klärpunkts aufweisen, sowie eine flüssigkristallines Material zu entwickeln, das einen breiten Bereich der nematischen Phase hat und geringe Werte der Schwellspannung und der Sättigungsspannung beim Twist-Effekt und kleine Schalt- und Ausschaltzeiten aufweist.

Die Aufgabe wird dadurch gelöst, daß neue flüssigkristalline Derivate von 2,5-disubstituiertem Pyridin der Formel

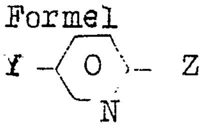

worin, wenn Y 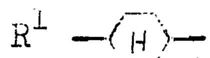 ist, Z für CN

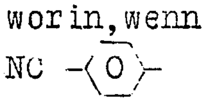 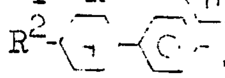, 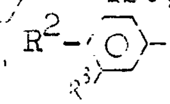 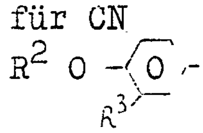 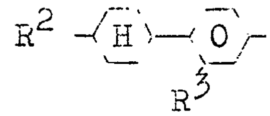

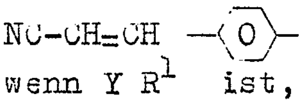 steht,

wenn Y $R^1$ ist, Z 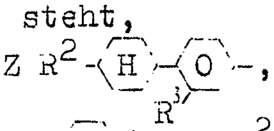, 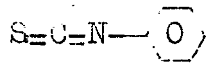, 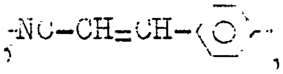,

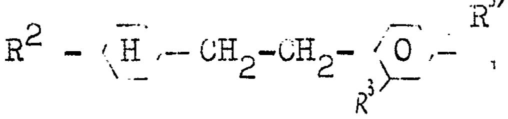, 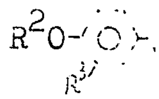, 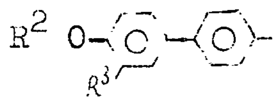

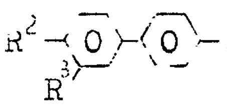, 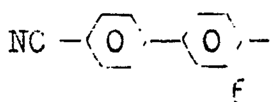 ist, wenn Y - CN ist,

Z R'- H - ist, wenn 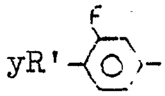, Z für 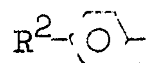, 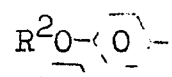 steht, wenn Y $R^1$ 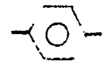 ist, Z 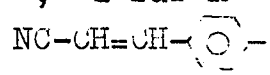 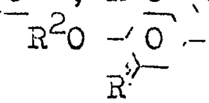 ist, $R^1$, $R^2$ eine Alkylgruppe mit 1 bis 12 Kohlenstoffatomen darstellen, $R^3$ für F, $NO_2$, $NH_2$, CN steht, als Komponenten des flüssigkristallinen Materials vorgeschlagen werden.

Nachstehend werden flüssigkristalline Derivate von 2,5-disubstituiertem Pyridin der allgemeinen Formel

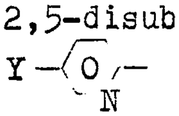 (I)

angeführt, welche erfindungsgemäß folgende Verbindungen sind:

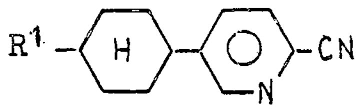
II

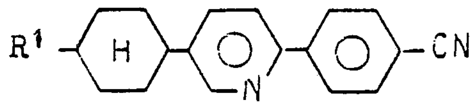
III

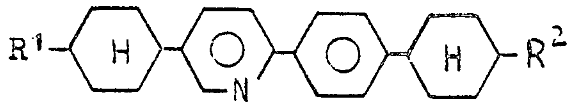
IY

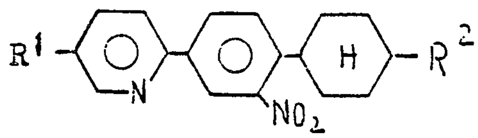
Y

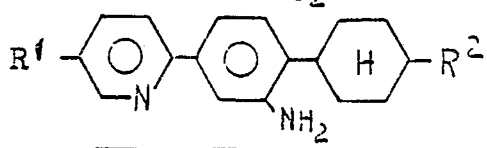
YI

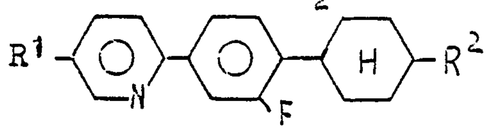
YII

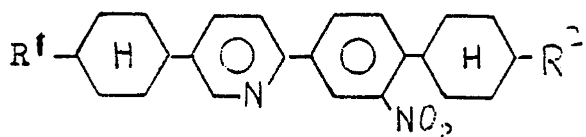
YIII

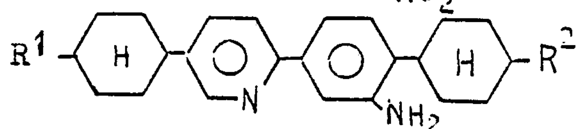
IX

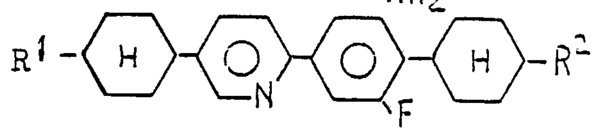
X

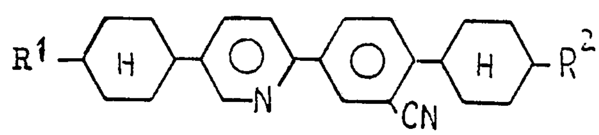
XI

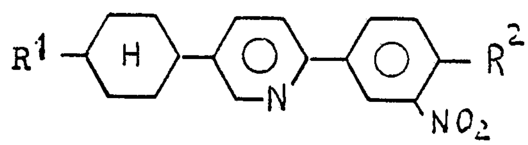
XII

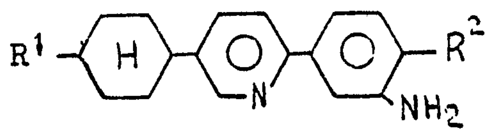

XIII

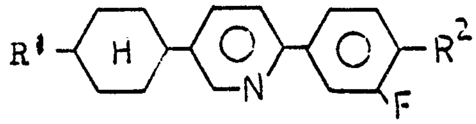

XIV

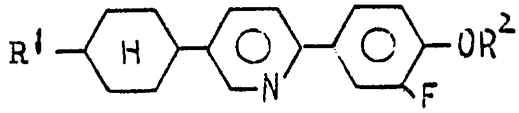

XV

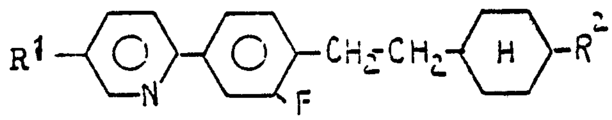

XVI

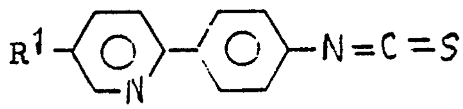

XVII

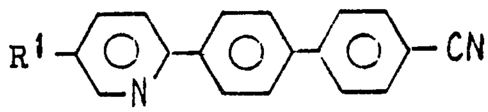

XVIII

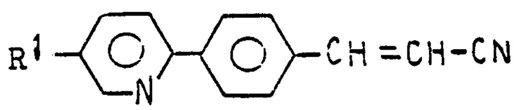

XIX

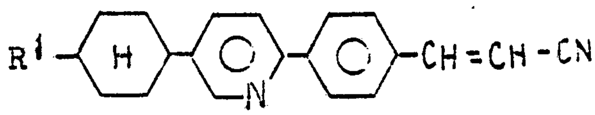

XX

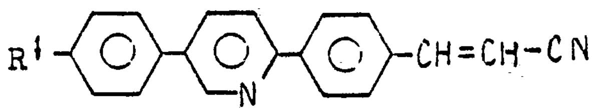

XXI

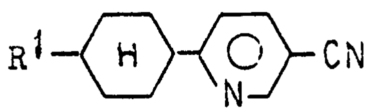

XXII

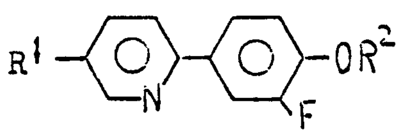

XXIII

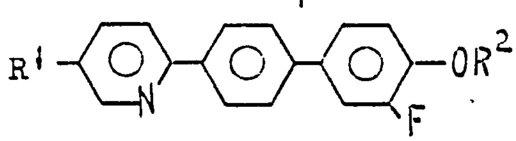

XXIV

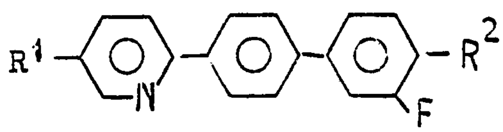

XXV

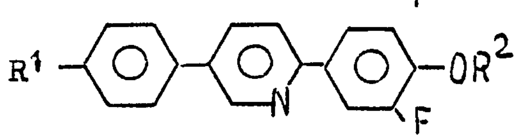

XXVI

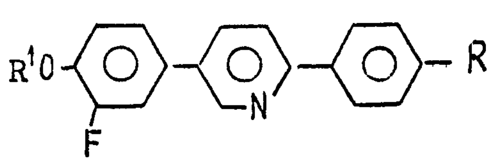

XXVII

worin $R^1$, $R^2$ für Alkyl mit 1 bis 12 Kohlenstoffatomen stehen.

Es wird auch ein flüssigkristallines Material vorgeschlagen, enthaltend zumindest zwei flüssigkristalline Komponenten, bei dem erfindungsgemäß mindestens eine der genannten flüssigkristallinen Komponenten ein flüssigkristallines Derivat von 2,5-disubstituiertem Pyridin der Formel I dient.

Es ist zweckmäßig, daß das flüssigkristalline Material 1 bis 75 Masse% flüssigkristallines Derivat von 2,5-disubstituiertem Pyridin der Formel I-XXVIII enthält.

Das flüssigkristalline Material mit dem angegebenen Mischungsverhältnis besitzt optimale Eigenschaften.

Beste Ausführungsvariante der Erfindung

Die erfindungsgemäßen Verbindungen weisen einen breiten Spektrumbereich der Eigenschaften auf, die es möglich machen, diese Verbindungen bei der Entwicklung der flüssigkristallinen Materialien zu verwenden, die in Mikrokalkulatoren, in den Uhren, elektrooptischen Einrichtungen für äußeren Verwendung sowie in Einrichtungen mit Sonnenbatteriespeisung eingesetzt werden.

Verbindungen der Formel II werden durch aufeinanderfolgende chemische Umsetzungen synthetisiert. Durch Umsetzung von 2-Methyl-5-lithium-pyridin mit 4-Alkyl-zyklohexanonen unter darauffolgender Dehydratisierung und Hydrierung sind 2-Methyl-5-(trans-4-alkylzyklohexyl)-pyridine hergestellt. Die letztgenannten sind nach den genormten Verfahren in 2-Zyano-5-(trans-4-alkylzyklohexyl)-pyridine überführt.

Nachstehend wird das Verfahren zur Synthese von Verbindungen der Formeln (III-XXI, XXII, XXIII) angegeben.

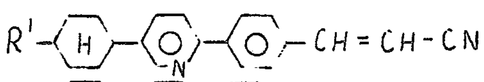
XX

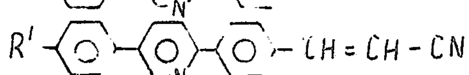
XXI

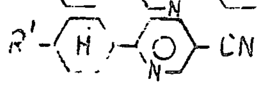
XXII

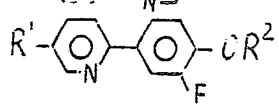
XXIII

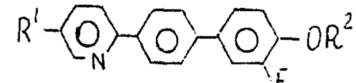 XXIV

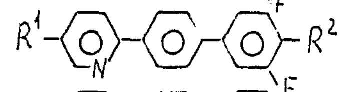 XXV

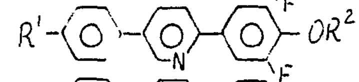 XXVI

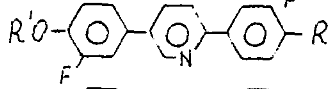 XXVII

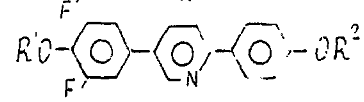 XXVIII

Durch Kondensation von Enaminen mit Vinylketonen sind 3,6-disubstituierte 3,4-Dihydro-2-N-piperidino-2H-pyrane hergestellt. Die letztgenannten sind durch Sieden mit Hydroxylammoniumchlorid in einem wäßrig-alkoholischen Medium in 2,5-disubstituierte Pyridine IV,XV, XXIII, XXIV oder ihre Zwischenprodukte überführt, in denen Brom durch Zyangruppe (III, XVIII), Akrylnitrilgruppe (XIX, XX, XXI) nach dem genormten Verfahren substituiert, die Nitrogruppe ist durch aufeinanderfolgende chemische Reaktionen in Isothiozyangruppe (XVII) überführt. Durch Nitrierung von 2,5-disubstituierten Pyridinen sind Verbindungen V, VIII, XII hergestellt, deren Reduktion zur Bildung von Aminopyridinen VI, IX, XIII führt, aus denen fluorsubstituierte Pyridine VII, X, XIV, XVI und Zyanopyridine XI synthetisiert sind.

Verbindungen der Formel XXII stellt man durch Zyklisierung des Natriumsalzes von Hydroxymethylen-methyl-4--(4-alkylzyklohexyl)-keton mit Zyanazetamid und derauffolgende Umwandlung der hergestellten 6-(4-Alkylzyklohexyl)--3-zyanopyridin-2-one in 2-Chlor-3-zyano-6-(4-alkylzyklohexyl)-pyridine her, in denen das Halogen durch katalytische Hydrierung reduziert wird.

Die Verbindungen der Formel XXVI-XXVIII stellt man aus 1-Dimethylamino-2-(4-alkylphenyl)-4-(4-alkoxy-3--fluorbenzoyl)butadien-1,3 (für XXVI) oder aus 1-Dimethylamino-2-(4-alkoxy-3-fluorphenyl)-4-(4-alkoxy- bzw. 4-alkylbenzoyl)butadien- 1,3 (für XXVII-XXVIII) durch aufeinanderfolgende Umwandlung in substituierte 2-Methoxy-2H-pyrane, Pyryliumsalze und Pyridinderivate (XXVI-XXVIII) her.

Das erfindungsgemäße flüssigkristalline Material wird durch Vermischen von mindestens zwei flüssigkristallinen Komponenten hergestellt, von denen die eine erfindungsgemäß das Derivat von 2,5-disubstituiertem Pyridin der Formel I in einer Menge von 1 bis 75 Masse% ist.

Das Vermischen erfolgt unter Erhitzen, bis die isotrope Phase auftritt, und anschließender Abkühlung.

Als andere Komponente des flüssigkristallinen Materials kann eine beliebige Verbindung, gewählt aus den Klassen der Derivate von Biphenyl, Zyklohexan, Dioxanen, Pyrimidin, Alkyl(alkoxy)phenylestern der Alkyl(alkoxy)benzoesäuren und trans-Zyklohexankarbonsäuren, Alkylphenylestern der 4-(trans-4-Alkylzyklohexyl)benzoesäure, 4-(trans-4-Alkylzyklohexyl)phenylestern der trans-4-Alkylzyklohexankarbonsäure, 1,2-Diphenyläthan, 1-Zyklohexyl-2-phenyläthan, dienen.

Die Menge von Komponenten und das Mischungsverhältnis nimmt man entsprechend den vorgegebenen Eigenschaften des flüssigkristallinen Materials.

Nachstehend sind Beispiele zur konkreten Ausführung der vorgeschlagenen Erfindung vorgeschlagen, in denen das Verfahren zur Herstellung und die Eigenschaften von Derivaten des 2,5-disubstituierten Pyridins der Formel I-XXIII sowie das Herstellungsverfahren und die Eigenschaften eines flüssigkristallinen Materials angeführt.

Beispiel 1

Herstellung von 2-Zyano-5-(trans-4-hexylzyklohexyl)-pyridin (IIa)

1.1. 1-(2-Methyl-5-pyridyl)-4-hexylzyklohexanol (1.1.a)

Zu einer Lösung von 0,504 Mol Butyllithium in 400 ml absolutem Äther wird im Argonstrom bei einer Temperatur von minus 70°C langsam unter innigem Umrühren eine Lösung von 72,2 g (0,42 Mol) 2-Methyl-5-brompyridin in 500 ml Äther getropft. Man rührt 1 Stunde lang bei minus 70 °C um und tropft dann eine Lösung von 114 g (0,63 Mol) 4-Hexylzyklohexanon in 270 ml Äther so zu, daß die Reaktionstemperatur minus 70 °C nicht übersteigt. Das Reaktionsgemisch wird noch 1 Stunde bei dieser Temperatur gerührt, danach die Nacht

über ohne Abkühlung stehengelassen. Man gießt das Reaktionsgemisch auf 1 Liter Wasser, trennt die Ätherphase ab, wäscht zweimal (mit je 300 ml) Wasser, trocknet über $Na_2SO_4$. Der Äther wird abdestilliert, und der Rückstand wird entweder unter Vakuum (230 $^0$/3 Torr) destilliert oder aus Azeton (durch Ausfrieren) umkristallisiert. Man isoliert 73 g (63,3 %) Verbindung 1.1.a als weißes Pulver mit $T_{Schmelz}$ > 100 $^0$C. Analog werden Verbindungen der allgemeinen Formel 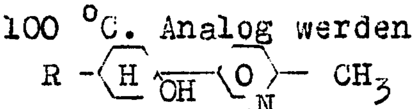 hergestellt (1.1.b-f, worin b für R = $C_3H_7$, c für R = $C_4H_9$, d für R = $C_5H_{11}$, e für R = $C_7H_{15}$, f für R $C_8H_{17}$ stehen). Die Ausbeute an Verbindungen I.I. b-f beträgt 63 bis 65 %.

1.2. 2-Methyl-5-(4-hexylzyklohexen-1-yl)-pyridin (1.2.a)

Zu 57,7 g (0,21 Mol) Verbindung 1.1.a fügt man 98 g (1 Mol) Schwefelsäure unter Rühren hinzu. Die Temperatur des Reaktionsguts wird unter 80 $^0$C gehalten. Dieses Gemisch rührt man bei der angegebenen Temperatur innerhalb von 0,5 Stunde um, kühlt ab, gießt auf Eis, alkalisiert, extrahiert die Ölschicht mit Chloroform, trocknet über Natriumsulfat, treibt Chloroform ab und destilliert den Rückstand unter Vakuum ab. Man isoliert 46 g (85 %) Verbindung 1.2. a als farbloses Öl mit $T_{Siede}$ = 200 $^0$/3 Torr.

Analog werden andere Verbindungen 1.2 (b-f) hergestellt, ihre Formeln und Eigenschaften gibt die Tabelle 1 an. Die Ausbeute an Verbindungen 1.2 b-f beträgt 85 bis 90 %.

1.3. 2-Methyl-5-(cis/trans-4-hexylzyklohexyl)-pyridin (1.3 a)

Die Lösung von 51,5 g (0,2 Mol) der Verbindung 1.2. a in 350 ml absolutem Äthanol wird bei 20 atm Druck über 7 g 5%igem Pd/C mit Wasserstoff hydriert, bis der Wasserstoff vollständig aufgenommen wird. (Die vollständige Aufnahme wird durch Gas-Flüssig-Chromatografie überwacht). Der Niederschlag wird abfiltriert, der Alkohol abgetrieben, der Rückstand unter Vakuum abdestilliert. Man isoliert 45,1 g (87 %) Verbindung 1.3 a mit $T_{Siede}$ = 170 $^0$C/2 Torr.

Gefunden in %: C 83,51; H 11,2; $C_{18}H_{29}N$. Berechnet

in %: C 83,33; H 11,27. Analog werden andere Verbindungen 1.3 b-f hergestellt, deren Eigenschaften in der Tabelle 1 angegeben sind. Die Ausbeute an Verbindungen 1.3 b-f beträgt 85 bis 90 %.

1.4. 2-Hydroxymethyl-5-(cis- und trans-4-hexylzyklohexyl)-pyridin (1.4.a)

Die Substanz 1.4.a ist in 65%iger Ausbeute durch Überführung der Verbindung 1.3 a in N⟶O-Derivat durch Sieden in Gegenwart von Wasserstoffperoxid in Essigsäure und darauffolgende Umlagerung unter Einwirkung von Essigsäureanhydrid in 2-Azetyloxymethyl-5-(cis/trans-4-hexylzyklohexyl)-pyridin hergestellt, welches ohne Isolieren nach der Abdestillation des überschüssigen Essigsäureanhydrids mit 10%igem wäßrigalkoholischem Alkali verseift wird. Man isoliert eine Verbindung 1.4. a mit $T_{Siede}$ = = 205 °C/1 Torr. Analog werden andere Verbindungen 1.4 b-f hergestellt, deren Formeln und Eigenschaften in der Tabelle 1 angegeben sind. Die Ausbeute an anderen Homologen 1.4 b-f beträgt 60 bis 70 %.

2-Zyano-5-(trans-4-hexylzyklohexyl)-pyridin (II a)

Zu einer Lösung von 11,1 g (0,04 Mol) Verbindung 1.4. a in 40 ml (0,55 Mol) Dioxan (frisch destilliert über Ätznatron) fügt man 2,6 g (0,02 Mol) fein zerkleinerte selenige Säure hinzu. Man erhitzt das Reaktionsgut auf 80 °C bei einem schnellwirkenden Rührwerk und hält diese Temperatur innerhalb von 2 Stunden unter ununterbrochenem Rühren konstant. Man filtriert durch eine dünne Aluminiumoxidschicht, treibt Dioxan aus dem Filtrat ab. Dem Rückstand werden 100 ml Äthanol, 8 g (0,12 Mol) Hydroxylammoniumhydrogenchlorid und 13,3 g (0,33 Mol) Ätznatron in 50 ml Wasser zugegeben. Das Gemisch wird innerhalb von 1 Stunde zum Sieden erhitzt, in 200 ml Wasser gegossen, mit der verdünnten Salzsäure angesäuert. Der ausgefallene amorphe Niederschlag wird abfiltriert, aus Alkohol umkristallisiert, mit Hexan gewaschen. Man isoliert 4,4 g (38 %) 5-(trans-4--Hexylzyklohexyl)-pyridin-2-oxim, welches man mit 20 ml trockenem Chloroform verdünnt, setzt 5,9 g Phosphoroxidchlorid zu und siedet innerhalb von 1 Stunde mit Rückflußkühler. Die Reaktionsmasse wird auf Wasser gegossen, mit Natriumhydrogenkarbonatlösung neutralisiert, die organische Phase mit Wasser gewaschen, durch eine kleine mit $Al_2O_3$ aufgefüllte Säule gelassen und das Lösungsmittel aus dem Filtrat abdestilliert. Der Rückstand wird aus Hexan (viermal) kristallisiert. Man isoliert 1 g Substanz II a (9,1 %) mit $T_{C-N}$ = 35,3 ° und $T_{N-I}$ = 49,4 °.

Gefunden in %: C 80,01; H 9,54. $C_{18}H_{26}N_2$.

Berechnet in %: C 79,95; H 9,69.

Analog werden andere 2-Zyano-5-(trans-4-alkylzyklohexyl)-pyridine II b-f hergestellt, deren Eigenschaften, Bruttoformel und Elementaranalysenangaben in der Tabelle 1 dargestellt sind. Die Gesamtausbeute an Verbindungen II a-f beträgt 9-13%. Alle neuen Stoffe der Formel II sind bei Raumtemperatur weiße kristalline Pulver.

Der Aufbau der Verbindungen II ist durch magnetische Kernresonanzspektroskopie nachgewiesen. Das Spektrum von Verbindungen II stellt ein Dreispinsystem ABX (δ ppm $CCl_4$): 8,24 ($H_4$), 8,34 ($H_3$), 9,06 ($H_6$).

Die Struktur der Verbindungen II wird auch durch magnetische Kernresonanz mit $^{13}C$ nachgewiesen.

Die Formeln und die Eigenschaften der synthetisierten Verbindungen 1.2, 1.3, 1.4, II sind in der Tabelle 1 angegeben.

Tabelle 1

| lfd. Nr. | 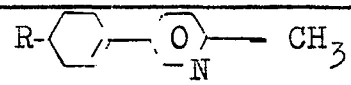 (I.2.) | | |
|---|---|---|---|
| 1 | R | $T_{Siede}$ in °C Torr | $T_{Schmelz}$ °C |
| | 2 | 3 | 4 |
| a | $C_6H_{13}$ | 200/3 | - |
| | $C_3H_7$ | 201/20 | - |
| c | $C_4H_9$ | 196/22 | - |
| d | $C_5H_{II}$ | 210/14 | 32 |
| e | $C_7H_{15}$ | 197/2 | 36,5 |
| f | $C_8H_{17}$ | 190/1 | 24,9 (24,6)$^x$ |

$^x$monotrope Umwandlung

Fortsetzung der Tabelle 1

| lfd. Nr. | R 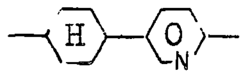 $-CH_3$ (1.3): $T_{Siede}$ in °C/Torr | R- 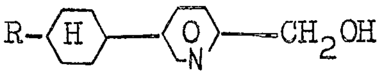 $CH_2OH$(1.4.) $T_{Siede}$ in °C/Torr |
|---|---|---|
| 1 | 5 | 6 |
| a | 170/2 | 240/10 |
| b | 135/2 | 170/2 |
| c | 145/2 | 175/2 |
| d | 168/2 | 215/8 |
| e | 171/1 | 215/1 |
| f | 186/1 | 230/8 |

Fortsetzung der Tabelle 1

R 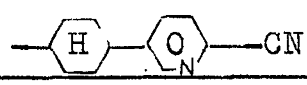 CN (II)

| lfd. Nr. | $T_{C-N}$, in °C | $T_{N-I}$, °C | Gefunden in % C | Gefunden in % H | Brutto-formel | Berechnet in% C | Berechnet in% H |
|---|---|---|---|---|---|---|---|
| 1 | 7 | 8 | 9 | 10 | 11 | 12 | 13 |
| a | 35,3 | 49,4 | 80,01 | 9,54 | $C_{18}H_{26}N_2$ | 79,95 | 9,69 |
| b | 68 | 25* | 78,96 | 8,82 | $C_{15}H_{20}N_2$ | 78,90 | 8,82 |
| c | 60,7 | 37* | 79,14 | 9,25 | $C_{16}H_{22}N_2$ | 79,29 | 9,15 |
| d | 45,4 | 55,4 | 79,71 | 9,36 | $C_{17}H_{24}N_2$ | 79,64 | 9,44 |
| e | 50,1 | 60,2 | 80,16 | 10,01 | $C_{19}H_{28}N_2$ | 80,23 | 9,92 |
| f | 49,0 | 57,9 | 80,54 | 10,24 | $C_{20}N_{30}N_2$ | 80,48 | 10,13 |

* monotrope Umwandlung

Zur Messung der elektrooptischen Parameter der Verbindungen der Formel II verwendete man das Gemisch des fünften und des siebten Homologen, welches einen breiten Temperaturenbereich der nematischen Phase aufweist. Es ist auch zu berücksichtigen, daß die Gesetzmäßigkeit der Änderung der Eigenschaften bei Gliedern der homologen Reihe mit dem ausreichenden Genauigkeitsgrad gewöhnlich bekannt ist, d.h. nach den Eigenschaften des Gemisches aus dem fünften und siebten Homologen kann man auf die Eigenschaften der Glieder der ganzen homologen Reihe schließen.

Es wurden deshalb folgende binäre Mischungen hergestellt: Mischung A aus 40 Mol.% 2-Zyano-5-(trans-4-amylzyklohexyl)-pyridin und 60 Mol.% 2-Zyano-5-(trans-4-heptylzyklohexyl)-pyridin und vergleichsweise Mischung B aus 40 Mol.% trans-4-Amyl-(4-zyanophenyl)-zyklohexan und 60 Mol% trans-4-Heptyl-(4-zyanophenyl)-zyklohexan (DE,B 2636684).

Vergleichswerte von Klärpunkten ($T_{N-I}$), Anisotropien der Dielektrizitätskonstante, Schwellspannung und Sättigungsspannung beim Twist-Effekt bei Mischungen A und B sind in der Tabelle 2 angegeben.

Tabelle 2

| Mischungen | $T_{N-I}$, °C | $\Delta\varepsilon$ | $U_{Schwell}$ in V | $U_{Sätt}$ in V |
|---|---|---|---|---|
| 1 | 2 | 3 | 4 | 5 |
| A | 58 | +12,4 | 1,25 | 1,8 |
| B | 56,4 | +11,7 | 1,7 | 2,3 |

Darin bedeuten:

$\Delta\varepsilon = \varepsilon_{\parallel} - \varepsilon_{\perp}$ Anisotropie der Dielektrizitätskonstante bei der reduzierten Temperatur $\tau = 0,95$ ( $\tau = T_{Meß} / T_{N-I}$ in °K)

$U_{Schwell}$ und $U_{Sätt}$ Schwellspannung bzw. Sättigungsspannung beim Twist-Effekt, normaler Lichteinfall, Zellendicke 10 $\mu$m.

Aus der Tabelle 2 ist zu ersehen, daß der Klärpunkt der Mischung aus neuen Verbindungen der Formel II (Mischung A) den der Mischung aus trans-4-Alkyl-(4-zyanophenyl)-zyklohexanen (Mischung B) übersteigt. Wie die Tabelle 2 zeigt,

beträgt außerdem die Schwellspannung und die Sättigungsspannung beim Twist-Effekt für Mischung A (neue Substanzen) 1,25 bzw. 1,8 V und ist bedeutend geringer als die für Mischung B ($U_{Schwell}$ = 1,7 V $U_{Sätt}$ = 2,3 V). Die nachstehenden Beispiele 2 bis 24 veranschaulichen die Verwendung der erfindungsgemäßen Verbindungen II als Komponenten von flüssigkristallinen Materialien, hergestellt nach dem auf der Seite 6 beschriebenen Verfahren.

In Beispielen 2 bis 24 sind die Zusammensetzungen in Masseprozent angegeben.

Beispiel 2

2-Zyano-5-(trans-4-propylzyklohexyl)-pyridin (II ) 6
2-Zyano-5-(trans-4-butylzyklohexyl)-pyridin (II c) 7
2-Zyano-5-(trans-4-amylzyklohexyl)-pyridin (II d) 25
2-Zyano-5-(trans-4-hexylzyklohexyl)-pyridin (II a) 10
2-Zyano-5-(trans-4-heptylzyklohexyl)-pyridin (II e) 35
2-Zyano-5-(trans-4-oktylzyklohexyl)-pyridin (II f) 17

Bei diesem flüssigkristallinen Material ist $U_{Schwell}$= = 1,15 V und $U_{Sätt}$ =1,7 V. Der Temperaturenbereich der nematischen Phase beträgt -5 bis + 55 °C.

Beispiel 3

2-Zyano-5-(trans-4-propylzyklohexyl)-pyridin (II b) 10
2-Zyano-5-(trans-4-amylzyklohexyl)-pyridin (II d) 20
2-Zyano-5-(trans-4-heptylzyklohexyl)-pyridin (II e) 23
4-Amyl-4'-zyanobiphenyl 40
4-Amyl-4"-zyano-p-terphenyl 7

Bei diesem flüssigkristallinen Material ist $U_{Schwell}$= =1,3 V, $U_{Sätt}$ = 1,85 V. Der Temperaturenbereich der nematischen Phase beträgt -9 bis + 60 °C

Beispiel 4

2-Zyano-5-(trans-4-butylzyklohexyl)-pyridin (II c) 20
2-Zyano-5-(trans-4-hexylzyklohexyl)-pyridin (II a) 28
trans-4-Propyl-(4-zyanophenyl)-zyklohexan 20
trans-4-Amyl-(4-zyanophenyl)-zyklohexan 25
trans-4-Amyl-(4-zyanobiphenyl)-zyklohexan 7

Bei diesem flüssigkristallinen Material ist $U_{Schwell}$= = 1,5 V und $U_{Sätt}$ = 2,1 V. Der Temperaturenbereich der nematischen Phase beträgt -12 bis + 60 °C.

Beispiel 5

| | | |
|---|---|---|
| 2-Zyano-5-(trans-4-hexylzyklohexyl)-pyridin | (II a) | 15 |
| 2-Zyano-5-(trans-4-oktylzyklohexyl)-pyridin | (II f) | 15 |
| trans-4-Propylzyklohexankarbonsäure-4'-äthoxyphenylester | | 17,5 |
| trans-4-Butylzyklohexankarbonsäure-4'-äthoxyphenylester | | 17,5 |
| trans-4-Amylzyklohexankarbonsäure-4'-äthoxyphenylester | | 17,5 |
| trans-4-Hexylzyklohexankarbonsäure-4'-äthoxyphenylester | | 17,5 |

Bei diesem flüssigkristallinen Material ist $U_{Schwell}$ = 1,7 V und $U_{Sätt}$ =2,25 V. Der Temperaturenbereich der nematischen Phase beträgt -6 bis + 60 °C.

Beispiel 6

| | | |
|---|---|---|
| 2-Zyano-5-(trans-4-propylzyklohexyl)-pyridin | (II b) | 5 |
| trans-4-Propyl-(4-zyanophenyl)-zyklohexan | | 22 |
| trans-4-Amyl-(4-zyanophenyl)-zyklohexan | | 27 |
| trans-4-Heptyl-(4-zyanophenyl)-zyklohexan | | 22 |
| 4-Äthyl-4-(trans-4-amylzyklohexyl)-biphenyl | | 12 |
| 4-(trans-4-Amylzyklohexyl)-4'-(trans-4-propylzyklohexyl)-biphenyl | | 12 |

Bei diesem flüssigkristallinen Material ist $U_{Schwell}$ =1,5 V und $U_{Sätt}$ = 2,1 V. Der Temperaturenbereich der nematischen Phase beträgt -10 bis +80 °C.

Beispiel 7

| | | |
|---|---|---|
| 2-Zyano-5-(trans-4-propylzyklohexyl)-pyridin | (II b) | 10 |
| 2-Zyano-5-(trans-4-butylzyklohexyl)-pyridin | (II c ) | 15 |
| 2-Zyano-5-(trans-4-amylzyklohexyl)-pyridin | (II b) | 20 |
| 2-Zyano-5-(trans-4-hexylzyklohexyl)-pyridin | (II a) | 15 |
| 2-Zyano-5-(trans-4-heptylzyklohexyl)-pyridin | (II e) | 15 |
| 4-Propyl-4'-(trans-4-amylzyklohexyl)-biphenyl | | 15 |
| 4-(trans-4-Amylzyklohexyl)-4'-(trans-4-propylzyklohexyl)-biphenyl | | 10 |

Bei diesem flüssigkristallinen Material ist $U_{Schwell}$ = 1,2 V und $U_{Sätt}$ = 1,7 V. Der Temperaturenbereich der nematischen Phase beträgt -8 bis + 75°C.

Beispiel 8

| | |
|---|---|
| 2-Zyano-5-(trans-4-amylzyklohexyl)-pyridin (II d) | 3 |
| trans-4-Propyl-(4-zyanophenyl)-zyklohexan | 24 |
| trans-4-Amyl-(4-zyanophenyl)-zyklohexan | 27 |
| trans-4-Heptyl-(4-zyanophenyl)-zyklohexan | 22 |
| 4-Äthyl-4'-(trans-4-amylzyklohexan)-biphenyl | 12 |
| 4-(trans-4-Amylzyklohexyl)-4'-(trans-4-propylzyklohexyl)-biphenyl | 12 |

Bei diesem flüssigkristallinen Material ist $U_{Schwell}$ =1,6 V und $U_{Sätt}$ = 2,15 V. Der Temperaturenbereich der nematischen Phase beträgt -12 bis +80 °C.

Beispiel 9

| | |
|---|---|
| 2-Zyano-5-(trans-4-propylzyklohexyl)-pyridin (II b) | 20 |
| 2-Zyano-5-(trans-4-butylzyklohexyl)-pyridin (II c) | 15 |
| 2-Zyano-5-(trans-4-amylzyklohexyl)-pyridin (II d) | 25 |
| 2-Zyano-5-(trans-4-heptylzyklohexyl)-pyridin (II a) | 20 |
| 4-Amyl-4'-zyanobiphenyl | 20 |

Bei diesem flüssigkristallinen Material ist $U_{Schwell}$ =1,2 V und $U_{Sätt}$ = 1,75 V. Der Temperaturenbereich der nematischen Phase beträgt -6 bis +54 °C.

Beispiel 10

| | |
|---|---|
| 2-Zyano-5-(trans-4-amylzyklohexyl)-pyridin (II d) | 40 |
| 2-Zyano-5-(trans-4-heptylzyklohexyl)-pyridin (II e) | 25 |
| trans-4-Butyloxy-(4-zyanophenyl)-zyklohexan | 35 |

Bei diesem flüssigkristallinen Material ist $T_{N-I}$=58 °C, $U_{Schwell}$ = 1,3 V und $U_{Sätt}$ =1,8 V.

Beispiel 11

| | |
|---|---|
| 2-Zyano-5-(trans-4-butylzyklohexyl)-pyridin (II c) | 30 |
| 4-Butyl-4'-methoxyazoxybenzol | 46,9 |
| 4-Äthyl-4'-methoxyazoxybenzol | 23,1 |

Bei diesem flüssigkristallinen Material ist $U_{Schwell}$ =1,25 V und $U_{Sätt}$ =1,75 V. Der Temperaturenbereich der nematischen Phase beträgt +5 bis +66 °C.

Beispiel 12

| | |
|---|---|
| 4-Amyl-4'-zyanobiphenyl | 35 |
| 4-Hexyloxy-4'-zyanobiphenyl | 11 |
| 2-Zyano-5-(trans-4-amylzyklohexyl)-pyridin (II d) | 32 |
| 2-Zyano-5-(trans-4-heptylzyklohexyl)-pyridin (II e) | 22 |

Bei diesem flüssigkristallinen Material ist $U_{Schwell}$ = 1,4 V und $U_{Sätt}$ = 1,85 V. Der Temperaturenbereich der nematischen Phase beträgt -3 bis +51 °C.

Beispiel 13

| | |
|---|---|
| 4'-Methoxybenzoesäure-4-propylphenylester | 15 |
| 4'-Methoxybenzoesäure-4-amylphenylester | 20 |
| 4-Hexanoyloxybenzoesäure-4 -propylphenylester | 17 |
| 4 -Butylbenzoesäure-4-(4-propyl)-biphenylester | 10 |
| 4' -Amylbenzoesäure-4-propylbiphenylester | 8 |
| 2-Zyano-5-(trans-4-propylzyklohexyl)-pyridin (II b) | 30 |

Bei diesem flüssigkristallinen Material ist $U_{Schwell}$ =1,5 V und $U_{Sätt}$ = 2,0 V. Der Temperaturenbereich der nematischen Phase beträgt -2 bis +61 °C.

Beispiel 14

| | |
|---|---|
| 2-Zyano-5-(trans-4-amylzyklohexyl)-pyridin (II d) | 27 |
| 4-Amyl-4"-zyano-p-terphenyl | 8 |
| 4 -Methoxybenzoesäure-4-phenylpropylester | 17 |
| 4 -Methoxybenzoesäure-4-amylphenylester | 48 |

Bei diesem flüssigkristallinen Material ist $U_{Schwell}$ = 1,55 V und $U_{Sätt}$ = 2,2 V. Der Temperaturenbereich der nemätischen Phase beträgt 0 bis + 55 °C.

Beispiel 15

| | |
|---|---|
| 4-Butyl-4'-methoxyazoxybenzol | 33,5 |
| 4-Äthyl-4'-methoxyazoxybenzol | 16,5 |
| 5-(4-Zyanophenyl)-4-amyl(thiobenzoat) | 10 |
| 2-Zyano-5-(trans-4-propylzyklohexyl)-pyridin (II b) | 20 |
| 2-Zyano-5-(trans-4-butylzyklohexyl)-pyridin (II c) | 20 |

Bei diesem flüssigkristallinen Material ist $U_{Schwell}$ = 1,4 V, und $U_{Sätt}$ = 1,9 V. Der Temperaturenbereich der nematischen Phase beträgt 0 bis 64 °C.

Beispiel 16

| | |
|---|---|
| 4- Butyl-4'-methoxyazoxybenzol | 26,8 |
| 4- Äthyl-4 -methoxyazoxybenzol | 13,2 |
| 4- Amylbenzoesäure-4'-zyanophenylester | 10 |
| 4'-Heptylbenzoesäure-4-zyanophenylester | 10 |
| 2-Zyano-5-(trans-4-butylzyklohexyl)-pyridin (II c) | 20 |
| 2-Zyano-5-(trans-4-amylzyklohexyl)-pyridin (II d) | 20 |

Bei diesem flüssigkristallinen Material ist $U_{Schwell}$= 1,4 V und $U_{Sätt}$ = 1,85 V. Der Temperaturenbereich der nematischen Phase beträgt -2 bis + 62 °C.

**Beispiel 17**

| | |
|---|---|
| 2-Zyano-5-(trans-4-heptylzyklohexyl)-pyridin (II e) | 30 |
| N-(4- Hexylbenzyliden)-4-zyanoanilin | 49 |
| N-(4- Propylbenzyliden)-4-zyanoanilin | 21 |

Bei diesem flüssigkristalinen Material ist $U_{Schwell}$ = 1,5 V und $U_{Sätt}$ = 2,1 V. Der Temperaturenbereich der nematischen Phase betragt -3 bis +64 °C.

Beispiel 18

| | |
|---|---|
| 2-Zyano-5-(trans-4-propylzyklohexyl)-pyridin (II b) | 10 |
| 2-Zyano-5-(trans-4-butylzyklohexyl)-pyridin (II c) | 10 |
| 2-Zyano-5-(trans-4-amylzxklohexyl)-pyridin (II d) | 25 |
| 4- Butylbenzoesäure-4'-zyanophenylester | 15 |
| 4- Amylbenzoesäure-4'-zyanophenylester | 20 |
| 4- Hexylbenzoesäure-4'-zyanophenylester | 20 |

Bei diesem flüssigkristallinen Material ist $U_{Schwell}$ = 1,35 V und $U_{Sätt}$ = 1,7 V. Der Temperaturenbereich der nematischen Phase beträgt 0 bis +53 °C.

Beispiel 19

| | |
|---|---|
| 4- Amyl-4'-zyanobiphenyl | 20 |
| 4- Heptyl-4'-zyanobiphenyl | 20 |
| 4- Amyl-4" -zyano-p-terphenyl | 10 |
| 2-Zyano-5-(trans-4-amylzyklohexyl)-pyridin (II d) | 25 |
| 2-Zyano-5-(trans-4-heptylzyklohexyl)-pyridin (II e) | 25 |

Bei diesem flüssigkristallinen Material ist $U_{Schwell}$ =1,3 V und $U_{Sätt}$= 1,7 V. Der Temperaturenbereich der nematischen Phase beträgt -1 bis +66 °C.

Beispiel 20

| | |
|---|---|
| 4- Propyloxy-4'-zyanobiphenyl | 15 |
| 4- Amyloxy-4'-zyanobiphenyl | 20 |
| 4- Amyl-4" -zyano-p-terphenyl | 15 |
| 2-Zyano-5-(trans-4-propylzyklohexyl)-pyridin (II b) | 20 |
| 2-Zyano-5-(trans-4-butylzyklohexyl)-pyridin (II c) | 30 |

Bei diesem flüssigkristallinen Material ist $U_{Schwell}$ = 1,25 V und $U_{Sätt}$ =1,6 V. Der Temperaturenbereich der nematischen Phase beträgt +4 bis +70 °C.

Beispiel 21

| | |
|---|---|
| 2-Zyano-5-(trans-4-heptylzyklohexyl)-pyridin (II e) | 29,2 |
| 4-(2-Methylbutyl)-1(2-zyanophenyl)-zyklohexan | 0,8 |

N-(4- Hexylbenzyliden)-4-zyanoanilin 49

N-(4- Propylbenzyliden)-4-zyanoanilin 21

Bei diesem flüssigkristallinen Material ist $U_{Schwell}$ =1,5 V und $U_{Sätt}$ =2,1 V. Der Temperaturenbereich der nematischen Phase beträgt -5 bis +67 °C.

Beispiel 22

4- Propyloxy-4''-zyan -p-terphenyl 15

4- Amyloxy-4' -zyanobiphenyl 20

4- Amyl-4' zyano-p-terphenyl 15

2-Zyano-5-(trans-4-butylzyklohexyl)-pyridin (II c) 30

2-Zyano-5-(trans-4-propylzyklohexyl)-pyridin 19

4-(Methoxyheptyl)-1-(4-zyanophenyl)-zyklohexan 1

Bei diesem flüssigkristallinen Material ist $U_{Schwell}$ = 1,2 V und $U_{Sätt}$ = 1,55 V. Der Temperaturenbereich der nematischen Phase beträgt +4 bis +68 °C.

Beispiel 23

2-Zyano-5-(trans-4-amylzyklohexyl)-pyridin (II d) 20

2-Zyano-5-(trans-4-heptylzyklohexyl)-pyridin (II e) 20

5-Amyl-2-(4-zyanophenyl)-pyridin 10

trans-4-Butylzyklohexankarbonsäure-4-äthoxyphenylester 25

trans-4-Hexylzyklohexankarbonsaure-4-äthoxyphenylester 15

trans-4-Butylzyklohexankarbonsäure-4-zyanobiphenylester 10

Bei diesem flüssigkristallinen Material ist $U_{Schwell}$=1,5 V und $U_{Sätt}$ =2,1 V. Der Temperaturenbereich der nematischen Phase beträgt -9 bis +71 °C.

Beispiel 24

2-Zyano-5-(trans-4-amylzyklohexyl)-pyridin (II d) 25

5-Amyl-2-(4-zyanophenyl)-1,3-dioxan 25

trans-4-Butylzyklohexankarbonsäure-4-äthoxyphenylester 25

trans-4-Hexylzyklohexankarbonsäure-4-äthoxyphenylester 15

4-Äthyl-4'-(trans-4-amyl)-zyklohexylbiphenyl 10

Bei diesem flüssigkristallinen Material ist $U_{Schwell}$ =1,35 V und $U_{Sätt}$ = 1,75 V. Der Temperaturenbereich der nematischen Phase beträgt -11 bis +67 °C.

Beispiele 2 bis 24 zeigen, dass die Verbindungen der

Formel II einen breiten Bereich der nematischen Phase und geringe Steuerspannungen für flüssigkristalline Materialien sichern werden. Diese Materialien sind zweckmäßigerweise in elektrooptischen Einrichtungen mit Sonnenbatteriespeisung zu verwenden.

Beispiel 25

Herstellung von 2-(4-Zyanophenyl)-5-(trans-4-butylzyklohexyl)-pyridin (III a)

25.1. trans-1-Butyl-4-zyklohexylazetaldehyd 25.1. a

Zu einem Gemisch von 0,05 Mol Magnesiummetall in 20 ml absolutem Tetrahydrofuran werden 0,05 Mol trans-1-Butyl-4-brommethylzyklohexan langsam unter Sieden getropft. Nach der Auflösung von Magnesium wird die erhaltene Lösung zu einer auf -40 °C abgekühlten Lösung von 0,045 Mol N-Methyl-N-formyl-2-aminopyridin in 50 ml absolutem Tetrahydrofuran getropft. Man rührt das Reaktionsgemisch bei einer Temperatur von -20 °C innerhalb von 15 bis 20 Minuten um und gießt in 150 ml 5%ige auf 0 °C vorgekühlte Salzsäure aus. Man extrahiert mit Äther, wäscht den Extrakt mit einer gesättigten wäßrigen Natriumchloridlösung, mit Wasser, trocknet über Natriumsulfat, treibt den Äther ab, destilliert den Rückstand unter Vakuum und isoliert die Verbindung 25.1.a in 73%iger Ausbeute mit $T_{Siede}$ = 85 bis 87 °C/1 Torr. IR-Spektrum mit Co-$\gamma$- Strahlung, 1720 $cm^{-1}$ Wellenlänge, kernmagnetisches $^{1}H$-NMR-Spektrum ($CCl_4$, $\delta$, ppm):

Triplett 0,85 (J 6 Hz, $CH_3$); Multiplett 0,97-1,85 ($CH_2$, CH);

Doppelduplett 2,22 (J 6 und 2 Hz, $CH_2CO$); Triplett 9,65 (J 2 Hz, CHO).

2,4-Dinitrophenylhydrazon (aus Äthanol) hat $T_{Schmelz}$= 117 bis 118 °C.

Gefunden in %: C 59,45; H 7,38; N 15,36; $C_{18}H_{26}N_4O_4$.

Berechnet in %: C 59,65; H 7,23; N 15,46.

Analog sind trans-1-Alkyl-4-zyklohexylazetaldehyde (25.1.b-d) hergestellt, worin b für R = $C_3H_7$, c für R = $C_5H_{11}$, d für R = $C_6H_{13}$ stehen, wobei ihre Eigenschaften die Tabelle 3 angibt.

Tabelle 3

| Verbindung | Ausbeute in % | $T_{Siede}$ in °C mm/Torr | $T_{Schmelz}$ von 2,4-Dinitrophenylhydrazon in °C |
|---|---|---|---|
| 25.I.b | 5I | 69-7I/I | I05 - I08 |
| 25.I.c | 72 | 93-95/I | II4 - II8 |
| 25.I.d | 70 | i39-I42/I | II3-II3,5 |

Anmerkung. Verbindungen 25.1.b, c sind aus 1-Alkyl-4-brommethylzyklohexanen erhalten, welche eine Mischung von trans- und cis-Isomeren im Verhältnis von 7:3 darstellen.

25.2. 1-[β-(4-trans-Butylzyklohexyl)äthylenyl]-piperidin (25.2.a)

Man vermischt 0,078 Mol wasserfreies Natriumsulfat mit 0,086 Mol Piperidin und tropft 0,039 Mol Verbindung 25.1.a bei -5 °C im Stickstoffstrom zu, rührt innerhalb von 3 Tagen bei Raumtemperatur um, filtriert den Niederschlag ab, wäscht mit Hexan, vereinigt die Filtrate und destilliert unter Vakuum. Man isoliert die Verbindung 25.2.a in 53%iger Ausbeute mit $T_{Siede}$ von 139 bis 141 °C/3 Torr.
Gefunden in %: N 5,80; $C_{17}H_{31}N_9$. Berechnet in %: N 5,61. $n_D^{20}$ = 1,4973. $^1$H-NMR-Spektrum ($CCl_4$, δ, ppm): Triplett 0,85 (J 6 Hz, $CH_3$), Multiplett 0,97-1,85 ($CH_2$,CH), Multiplett 2,52-2,75 ($CH_2N$), Doppelduplett 4,10 (J 7 und 14 Hz, CH = C), Duplett 5,60 (J 14 Hz, C=CH-N)

Analog sind dazu gehörige Verbindungen 1-[β-(4-trans-Alkylzyklohexyl)-äthylenyl]-piperidine 25.2 b-d hergestellt, deren Eigenschaften und Elementaranalyse in der Tabelle 4 angegeben sind.

Tabelle 4

| Verbindung | Ausbeute in % | $T_{Siede}$ in °C/Torr | Gefunden in %, N | Bruttoformel | Berechnet in %, N |
|---|---|---|---|---|---|
| 25.2.b | 75 | II8-I20(I) | 6,I3 | $C_{I6}H_{29}N$ | 5,95 |
| 25.2.c | 30 | I53-I55/I | 5,52 | $C_{I8}H_{33}N$ | 5,3I |
| 25.2.d | 56 | I63-I65/I | 5,I5 | $C_{I9}H_{35}N$ | 5,05 |

25.3. 3-(trans-4-Butylzyklohexyl)-6-(4-bromphenyl)-
-3,4-dihydro-2-N-piperidino-2H-pyran (25.3 a)

Man vermischt in der Stickstoffatmosphäre bei 20 °C 0,034 Mol Verbindung 25.2. a mit 0,037 Mol 1-Akryloyl-4-brombenzol. Das Gemisch wird innerhalb von 1 Stunde gerührt und über Tag und Nacht stehengelassen. Man kühlt es auf -20 °C ab, setzt 10 ml Methylalkohol zu, filtriert den ausgefallenen Niederschlag ab und trocknet im Vakuum. Man isoliert die Verbindung 25.3.a in 87%iger Ausbeute mit $T_{Schmelz}$ von 93,5 bis 95,5 °C.

Gefunden in %: C 67,67; H 8,16; N 3,08; $C_{26}H_{38}$ BrNO
Berechnet in %: C 67,81; H 8,32; N 3,04.

$^1$H-NMR-Spektrum ($CCl_4$, $\delta$ ,ppm): Triplett 0,90 (J 6 Hz, $CH_3$), Multiplett 0,95-2,12 ($CH_2$, CH), Multiplett 2,45-3,07 ($CH_2N$), Duplett 4,27 (J 8 Hz, NCHO), Triplett 5,10 (3 Hz, C = CH), Singulett 7,31 (ArH).

UV-Spektrum $\lambda_{max}$(lg $\varepsilon$): 207 (4,23), 258 (4,35) (95%iges Äthanol).

Analog sind dazu gehörige 3-(trans-4-Alkylzyklohexyl)-6-(4-bromphenyl)-3,4-dihydro-2-N-piperidino-2H-pyrane (25.3 b-d) hergestellt, deren Eigenschaften und Elementaranalyse in der Tabelle 5 angegeben sind.

Tabelle 5

| lfd. Nr. | Verbindung | Ausbeute in % | $T_{Schmelz}$ in °C | Gefunden in % C | H | N |
|---|---|---|---|---|---|---|
| 1 | 2 | 3 | 4 | 5 | 6 | 7 |
| 1. | 25.3.b | 93 | 102 | 66,96 | 8,09 | 2,94 |
| 2. | 25.3.c | 42 | 90 | 68,09 | 8,3I | 2,70 |
| 3. | 25.3.d | 79 | 59 | 68,58 | 8,49 | 2,74 |

Fortsetzung der Tabelle 5

| lfd. Nr. | Bruttoformel | Berechnet in % C | H | N |
|---|---|---|---|---|
| 1 | 8 | 9 | 10 | 11 |
| 1. | $C_{25}H_{36}BrNO$ | 67,25 | 8,I3 | 3,I4 |
| 2. | $C_{27}H_{40}BrNO$ | 68,34 | 8,50 | 2,95 |
| 3. | $C_{28}H_{42}BrNO$ | 68,84 | 8,67 | 2,87 |

25. 4. 4-Formyl-4-(trans-4-butylzyklohexyl)-1-(4-bromphenyl-butanon (25.4.a)

Zu einer Lösung von 0,074 Mol Oxalsäure in 70 ml Wasser werden 0,037 Mol Verbindung 25.3.a gegeben und innerhalb von 24 Stunden bei 20 $^{0}C$ gerührt. Man extrahiert mit Äther, wäscht den Extrakt mit Wasser, einer gesättigten wäßrigen Natriumchloridlösung, trocknet über $Na_2SO_4$ und treibt das Lösungsmittel unter Vakuum ab. Man isoliert die Verbindung 24.4.a in 77%iger Ausbeute mit $T_{Schmelz}$ von 71 bis 72°C (Hexan).

Gefunden in %: C 64,4; H 7,57; Br 20,03; $C_{21}H_{29}BrO_2$
Berechnet in %: C 64,12; H 7,43; Br 20,31.

$^1H$-NMR-Spektrum ($CCl_4$, $\delta$, ppm): Multiplett 0,73 (J 6 Hz, $CH_3$), Multiplett 0,85-2,12 ($CH_2$, CH), Multiplett 2,77-3,10 ($CH_2CO$, CHO), Duplett 7,50 und 7,75 (J 9 Hz ArH), Duplett 9,52 (J 2 Hz, CHO).

IR-Spektrum mit Co-$\gamma$-Strahlung 1685 und 1720 $cm^{-1}$ (Vaselinöl)

UV-Spektrum: $\lambda_{max}$(lg $\varepsilon$): 210 (4,11), 258 (4,25) (95%iges Äthanol)

Analog sind dazu gehörige 4-Formyl-4-(trans-4-alkylzyklohexyl)-1-(4-bromphenyl)-butanone (25.4 b-d) hergestellt, deren Elementaranalyse und Eigenschaften in der Tabelle 6 angegeben sind.

Tabelle 6

| lfd. Nr. | Verbindung | Ausbeute in % | $T_{Schmelz}$ in °C (Hexan) | Gefunden in % C | H | Br |
|---|---|---|---|---|---|---|
| 1 | 2 | 3 | 4 | 5 | 6 | 7 |
| 1. | 25.4.b | 87 | - | 63,51 | 7,25 | 21,24 |
| 2. | 25.4.c | 92 | 72 | 64,69 | 7,39 | 19,41 |
| 3. | 25.4.d | 98 | 57 | 65,27 | 7,74 | 18,46 |

Fortsetzung der Tabelle 6

| lfd. Nr. | Bruttoformel | Berechnet in % C | H | Br |
|---|---|---|---|---|
| 1 | 8 | 9 | 10 | 11 |
| 1. | $C_{20}H_{27}BrO_2$ | 63,33 | 7,17 | 21,06 |
| 2. | $C_{22}H_{31}BrO_2$ | 64,86 | 7,67 | 19,61 |
| 3. | $C_{23}H_{33}BrO_2$ | 65,55 | 7,89 | 18,96 |

25.5. 2-(4-Bromphenyl)-5-(trans-4-butylzyklohexyl)-pyridin (25.5.a)

Man löst 0,005 Mol Verbindung 25.4.a in 15 ml Eisessig auf, setzt 0,006 Mol Triphenylmethylborfluorid zu, kocht innerhalb von 15 Minuten. Das Reaktionsgut wird abgekühlt, mit absolutem Äther verdünnt, der ausgefallene Niederschlag abfiltriert und im Vakuum getrocknet. Man isoliert 2-(4-Bromphenyl)-5-(trans-4-butylzyklohexyl)-pyriliumborfluorid in 35%iger Ausbeute. Die Lösung von 0,003 Mol 2-(4-Bromphenyl)-5-(trans-4-butylzyklohexyl)-pyryliumborfluorid und 0,05 Mol Ammoniumazetat in 30 ml Eisessig wird innerhalb von 2 Stunden gekocht, die Essigsäure im Vakuum abdestilliert. Den Rückstand verdünnt man mit Wasser, extrahiert mit Benzol, wäscht den Extrakt mit Wasser, trocknet über Natriumsulfat und filtriert durch Silikagelschicht (100/160, 3 cm).

Nach der Abdestillation des Lösungsmittels wird der Rückstand aus $CCl_4$ umkristallisiert. Man isoliert eine Verbindung 25.5. a in 92%iger Ausbeute mit $T_{C-S}$ von I55,2 °C, $T_{S-N}$ von 160 °C, $T_{N-I}$ von 196,2 °C.

Gefunden in %: C 67,78; H 7,20; N 3,78; $C_{21}H_{26}BrN$

Berechnet in %: C 67,74; H 7,04; N 3,76.

UV-Spektrum $\lambda_{max}$ (lg $\varepsilon$): 208 (4,35), 257 (4,33), 284 (4,32) (95%iges Äthanol).

$^1$H-NMR-Spektrum ($CCl_4$, $\delta$ ppm): Triplett 0,90 (J 6 Hz, $CH_3$), Multiplett 0,95-2,12 ($CH_2$, CH), Multiplitt 2,32-2,75 (CH-Py), Multiplett 7,43-7,64 (ArH), Duplett 7,83 (J 9 Hz, 2-H, 6-H), Duplett 8,51 (J 2 Hz, 6-H).

Analog sind dazu gehörige 2-(4-Bromphenyl)-5-(trans-4"-alkylzyklohexyl)-pyridine (25.5. b-d) hergestellt, deren Eigenschaften und Elementaranalyse in der Tabelle 7 angegeben sind.

Tabelle 7

| lfd. Nr. | Verbindung | Ausbeute in % | $T_{c-s}$ in °C | $T_{S-N}$ in °C C-N | $T_{N-I}$ in °C |
|---|---|---|---|---|---|
| 1 | 2 | 3 | 4 | 5 | 6 |
| 1. | 25.5.b | 80 | - | I7I,4 | 20I,4 |
| 2. | 25.5.c | 8I | I5I | I64,0 | 200,I |
| 3. | 25.5.d | 87 | I42 | I70,6 | I93,3 |

Fortsetzung der Tabelle 7

| lfd. Nr. | Gefunden in % C | H | N | Bruttoformel | Berechnet in % C | H | N |
|---|---|---|---|---|---|---|---|
| 1 | 7 | 8 | 9 | 10 | 11 | 12 | 13 |
| 1. | 67,16 | 6,90 | 3,71 | $C_{20}H_{24}BrN$ | 67,04 | 6,75 | 3,9 |
| 2. | 68,25 | 7,30 | 3,55 | $C_{22}H_{28}BrN$ | 68,39 | 7,30 | 3,6 |
| 3. | 69,11 | 7,54 | 3,40 | $C_{23}H_{30}BrN$ | 68,99 | 7,55 | 3,5 |

25.6. 2-(4-Zyanophenyl)-5-(trans-4-butylzyklohexyl)-pyridin (III a)

Man vermischt 0,003 Mol Verbindung 25.5. a, 0,004 Mol Kupferzyanid und 10 ml N-Methylpyrrolidon und kocht bis zum Verschwinden der Ausgangsverbindung 25.5. a (etwa 3 Stunden lang). Das Reaktionsgut wird auf Raumtemperatur abgekühlt und in 20 ml wäßrige Ammoniaklösung gegossen. Man extrahiert mit Dichlormethan, wäscht den Extrakt mit Wasser, trocknet über $Na_2SO_4$ und filtriert durch Aluminiumoxid. Das Lösungsmittel wird im Vakuum abgetrieben, der Rückstand aus Alkohol und Azeton umkristallisiert. Man isoliert die Verbindung IIIa in 70%iger Ausbeute mit $T_{C-N}$ von 124 °C und $T_{N-I}$ von 228 °C.

Gefunden in %: N 8,81; $C_{22}H_{26}N_2$. Berechnet in %: N 8,79.

IR-Spektrum: $\gamma_{(CN)}$ mit 2236 $cm^{-1}$ Wellenlänge

$^1$H-NMR-Spektrum ($CCl_4$, $\delta$ ,ppm): Triplett 0,90 (J 6 Hz, $CH_3$), Multiplett 0,95-2,12 ($CH_2$, CH), Multiplett 2,32-2,75 (CH-Py) 7,43-7,65 Ar H), Duplett 7,83 (J 9 Hz, 2-H, 6-H), Singulett 7,60 (3H, 4H), 7,65 (2'-H), 6'-H), 8,08 (3'-H, 5'-H), Duplett 8,53 (6-H), (System AA' BB' ). Analog werden Verbindungen III b-d hergestellt.

Phasenübergangstemperaturen und die Elementaranalyse der Verbindungen der Formel III a-d gibt die Tabelle 8 an.

Tabelle 8

| lfd. Nr. | R | $T_{C-N}$ in °C | $T_{N-I}$ in °C | Gefunden in %, N | Bruttoformel | Berechn. in % N |
|---|---|---|---|---|---|---|
| a | $C_4H_9$ | 124 | 228 | 8,81 | $C_{22}H_{26}N_2$ | 8,79 |
| b | $C_3H_7$ | 123 | 237 | 9,31 | $C_{21}H_{24}N_2$ | 9,20 |
| c | $C_5H_{II}$ | 113 | 228 | 8,36 | $C_{23}H_{28}N_2$ | 8,42 |
| d | $C_6H_{13}$ | 114 | 216 | 8,18 | $C_{24}H_{30}N_2$ | 8,08 |

Vergleichsdaten über Phasenübergangstemperaturen und Koeffizienten der Rotationsviskosität $\gamma_1$ bei den bekannten 2-(4-Zyanophenyl)-5-(4-alkylphenyl)-pyridinen und neuen Verbindungen 2-(4-Zyanophenyl)-5-(trans-4-alkylzyklohexyl)-pyridinen (III a-d) sind in der Tabelle 9 dargestellt.

Koeffizienten der Rotationsviskosität $\gamma_1$ für Homologen 2-(4-Zyanophenyl)-5-(4-alkylphenyl)-pyridine und 2-(4-Zyanophenyl)-5-(trans-4-alkylzyklohexyl)-pyridine werden ausgehend von Werten der entsprechenden binären Mischungen B und D ermittelt.

Mischung B: 10 Masse% 2-(4-Zyanophenyl)-5-(4-alkylphenyl)-pyridin und 90 Masse% Mischung A.

Mischung D: 10 Masse% 2-(4-Zyanophenyl)-5-(trans-4-alkylzyklohexyl)-pyridin und 90 Masse% Mischung A.

Mischung A: 40 Mol.% trans-4-Pentyl-(4-zyanophenyl)-zyklohexan und 60 Mol.% trans-4-Heptyl-(4-zyanophenyl)-zyklohexan $\gamma_1 = 1$ P bei 25 °C.

Tabelle 9

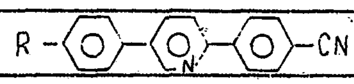

| Nr. | R | $T_{C-N}$ C-S, °C | $T_{S-N}$, °C | $T_{N-I}$, °C | $\gamma_I$, Mischung B P bei 25°C | $\gamma_I$, Homologe P bei 25°C |
|---|---|---|---|---|---|---|
| 1 | 2 | 3 | 4 | 5 | 6 | 7 |
| a | $C_4H_9$ | 93 | - | 223 | 1,450 | 41,00 |
| b | $C_3H_7$ | - | - | - | | - |
| c | $C_5H_{II}$ | 76 | - | 232 | 1,280 | 11,80 |
| d | $C_6H_{13}$ | 72 | 166 | 221 | 1,502 | 58,75 |

Fortsetzung der Tabelle 9

| | | | | |
|---|---|---|---|---|
| | | 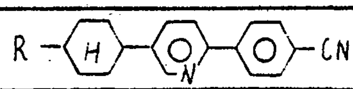 | | (III) |
| Nr. | $T_{C-N}$ °C | $T_{N-I}$ °C | $\gamma_{I}$ D P Mischung bei 25°C | $\gamma_{I}$, Homologe P bei 25°C |
| I | 8 | 9 | 10 | 11 |
| a | 124 | 228 | 1,330 | 17,33 |
| b | 123 | 237 | 1,170 | 4,81 |
| c | 114 | 228 | 1,200 | 6,20 |
| d | 114 | 216 | 1,408 | 30,60 |

Die Tabelle 9 zeigt, daß Klärpunkte $T_{N-I}$ bei neuen Verbindungen der Formel III a-d mit denen der bekannten Verbindungen verglichen sind. Die neuen Verbindungen 2-(4--Zyanophenyl)-5-(trans-4-alkylzyklohexyl)-pyridine weisen dabei bedeutend kleinere Koeffizienten der Rotationsviskosität gegenüber den bekannten Verbindungen 2-(4-Zyanophenyl)-5-(alkylphenyl)-pyridine auf. So hat 2-(4-Zyanophenyl)-5-(trans-4-butylzyklohexyl)-pyridin einen Koeffizienten $\gamma_1$ von 17,33 P und 2-(4-Zyanophenyl)-5-(4-butylphenyl)-pyridin einen Koeffizienten $\gamma_1$ von 41 P bei 25 °C.

Beispiel 26

Herstellung eines flüssigkristallinen Materials unter Verwendung der erfindungsgemäßen Verbindung der Formel III a-d

| | |
|---|---|
| 1,0 g (10 Masse%) | 5 Propyl-2-(4-zyanophenyl)-pyridin |
| 1,5 g (15 Masse%) | 5-Pentyl-2-(4-zyanophenyl)-pyridin |
| 2,0 g (20 Masse%) | 2-Zyano-5-heptylphenylpyridin |
| 1,5 g (15 Masse%) | trans-4'-Propylzyklohexankarbonsäure-4-äthoxyphenylester |
| 1,0 g (10 Masse%) | trans-4'-Butylzyklohexankarbonsäure-4-äthoxyphenylester |
| 1,5 g (15 Masse%) | trans-4 -Hexylzyklohexankarbonsäure-4-äthoxyphenylester |
| 0,5 g (5 Masse%) | 2 [4-(trans-4-Pentylzyklohexyl)-phenyl]-5-ätnylpyridin |
| 1,0 g (10 Masse%) | 2-(4-Zyanophenyl)-5-(trans-4-pentylzyklohexyl)-pyridin (III c) |

bringt man in ein Gefäß ein, rührt innig unter Erhitzen bis zum Auftreten der isotropen Phase um, kühlt ab und erhält das gebrauchsfertige flüssigkristalline Material mit einem Temperaturenbereich von -15 bis 75,1 °C für die nematische Phase und einem Koeffizienten der Rotationsviskosität $\gamma_1$ = 1,47 P bei 25 °C.

Nachstehend gibt die Tabelle 10 Zusammensetzungen des flüssigkristallinen Materials, enthaltend die Verbindungen der Formel III a-d, und Eigenschaften desselben an.

Tabelle 10 

| Beispiel Nr. | Zusammensetzung des flüssigkristallinen Materials gemäß der Erfindung | Masse% | $\gamma_1$, 25°C P | Temperaturenbereich der nematischen Phase |
|---|---|---|---|---|
| 1 | 2 | 3 | 4 | 5 |
| 26. | 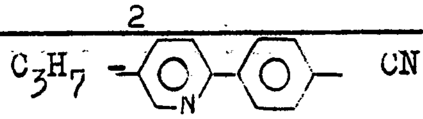 | 10 | 1,47 | -15-75 |
| | 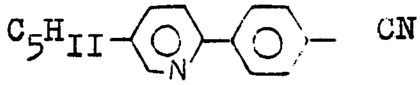 | 15 | | |
| | 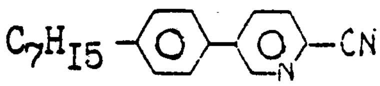 | 20 | | |
| | 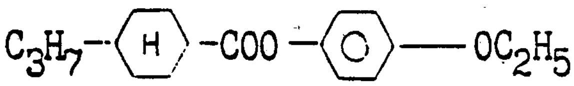 | I5 | | |
| | 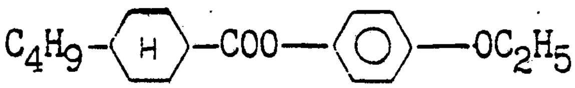 | I0 | | |
| | 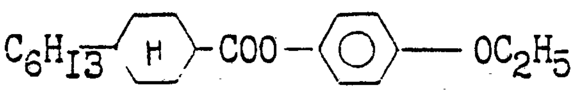 | I5 | | |
| | 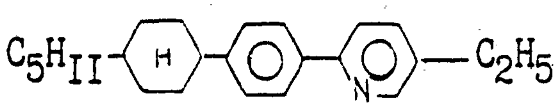 | 5 | | |
| | 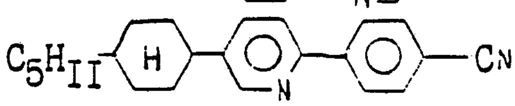 (IIIc) | I0 | | |
| 27. | 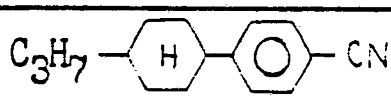 | 5 | I,49 | -I3 -88,7 |
| | 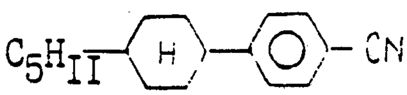 | 5 | | |
| | 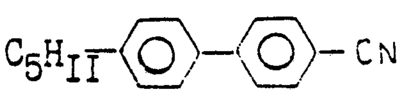 | 5 | | |
| | 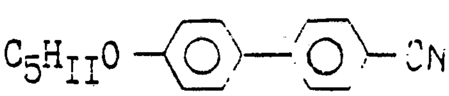 | 5 | | |
| | 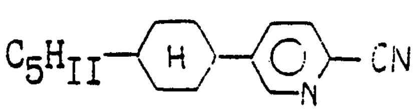 (IId) | 5 | | |
| | 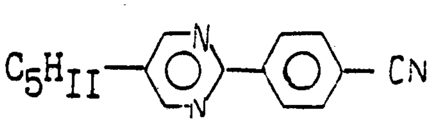 | 5 | | |
| | 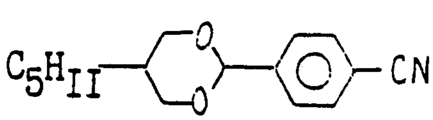 | 5 | | |

Fortsetzung der Tabelle 10

| 1 | 2 | 3 | 4 | 5 |
|---|---|---|---|---|
| | 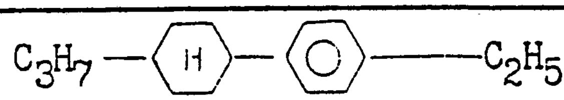 | 5 | | |
| | 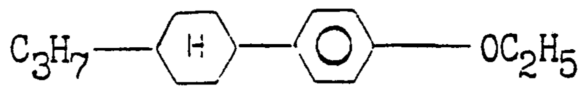 | 10 | | |
| | 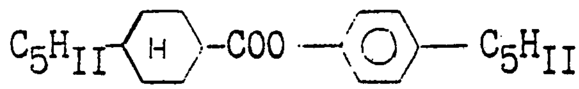 | 5 | | |
| | 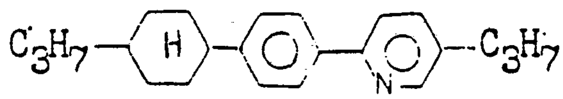 | 10 | | |
| | 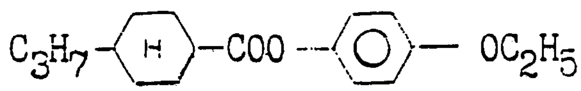 | 10 | | |
| | 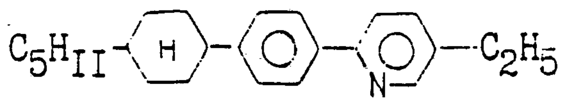 | 10 | | |
| | 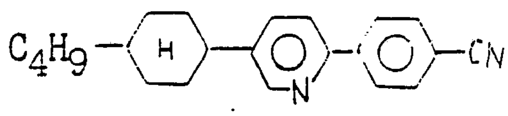 (IIIa) | 15 | | |
| 28. | 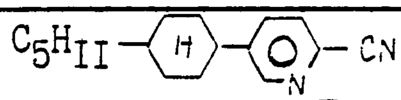 (IId) | 5 | 1,57 | -13-103,7 |
| | 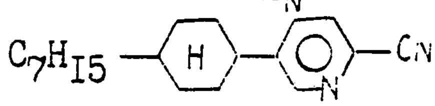 (IIe) | 5 | | |
| | 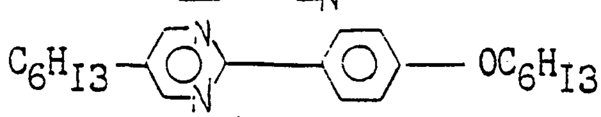 | 5 | | |
| | 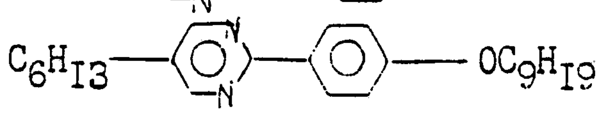 | 5 | | |
| | 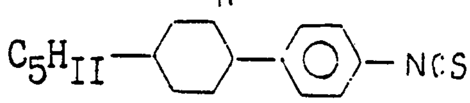 | 5 | | |
| | 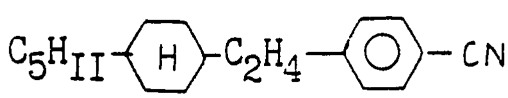 | 5 | | |
| | 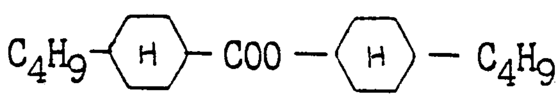 | 10 | | |

Fortsetzung der Tabelle 10

| I | 2 | 3 | 4 | 5 |
|---|---|---|---|---|
| | 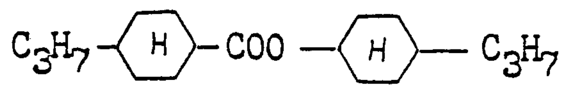 | 10 | | |
| | 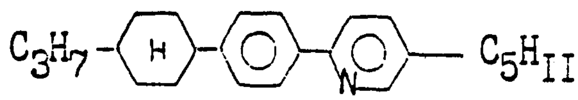 | 5 | | |
| | 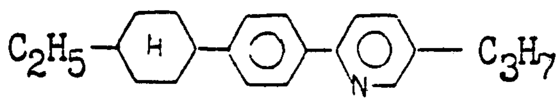 | 5 | | |
| | 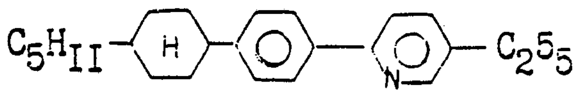 | 5 | | |
| | 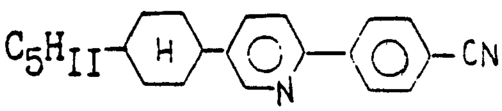 (IIIc) | 20 | | |
| | 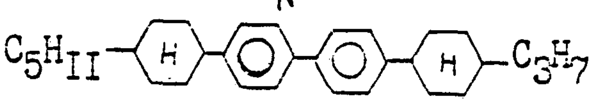 | 5 | | |
| | 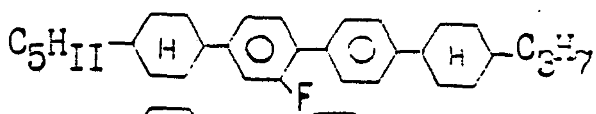 | 5 | | |
| | 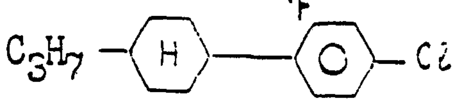 | 5 | | |
| 29. | 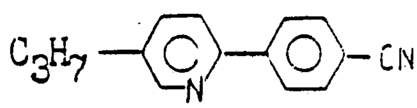 | 10 | 1,86 | -7-96,2 |
| | 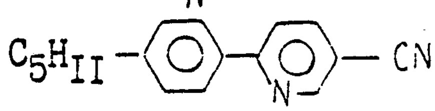 | 15 | | |
| | 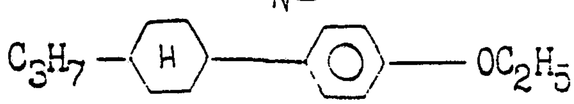 | 10 | | |
| | 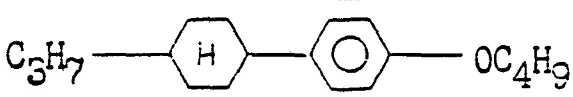 | 10 | | |
| | 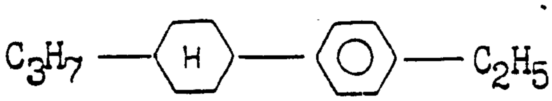 | 5 | | |
| | 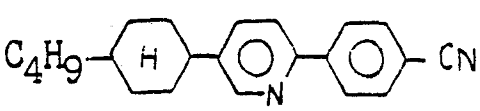 (IIIa) | 20 | | |
| | 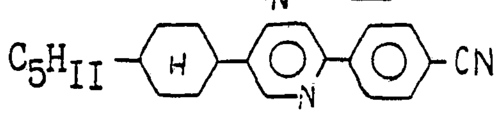 (IIIc) | 10 | | |
| | 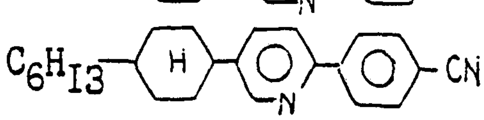 (IIId) | 10 | | |

Fortsetzung der Tabelle 10

| I | 2 | 3 | 4 | 5 |
|---|---|---|---|---|
| | 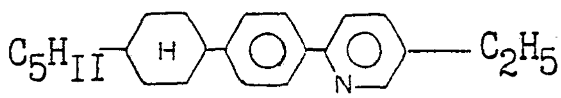 | I0 | | |
| 30. | 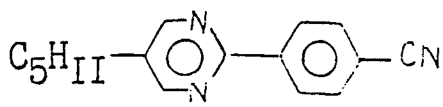 | I5 | I,83 | -9-9I,0 |
| | 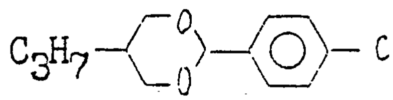 | I0 | | |
| | 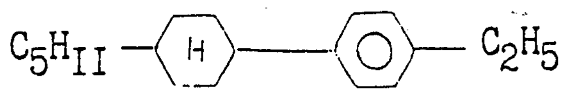 | I0 | | |
| | 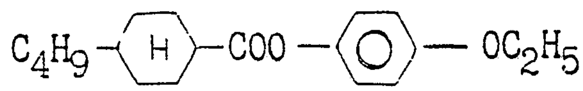 | I0 | | |
| | 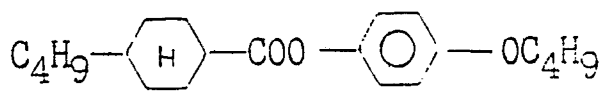 | I0 | | |
| | 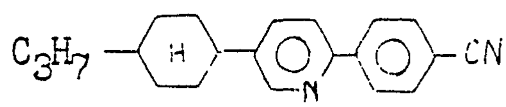 | I0 | | |
| | 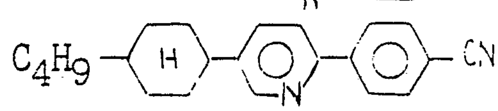 (IIIa) | I0 | | |
| | 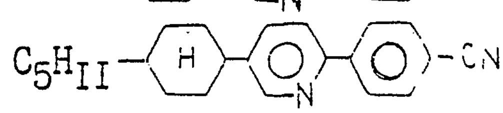 (IIIc) | I0 | | |
| | 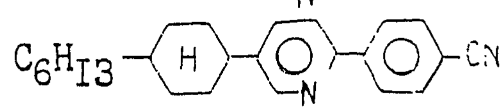 (IIIa) | I0 | | |
| | 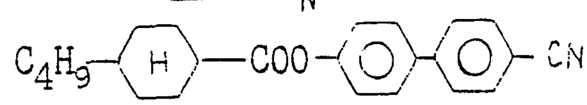 | 5 | | |
| 3I. | 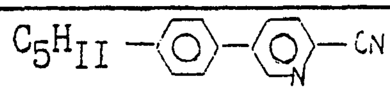 | I0 | 2,06 | -9-84,0 |
| | 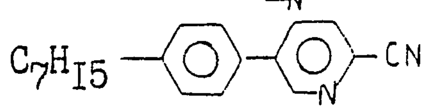 | I5 | | |
| | 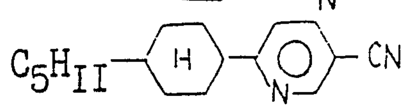 (XXII) | I0 | | |
| | 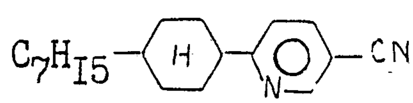 (XXII) | I5 | | |

Fortsetzung der Tabelle 10

| I | 2 | 3 | 4 | 5 |
|---|---|---|---|---|
| | 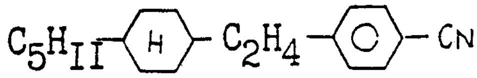 | 5 | | |
| | 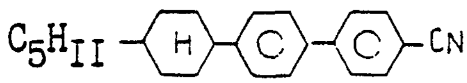 | IO | | |
| | 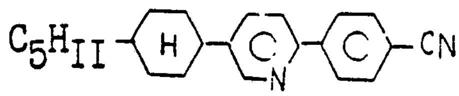 (IIIc) | 5 | | |
| | 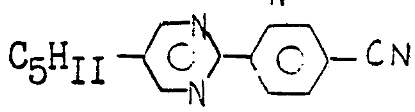 | IO | | |
| | 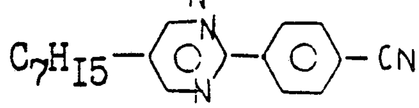 | IO | | |
| | 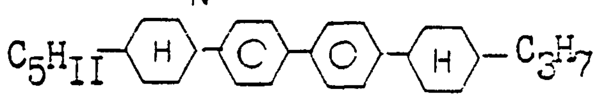 | IO | | |
| 32. | 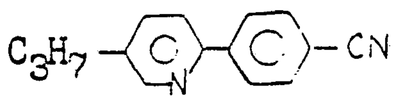 | IO | 2,I5 | -2-IOI,7 |
| | 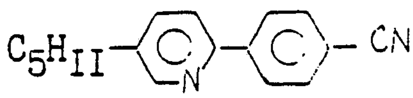 | IO | | |
| | 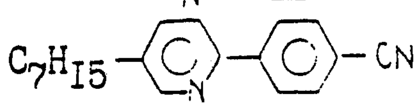 | IO | | |
| | 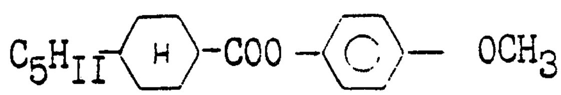 | IO | | |
| | 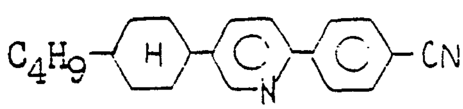 (IIIa) | I5 | | |
| | 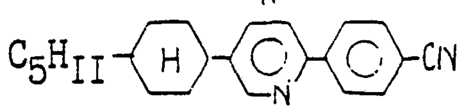 (IIIc) | I5 | | |
| | 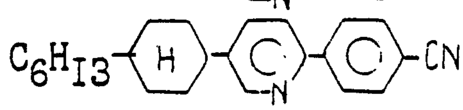 (IIId) | I5 | | |
| | 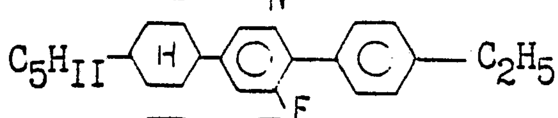 | IO | | |
| | 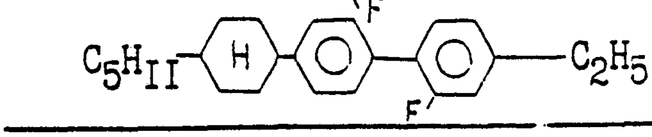 | 5 | | |

Aus der Tabelle 10 ist zu ersehen, daß durch Verwendung neuer Verbindungen der Formel III a-d geringe Koeffizienten der Rotationsviskosität $\gamma_1$ und einen breiten Temperaturenbereich der nematischen Phase bei flüssigkristallinen Materialien erreicht werden. Dies wird es möglich machen, diese Materialien in schnellwirkenden elektrooptischen Einrichtungen zu verwenden.

Beispiel 33

Herstellung von 2- [4-(trans-4-Alkylzyklohexyl)-phenyl] -5-(trans-4-alkylzyklohexyl)-pyridinen (IV)

33.1. 1-(3-Dimethylaminopropionyl)-4 (trans-4-pentylzyklohexyl)-benzol (33.1.d)

Eine Mischung aus 0,13 Mol 4-(trans-4-Pentylzyklohexyl)-azetophenon, 0,22 Mol Paraform, 0,13 Mol Dimethylaminhydrogenchlorid, 0,25 ml konzentrierter Salzsäure, 15 ml absolutem Äthylalkohol wird innerhalb von 8 bis 10 Stunden gesiedet, abgekühlt, mit 100 ml absolutem Azeton verdünnt. Der ausgefallene Niederschlag von 1-(3-Dimethylaminopropionyl)-4-(4-trans-pentylzyklohexyl)-phenylhydrogenchlorid wird abfiltriert, in 200 ml Wasser aufgelöst, mit wäßriger Natriumhydroxidlösung angesäuert und mit Dichlormethan extrahiert. Man trocknet die organische Phase über $Na_2SO_4$ und treibt das Lösungsmittel ab. Man isoliert die Base in 75%iger Ausbeute, welche ohne Analyse zur folgenden Stufe kommt.

33.2. 1-(3-Dimethylaminopropionyl)-4-(trans-4-pentylzyklohexyl)-benzol-jodmethylat (33.2.d)

Man löst 0,11 Mol Verbindung 33.1.d in 110 ml wasserfreiem Dichlormethan auf und setzt bei 10 °C 0,18 Mol Jodmethan zu, läßt das Reaktionsgut für 2 Tage in der Dunkelheit stehen. Der Niederschlag (der Verbindung 33.3.d) wird abfiltriert, mit wasserfreiem $CH_2Cl_2$ gewaschen und an der Luft getrocknet. Die Ausbeute beträgt 98 %. Die Verbindung kommt ohne Analyse zur Stufe 33.3.

33.3. 1-Akryloyl-4-(4-trans-4-pentylzyklohexyl)-benzol (33.3.d)

0,056 Mol Verbindung 33.2.d werden in einem Gemisch aus 110 ml Wasser und 180 ml Dichlormethan suspendiert.

Zur Suspension tropft man innerhalb von 15 Minuten unter innigem Umrühren die Lösung von 3,2 g KOH in 32 ml Wasser und rührt das Reaktionsgut noch 10 Minuten lang um, bis die vollständige Auflösung des Niederschlags erfolgt. Die organische Phase wird abgetrennt, die wäßrige Phase mit 20 ml $CH_2Cl_2$ extrahiert. Die organischen Extrakte werden vereinigt, mit Wasser gewaschen, man gibt Hydrochinon zu, trocknet über $Na_2SO_4$, filtriert durch $SiO_2$ (h = 3 cm), treibt das Lösungsmittel im Vakuum ab. Den hergestellten Rückstand kristallisiert man nicht um, um seine Zersetzung zu vermeiden, und leitet ihn zur folgenden Stufe.

$^1$H-NMR-Spektrum ($CCl_4$, $\delta$, ppm): Triplett 0,86 ($CH_3$), Multiplett 1,0 bis 2,0 ($CH_2$ und CH), Multiplett 2,65-2,78 (Ar-CH), Doppelduplett 5,73 (J 2 Hz und J 10 Hz, $C=CH_2$), Doppelduplett 6,29 (J 2 Hz und J 17 Hz, $C=CH_2$), Doppelduplett 7,03 (J 10 Hz und J 17 Hz, COCH), Duplett 7,14 (J 8 Hz, 3-H, 5-H), Duplett 7,75 (J 3 Hz, 2-H, 6-H).

Analog sind 1-Akryloyl-4(trans-4-alkylzyklohexyl)-benzole (33.3 e,f) hergestellt.

33.4 2-[4-(trans-4-Pentylzyklohexyl)-phenyl]-5-(trans-4-propylzyklohexyl)-pyridin (IV b)

Man vermischt bei einer Temperatur von 0 bis +10 °C 0,0375 Mol Verbindung 33.3. a, 0,034 Mol Verbindung 25.2. b und 15 ml absolutes Äthanol. Das Gemisch wird bei Raumtemperatur innerhalb von 2 Stunden gehalten, auf 0°C abgekühlt, der Niederschlag abfiltriert und mit 10 ml absolutem Äthanol (auf 0 °C vorgekühlt) gewaschen. Man löst das erhaltene Pyran in 90 ml Alkohol, setzt 0,14 Mol Hydroxylammoniumhydrogenchlorid, 10 ml Wasser und 5 ml konzentrierte Salzsäure zu. Das Reaktionsgut wird umgerührt und innerhalb von 8 Stunden beim Sieden erhitzt. Man treibt 2/3 Volumen Lösungsmittel ab, fügt zum Rückstand 150 ml Wasser hinzu und säuert bei einer zwischen 25 und 30 °C liegenden Temperatur auf einen pH-Wert von etwa 8 mit wäßriger Ätznatronlösung an. Der ausgefallene Rückstand wird abfiltriert, mit Wasser gewaschen, an der Luft getrocknet, in Benzol aufgelöst und durch $SiO_2$-Schicht L = 5/40 (h = 3 cm) filtriert. Aus dem Filtrat wird das Lösungsmittel abdestilliert. Der

Rückstand wird aus Azeton oder $CCl_4$ umkristallisiert. Man isoliert 0,24 Mol 2-[-4-(trans-4-Pentylzyklohexyl)-phenyl] -5-(trans-4-propylzyklohexyl)-pyridin in 70%iger Ausbeute.

$^{1}$H-NMR-Spektrum ($CCl_4$, $\delta$, ppm): Triplett 1,03 (J 6 Hz, CH), Multiplett 1,10-2,05 ($CH_2$, CH), Multiplett 2,23-2,63 (CH-A), Doppelduplett 7,08 (J 9 Hz, 3' -HH, 5' -H), Doppelduplett 7,29 (J 8 Hz und J 2 Hz, 4-H), Duplett 7,48 (J 8 Hz, 3-H), Duplett 7,71 (J 9 Hz, 2' -H, 6' -H), Duplett 8,34 (J 2 Hz, 6-H).

Analog sind andere 2- [4-(trans-4-Alkylzyklohexyl)--phenyl] -5-(trans-4-alkylzyklohexyl)-pyridine (IV) hergestellt. Verbindungen der Formel IV sind trans-Isomere. Die $^{1}$H-NMR - Spektren von $^{13}$C der Verbindungen der Formel IV sind mit dem Fourier-Spektrometer WP-80 in dem durch Protonen angeregten vollständigen Spin-Entladungszustand aufgenommen. Die Kohlenstoff-Signale von Zyklohexanringen 33,5 und 34,5 ppm sind doppelt stark. Der Signalverlauf entspricht der trans-Stellung von Substituenten in beiden Zyklohexanringen.

Nachstehend gibt die Tabelle 11 die Formel und die Eigenschaften der Verbindungen IV (a-d) sowie Phasenübergangstemperaturen verglichen mit 4-(trans-4-Alkylzyklohexyl)-4' -(trans-4-alkylzyklohexyl)-biphenylen (SU, A, 1055336) an.

Tabelle 11

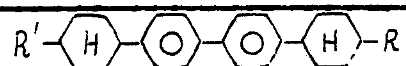

| lfd. Nr. | $R^1$ | R | $T_{C-S}$, in °C | $T_{S-N}$ in °C | $T_{N-I}$ in °C | $\Delta T_N$ in °C |
|---|---|---|---|---|---|---|
| 1 | 2 | 3 | 4 | 5 | 6 | 7 |
| a | $C_2H_5$ | $C_3H_7$ | | | | |
| b | $C_5H_{II}$ | $C_3H_7$ | 58 | 25I | 3II | 60 |
| c | $C_2H_5$ | $C_5H_{II}$ | | | | |
| d | $C_3H_7$ | $C_5H_{II}$ | | | | |
| e | $C_5H_{II}$ | $C_5H_{II}$ | 55 | 267 | 305 | 30 |
| f | $C_3H_7$ | $C_3H_7$ | I55 | 220 | 325 | I05 |

Fortsetzung der Tabelle 11

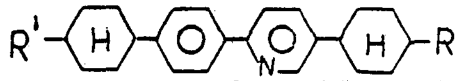 (IVa-g)

| lfd. Nr. | R' | R | $T_{C-S}$, °C | $T_{S-N}$, °C | $T_{N-I}$, °C | $\Delta T_N$, °C |
|---|---|---|---|---|---|---|
| I | 8 | 9 | I0 | II | I2 | I3 |
| a | $C_2H_5$ | $C_3H_7$ | I44 | I9I | 3I5 | I34 |
| b | $C_5H_{II}$ | $C_3H_7$ | 7I | 24I | 3I2 | 7I |
| c | $C_2H_5$ | $C_5H_{II}$ | 52 | 226 | 28I | 55 |
| d | $C_3H_7$ | $C_6H_{II}$ | 86 | 205 | 297 | 92 |
| e | $C_5H_{II}$ | $C_5H_{II}$ | 70 | 252 | 307 | 55 |
| f | $C_3H_7$ | $C_3H_7$ | I44 | 202 | 329 | I27 |
| g | $C_5H_{II}$ | $C_2H_5$ | 50 | 200 | 299 | 99 |

Fortsetzung der Tabelle 11

| lfd. Nr. | Gefunden in % C | Gefunden in % H | Brutto-formel | Berechnet in % C | Berechnet in % H |
|---|---|---|---|---|---|
| I4 | I5 | I6 | I7 | I8 | I9 |
| 69 | 86,30 | I0,37 | $C_{28}H_{39}N$ | 86,3I | I0,08 |
| 59 | 86,5I | I0,43 | $C_{3I}H_{45}N$ | 86,25 | I0,5I |
| 72 | 86,36 | I0,43 | $C_{30}H_{43}N$ | 86,27 | I0,38 |
| 70 | 86,42 | I0,40 | $C_{3I}H_{45}N$ | 86,25 | I0,5I |
| 69 | 86,30 | I0,79 | $C_{33}H_{49}N$ | 86,2I | I0,74 |
| 73 | 86,3I | I0,I7 | $C_{29}H_{4I}N$ | 86,29 | I0,24 |
| 70 | 86,30 | I0,29 | $C_{30}H_{43}N$ | 86,27 | I0,38 |

Aus der Tabelle 11 ist ersichtlich, daß die Temperaturen $T_{S-N}$ des Übergangs "smektisch-nematisch", d.h. die unteren Grenzen der Temperaturenbereiche der nematischen Phase von neuen Verbindungen der Formel IV (a-g) bedeutend geringer als die Temperaturen $T_{S-N}$ der bekannten Verbindungen (SU, A, 1055336) sind. Die Klärpunkte der neuen Verbindungen liegen dabei höher als die der bekannten Verbindungen, d.h. die neuen Verbindungen weisen breitere Bereiche der nematischen Phase als die bekannten Verbindungen auf.

Zusammensetzungen und Eigenschaften der flüssigkreistallinen Materialien einschließlich neuer Pyridinderivate der Formel IV sind in der Tabelle 12 dargestellt.

Tabelle 12

| Beispiel Nr. | Zusammensetzung des erfindungsgemäßen flüssigkristallinen Materials | Masse% | Bereich der nematischen Phase in °C |
|---|---|---|---|
| 1 | 2 | 3 | 4 |
| 34. | 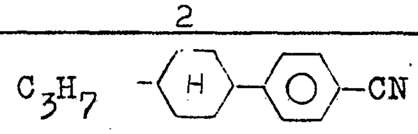 | 20 | -20-85,8 |
| | 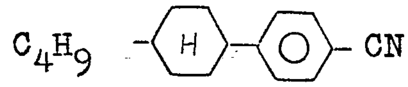 | 16 | |
| | 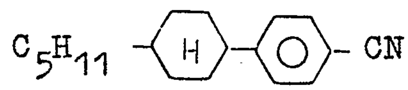 | 22 | |
| 35. | 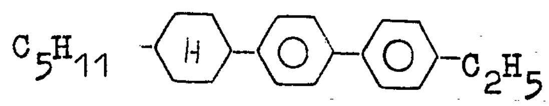 | 25 | |
| | 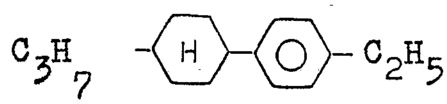 | 7 | |
| | 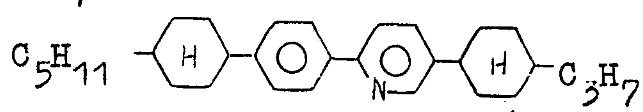 (IVb) | 10 | |
| | 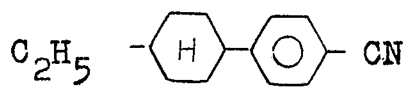 | 16 | -14-66,5 |
| | 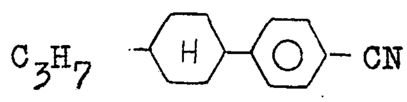 | 22 | |
| | 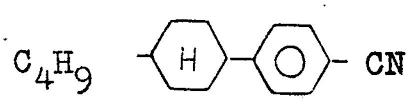 | 17 | |

Fortsetzung der Tabelle 12

| I | 2 | | 3 | 4 |
|---|---|---|---|---|
| | 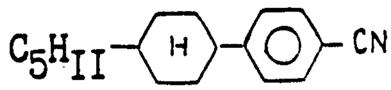 | | 27 | |
| | 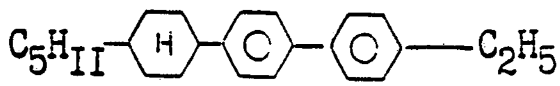 | | 8 | |
| | 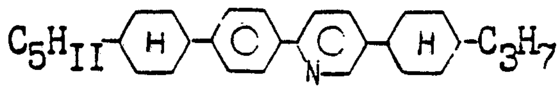 | (IVb) | IO | |
| 36. | 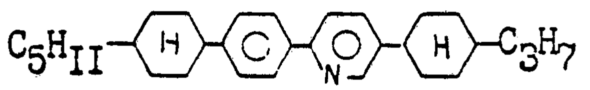 | (IVb) | 5 | -39+IOI,7 |
| | 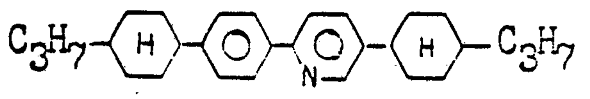 | (IVf) | 6 | |
| | 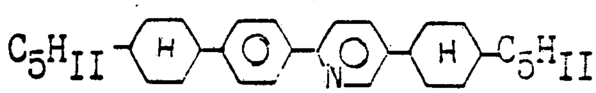 | (IVe) | 5 | |
| | 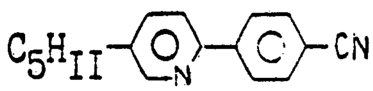 | | IO | |
| | 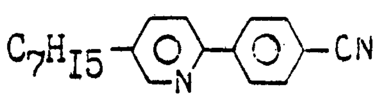 | | IO | |
| | 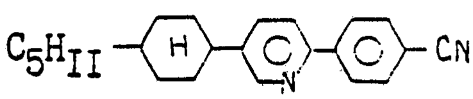 | (IIIc) | IO | |
| | 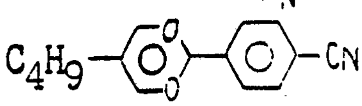 | | IO | |
| | 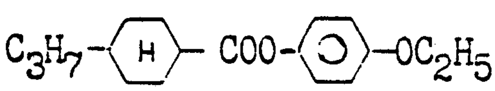 | | IO | |
| | 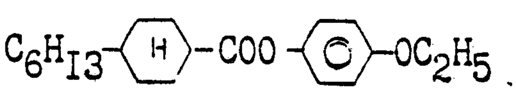 | | IO | |
| | 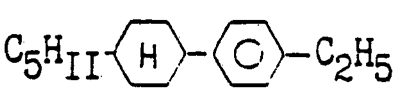 | | 24 | |
| 37. | 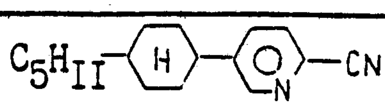 | (IId) | IO | |
| | 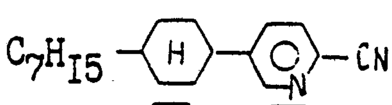 | (IIe) | 7 | |
| | 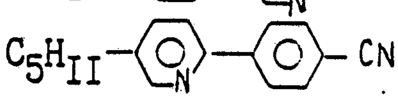 | | 8 | |

Fortsetzung der Tabelle 12

| I | 2 | 3 | 4 |
|---|---|---|---|
| 37. | 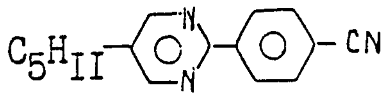 | I0 | |
| | 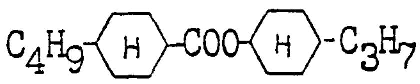 | I0 | |
| | 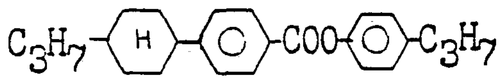 | I0 | |
| | 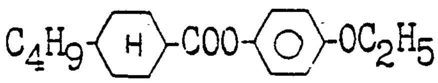 | I0 | |
| | 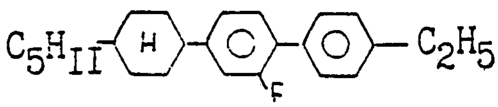 | I0 | |
| | 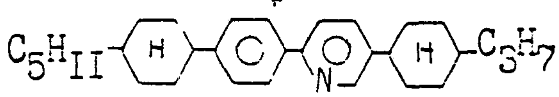 (IVb) | 2 | |
| | 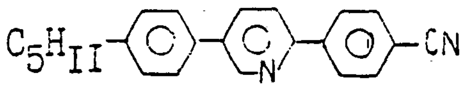 | I0 | |
| | 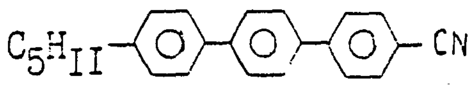 | 3 | |
| | 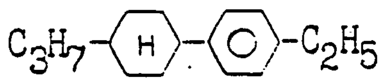 | I0 | |
| 38. | 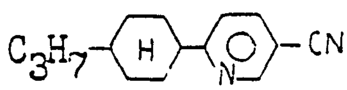 (XXII) | I0 | -I0-I08 |
| | 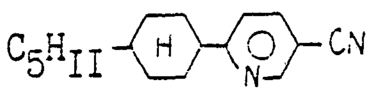 (XXII) | I0 | |
| | 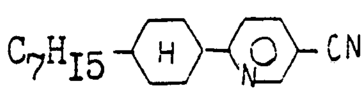 (XXII) | I0 | |
| | 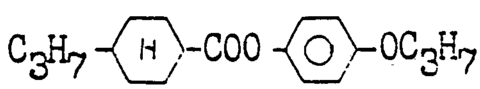 | I7 | |
| | 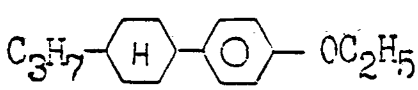 | 23 | |
| | 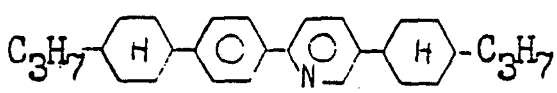 (IVf) | I3 | |

Fortsetzung der Tabelle 12

| I | 2 | 3 | 4 |
|---|---|---|---|
| 38. | 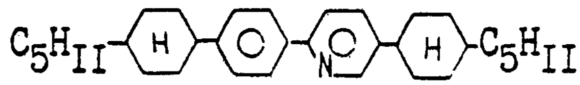 (IVe) | II | |
| | 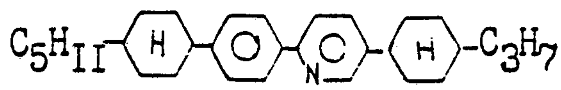 (IVb) | 6 | |
| 39. | 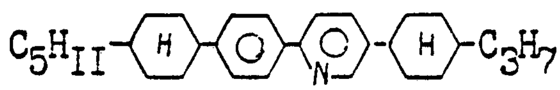 (IVb) | 4 | -I9+II3,3 |
| | 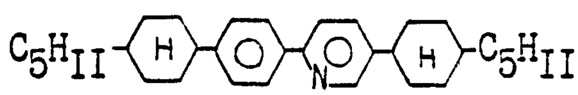 (IVe) | 4 | |
| | 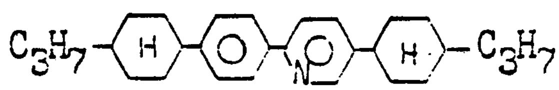 (IVf) | 4 | |
| | 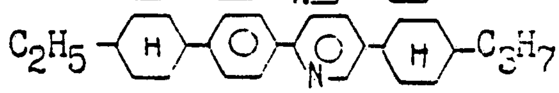 (IVa) | 3 | |
| | 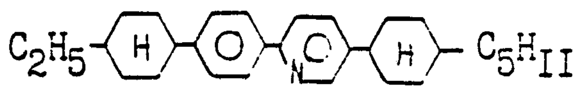 (IVe) | 6 | |
| | 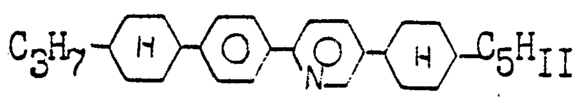 (IVd) | 5 | |
| | 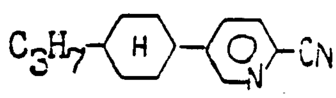 (IIe) | 8 | |
| | 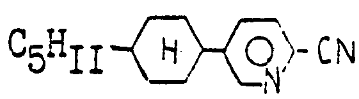 (IId) | I2 | |
| | 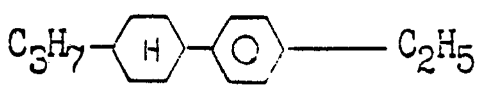 | 20 | |
| | 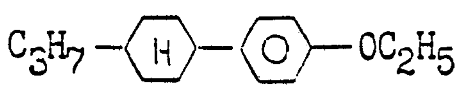 | I0 | |
| | 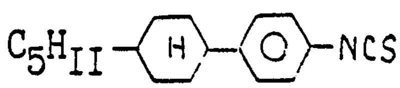 | 5 | |
| | 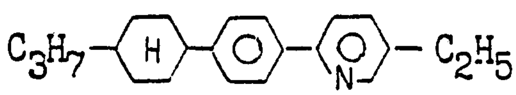 | 5 | |
| | 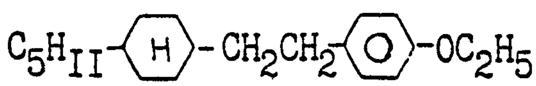 | I4 | |

Fortsetzung der Tabelle 12

| I | 2 | 3 | 4 |
|---|---|---|---|
| 40. | 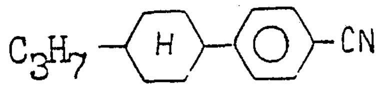 | I0 | -2I-99,5 |
| | 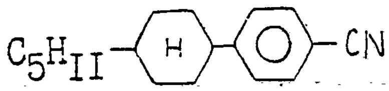 | I0 | |
| | 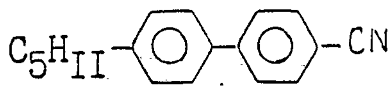 | I0 | |
| | 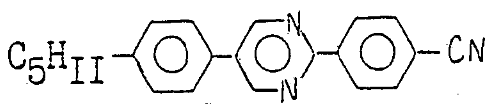 | 5 | |
| | 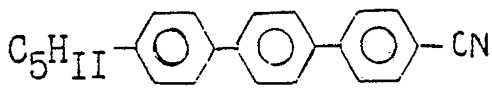 | 5 | |
| | 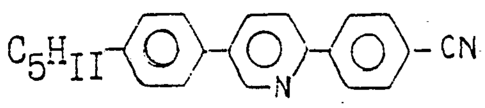 | 5 | |
| | 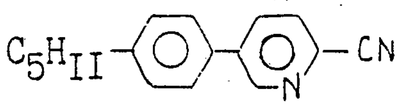 | I0 | |
| | 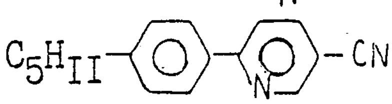 | I0 | |
| | 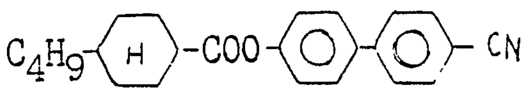 | I0 | |
| | 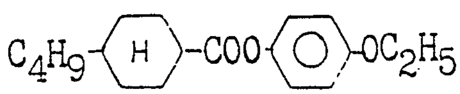 | 5 | |
| | 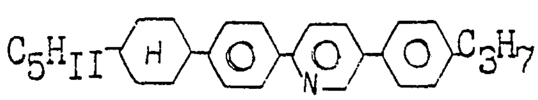 (IVe) | 5 | |
| | 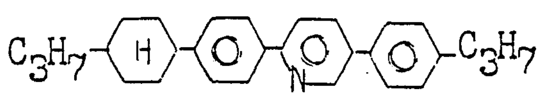 (IVf) | I5 | |
| 4I. | 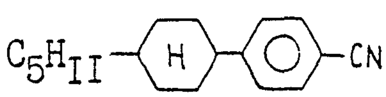 | 23 | -25-85,4 |
| | 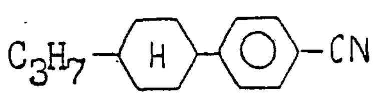 | I7 | |
| | 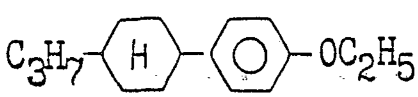 | I6 | |
| | 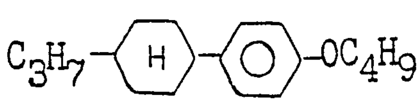 | I2 | |

Fortsetzung der Tabelle 12

| I | 2 | 3 | 4 |
|---|---|---|---|
| | 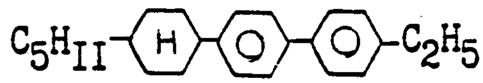 | 22 | |
| | 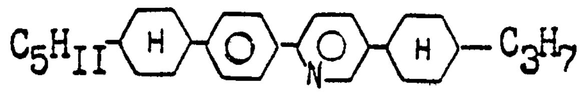 (IVb) | I0 | |
| 42. | 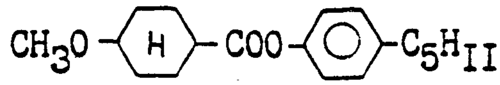 | 28 | -I-70,3 |
| | 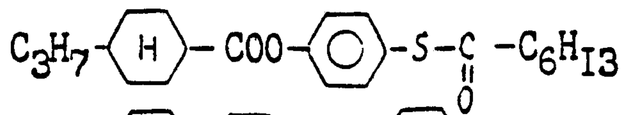 | 22 | |
| | 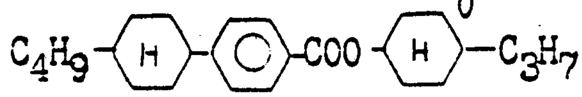 | 20 | |
| | 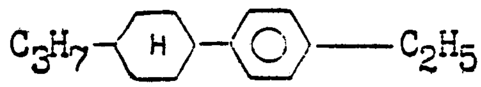 | I9 | |
| 42. | 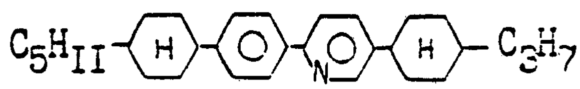 (IVℓ) | II | |
| 43. | 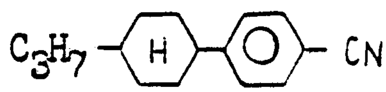 | 24 | |
| | 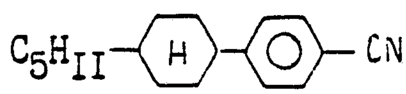 | 26 | 90 |
| | 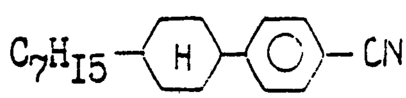 | 25 | |
| | 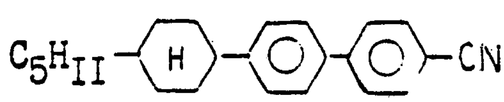 | I5 | |
| | 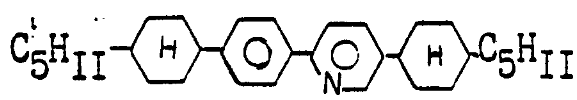 (IVe) | I0 | |
| 44. | 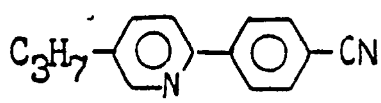 | I0 | -2I-86,6 |
| | 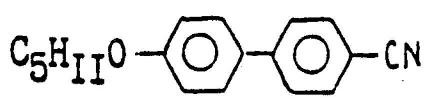 | I0 | |

Fortsetzung der Tabelle 12

| I | 2 | 3 | 4 |
|---|---|---|---|
| | 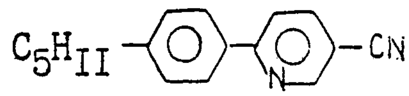 | 10 | |
| | 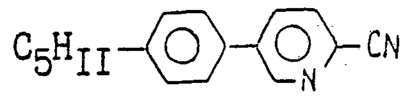 | 10 | |
| | 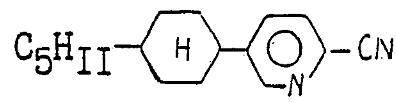 (IId) | 10 | |
| | 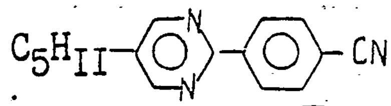 | 10 | |
| | 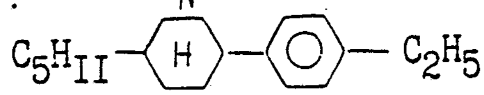 | 10 | |
| | 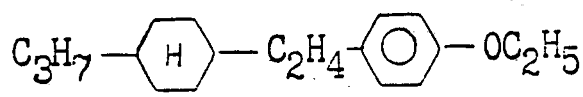 | 10 | |
| | 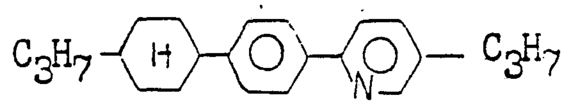 | 10 | |
| | 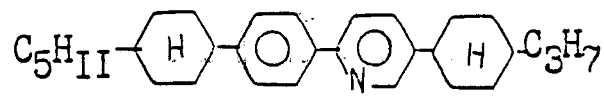 (IVb) | 10 | |
| 45. | 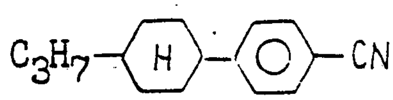 | 19 | -15-60 |
| | 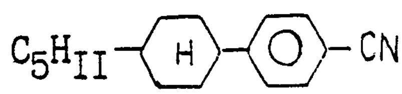 | 18 | |
| | 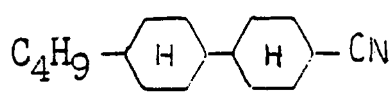 | 23 | |
| | 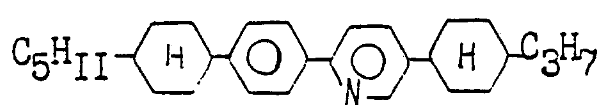 (IVb) | 5 | |
| | 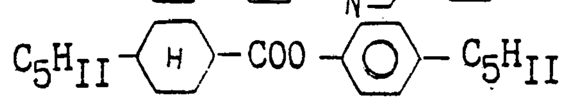 | 19 | |
| | 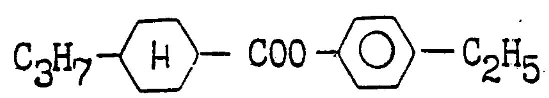 | 16 | |
| 46. | 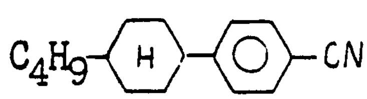 | 20 | -10-84,5 |

Fortsetzung der Tabelle 12

| I | 2 | 3 | 4 |
|---|---|---|---|
| | 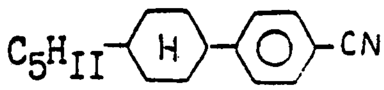 | I8 | |
| | 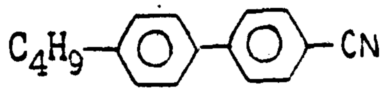 | I7 | |
| | 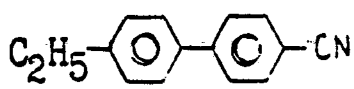 | I3 | |
| | 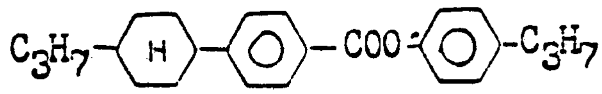 | I2 | |
| | 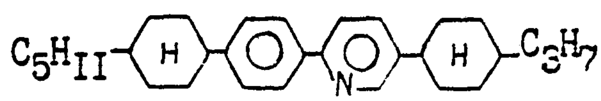 (IVB) | I0 | |
| | 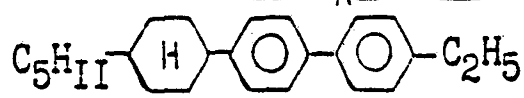 | I0 | |
| 47. | 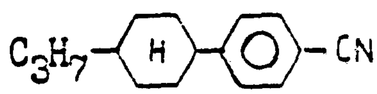 | I | -25-93,3 |
| | 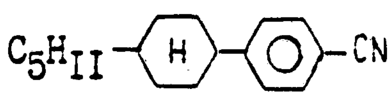 | I4 | |
| | 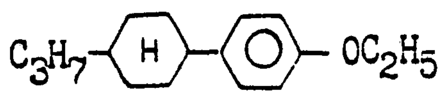 | I6 | |
| | 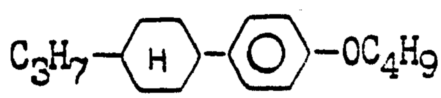 | I2 | |
| | 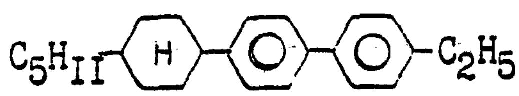 | 22 | |
| | 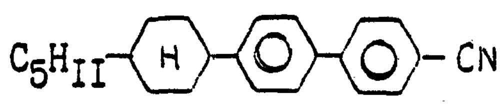 | 5 | |
| | 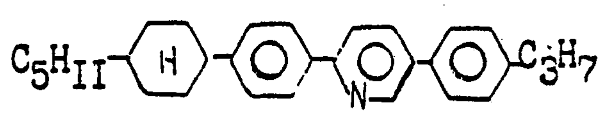 (IVB) | I0 | |
| 48. | 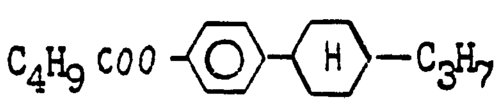 | 49 | -I6-59,3 |
| | 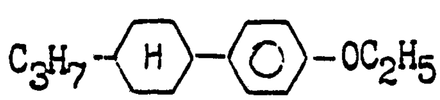 | 2I | |

Fortsetzung der Tabelle 12

| I | 2 | 3 | 4 |
|---|---|---|---|
| | 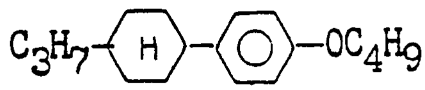 | I8 | |
| | 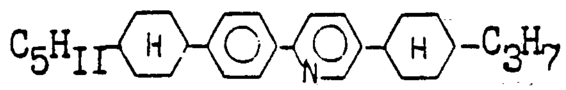 (IVě) | I2 | |
| 49. | 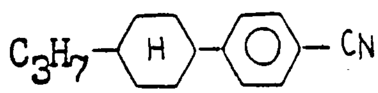 | I0 | -3I 84,I |
| | 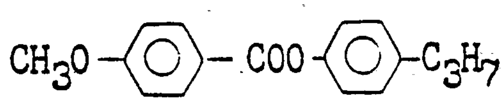 | I0 | |
| | 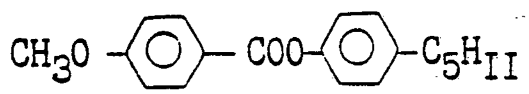 | 20 | |
| | 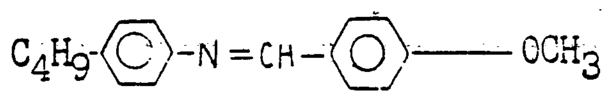 | 23 | |
| | 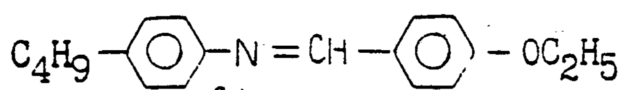 | I2 | |
| | 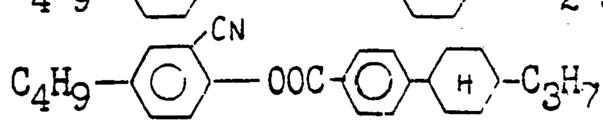 | I0 | |
| | 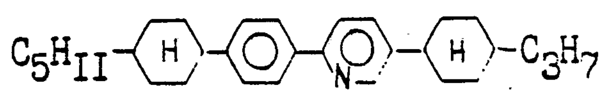 (IOš) | I0 | |
| | 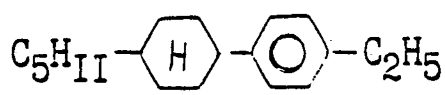 | 5 | |
| 50. | 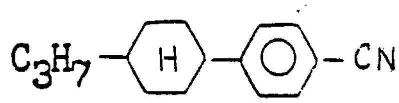 | I3 | -I5 84,3 |
| | 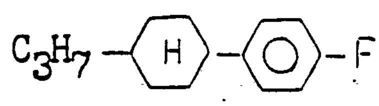 | I0 | |
| | 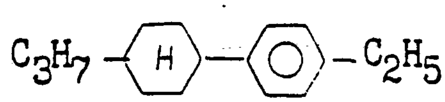 | 20 | |
| | 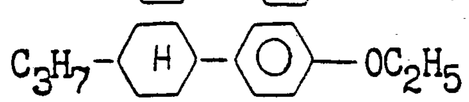 | I0 | |
| | 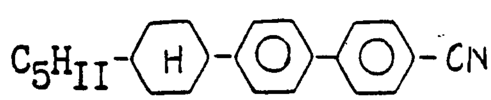 | 9 | |

Fortsetzung der Tabelle 12

| 1 | 2 | 3 | 4 |
|---|---|---|---|
| | 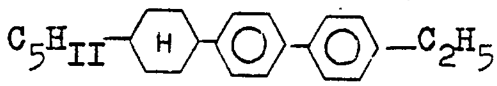 | 20 | |
| | 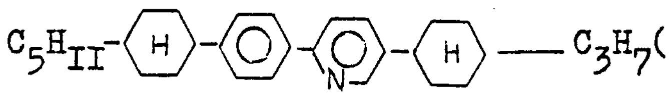 (IVb) | 10 | |
| | 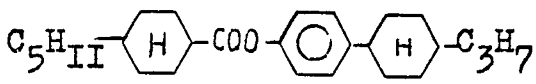 | 8 | |
| 51. | 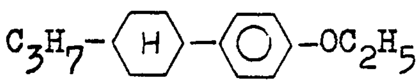 | 15 | -24+ 89,0 |
| | 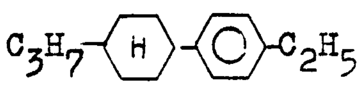 | 10 | |
| | 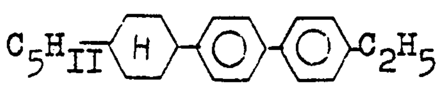 | 20 | |
| | 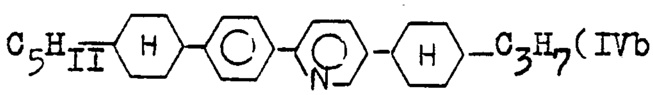 (IVb) | 5 | |
| | 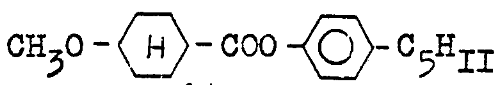 | 18 | |
| | 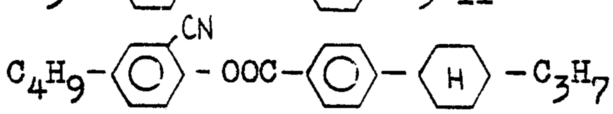 | 22 | |
| | 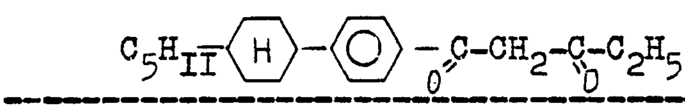 | 10 | |

Die in der Tabelle 12 angegebenen Beispiele für flüssigkristalline Materialien zeigen, daß die Verwendung neuer Pyridinderivate der Formel IV als Komponente der flüssigkristallinen Materialien ermöglicht, flüssigkristalline Materialien mit hohen oberen Grenzen und geringen unteren Grenzen des Temperaturenbereichs der nematischen Phase zu entwickeln, die zur Verwendung in äußeren elektrooptischen Einrichtungen geeignet sind.

Beispiel 52

Herstellung von 2- [4-(trans-4-Alkylzyklohexyl)-3-fluorphenyl] -5-alkylpyridinen (VII)

52.1. 2- [4-(trans-4-Propylzyklohexyl)-3-nitrophenyl]-5-äthylpyridin (V a)

Zu einer auf 0 °C abgekühlten Lösung von 3,07 g (0,01 Mol) 2-[4-(trans-4-Propylzyklohexyl)-phenyl] -5-äthylpyridin in 30 ml konzentrierter Schwefelsäure setzt man langsam 1,01 g (0,01 Mol) Kaliumnitrat zu und rührt das Reaktionsgut bei dieser Temperatur innerhalb von 1 Stunde um, bis die Ausgangsverbindung verschwindet. Das Reaktionsgemisch wird auf 100 g Eis gegossen und mit 20%iger KOH-Lösung bis zum Erreichen eines pH-Wertes von etwa 8 angesäuert. Man extrahiert mit Benzol (2x50 ml), wäscht die organische Phase mit Wasser bis neutral und filtriert durch $SiO_2$-Schicht L = 5/40 (h=2 cm). Die Mutterlauge wird in einem Verdampfer mit darin eingebautem Rotor verdampft, und der Rückstand aus Alkohol umkristallisiert.

Man isoliert 2,53 g (72 %), $R_f$ 0,5 Benzol, $T_{Schmelz}$= = 62 °C $^1H$-NMR-Spektrum ($\delta$, ppm, $CDCl_3$): 0,85 (6H, Triplett $CH_3$); 1,2-1,90 (14 H, verbr. Singulett $CH_2$); 2,62 (2 H, Quadruplett, $CH_2Py$); 7,60 (2 H, verbr. Singulett $H_{\beta\gamma}$ ); 8,30 (1 H, Duplett, $J_m$ 2Hz, $H_2$); 8,48 (1 H Duplett) $J_m$, 2 Hz, H); 8,08 (1 H, Doppelduplett, $J_o$ 8 Hz, $J_m$ 2 Hz, $H_6$); 7,48 (1 H, Doppelduplett $J_m$ 2 Hz, $J_n$ 0,6 Hz, $H_5$).

Gefunden in %: C 75,11; H 8,12; N 8,00; $C_{22}H_{28}N_2O_2$.
Berechnet in %: C 75,07; H 8,01; N 7,95.

Analog sind andere Verbindungen der Formel V b-f hergestellt. Elementaranalysenangaben, $T_{Schmelz}$ und Ausbeuten an Verbindungen V b-f sind in der Tabelle 13 angeführt.

Tabelle 13

| Verbindung V | Ausbeute in % | $T_{Schmelz}$ in °C | Gefunden in % C | H | N |
|---|---|---|---|---|---|
| 1 | 2 | 3 | 4 | 5 | 6 |
| b | 74 | 61 | 75,80 | 8,76 | 7,20 |
| c | 79 | 53 | 76,19 | 8,70 | 7,12 |
| d | 75 | 57 | 76,20 | 8,59 | 7,13 |
| e | 70 | 45 | 75,12 | 8,23 | 8,10 |
| f | 76 | 41 | 75,91 | 8,70 | 7,41 |

Fortsetzung der Tabelle 13

| Verbindung V | Bruttoformel | Berechnet in % C | H | N |
|---|---|---|---|---|
| 1 | 7 | 8 | 9 | 10 |
| b | $C_{24}H_{32}N_2O_2$ | 75,86 | 8,55 | 7,35 |
| c | $C_{25}H_{34}N_2O_2$ | 76,23 | 8,68 | 7,10 |
| d | $C_{25}H_{34}N_2O_2$ | 76,23 | 8,68 | 7,10 |
| e | $C_{22}H_{28}N_2O_2$ | 75,07 | 8,01 | 7,95 |
| f | $C_{24}H_{32}N_2O_2$ | 75,86 | 8,55 | 7,36 |

52.2 2-[4-(trans-4-Propylzyklohexyl)-3-aminophenyl]-5-äthylpyridin (VI. a)

Zu einer Lösung von 3,52 g (0,01 Mol) Verbindung V.a in 30 ml Alkohol werden 1 g Ni/Re, 4 ml 64%iges Hydrazin und 0,1 g NaOH hinzugefügt und zum Sieden erhitzt.

Die Vollendung der Reaktion wird chromatografisch am Verschwinden des rohen Nitroderivats 52.1. a (etwa 1 Stunde) überwacht. Man filtriert das Reaktionsgemisch, wäscht am Filter mit Alkohol und kühlt ab. Man isoliert 2,52 g (78 %) Verbindung VI. a, $T_{S-N}$ 86 °C, $T_{N-I}$ 127 °C.

Gefunden in %: C 82,3; H 9,46; N 8,54; $C_{22}H_{30}N_2$.
Berechnet in %: C 82,06; H 9,38; N 8,69.

Analog sind andere Verbindunge VI. b-f hergestellt. Elementaranalysenangaben, Phasenübergangstemperaturen und Ausbeuten an Verbindungen VI. b-f sind in der Tabelle 14

dargestellt.

Tabelle 14

| Verbindung VI | Ausbeute in % | $T_{S-N}$, in °C | $T_{N-I}$ in °C | Gefunden in % C | H | N |
|---|---|---|---|---|---|---|
| 1 | 2 | 3 | 4 | 5 | 6 | 7 |
| b | 76 | 80 | I28 | 82,I6 | 9,80 | 8,00 |
| c | 79 | I02 | I46 | 92,20 | 9,8I | 7,5 |
| d | 74 | II6 | I47 | 82,4I | 9,93 | 7,5I |
| e | 76 | 69 | I05 | 82,20 | 9,25 | 8,70 |
| f | 78 | 60 | II3 | 82,3I | 9,64 | 7,96 |

Fortsetzung der Tabelle 14

| Verbindungen VI | Bruttoformel | Gefunden in % C | H | N |
|---|---|---|---|---|
| 1 | 8 | 9 | 10 | 11 |
| b | $C_{24}H_{34}N_2$ | 82,23 | 9,78 | 7,9 |
| c | $C_{25}H_{36}N_2$ | 82,36 | 9,95 | 7,6 |
| d | $C_{25}H_{36}N_2$ | 82,36 | 9,95 | 7,68 |
| e | $C_{22}H_{30}N_2$ | 82,06 | 9,38 | 8,69 |
| f | $C_{24}H_{34}N_2$ | 82,23 | 9,78 | 7,99 |

52.3. 2-[4-(trans-4-Propylzyklohexyl)-3-fluorphenyl]-5-äthylpyridin (VII a)

Zu einer Suspension von 3,22 g (0,01 Mol) Verbindung VI.a in 30 ml $HBF_4$ tropft man bei einer zwischen 0 und 5 °C liegenden Temperatur eine Lösung von 0,69 g (0,01 Mol) $NaNO_2$ in 3 ml Wasser langsam unter Umrühren zu. Man rührt noch 1 Stunde lang bei dieser Temperatur um und filtriert dann das Reaktionsgut, wäscht mit 10 ml abgekühltem Äthylalkohol, trocknet am Filter an und wäscht mit absolutem Äther. Man bringt den trockenen Niederschlag in einen Einhalskolben mit 100 ml darin befindlichem absolutem Toluol ein und siedet das Gemisch innerhalb von 1 Stunde. Dann kühlt man ab und wäscht die Toluollösung mit gesättigter wäßriger Sodalösung und Wasser und filtriert

die organische Phase durch $SiO_2$-Schicht L 5/40 (h = 2 cm). Das Lösungsmittel wird in einem Verdampfer mit dem darin eingebauten Rotor verdampft. Der Rückstand wird aus Azeton umkristallisiert und chromatografiert. Man isoliert 0,88 g (27 %) Verbindung VII. a mit $T_{C-N}$ = 60,2 °C, $T_{N-I}$ = 145,1 °C.

Analog werden andere Verbindungen VII b-f hergestellt. Phasenübergangstemperaturen und Elementaranalysenangaben der Verbindungen VII a-f sind in der Tabelle 15 dargestellt.

Vergleichswerte von Phasenübergangstemperaturen und Temperaturbereichen der nematischen Phase $\Delta T_N$ für die bekannten, 2-[4-(trans-4-Alkylzyklohexyl)-3-fluorphenyl]-5-alkylpyridine (VII a-f) sind in der Tabelle 16 gegenübergestellt.

Tabelle 15

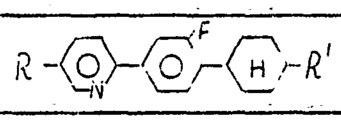

(VII a-f)

| Verbindung VII | R | R' | $T_{C-N}$, in °C | $T_{N-I}$, in °C | Gefunden in % C | H | N |
|---|---|---|---|---|---|---|---|
| 1 | 2 | 3 | 4 | 5 | 6 | 7 | 8 |
| a | $C_2H_5$ | $C_3H_7$ | 60,2 | I45,I | 8I,24 | 8,70 | 4,I9 |
| b | $C_2H_5$ | $C_5H_{II}$ | 60,0 | I42,0 | 8I,36 | 9,23 | 4,00 |
| c | $C_5H_{II}$ | $C_3H_7$ | 52,9 | I44,6 | 8I,73 | 9,20 | 3,66 |
| d | $C_3H_7$ | $C_5H_{II}$ | 66,0 | I5I,0 | 8I,65 | 9,40 | 3,90 |
| e | $C_3H_7$ | $C_2H_5$ | 47,6 | I27,5 | 8I,24 | 8,59 | 4,28 |
| f | $C_5H_{II}$ | $C_2H_5$ | 39,5 | II9,5 | 8I,60 | 9,23 | 3,78 |

Fortsetzung der Tabelle 15

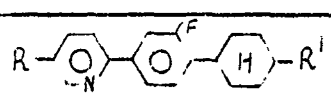

(VII a-f)

| Verbindung VII | Bruttoformel | Berechnet in % C | H | N | Ausbeute in % |
|---|---|---|---|---|---|
| 1 | 9 | 10 | 11 | 12 | 13 |
| a | $C_{22}H_{28}NF$ | 8I,I9 | 8,67 | 4,30 | 27 |
| b | $C_{24}H_{32}NF$ | 8I,54 | 9,I2 | 3,96 | 25 |
| c | $C_{25}H_{34}NF$ | 8I,70 | 9,32 | 3,8I | 25 |
| d | $C_{25}H_{34}NF$ | 8I,70 | 9,72 | 3,8I | 2I |
| e | $C_{22}H_{28}NF$ | 8I,54 | 8,67 | 4,30 | I8 |
| f | $C_{24}H_{32}NF$ | 8I,54 | 9,I2 | 3,96 | I7 |

Tabelle 16

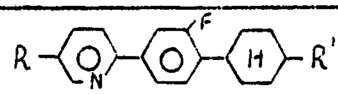

| lfd. Nr. | R | R' | $T_{c-s}$, C-N in °C | $T_{S-N}$, in °C | $T_{N-I}$, in °C | $\Delta T_N$ in °C |
|---|---|---|---|---|---|---|
| 1 | 2 | 3 | 4 | 5 | 6 | 7 |
| a | $C_2H_5$ | $C_3H_7$ | 50,8 | 87,7 | I74,4 | 86,4 |
| b | $C_2H_5$ | $C_5H_{II}$ | 65,8 | 78,0 | I68,0 | 90,0 |
| c | $C_5H_{II}$ | $C_3H_7$ | 48,0 | 93,8 | I77,4 | 83,6 |
| d | $C_3H_7$ | $C_5H_{II}$ | 37,0 | 93,5 | I79,0 | 85,5 |
| e | $C_3H_7$ | $C_2H_5$ | 5I,9 | 92,7 | I58,8 | 66,I |
| f | $C_5H_{II}$ | $C_2H_5$ | 40,2 | 84,6 | I53,I | 68,5 |

Fortsetzung der Tabelle 16

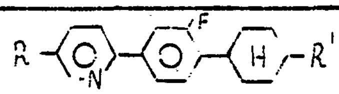
(VII)

| Nr. | $T_{C-N}$ in °C | $T_{N-I}$ in °C | $\Delta T_N$ in °C |
|---|---|---|---|
| 1 | 8 | 9 | 10 |
| a | 60,2 | I45,I | 84,9 |
| b | 60,0 | I42,0 | 82,0 |
| c | 52,9 | I44,6 | 9I,7 |
| d | 66,0 | I5I,0 | 85,0 |
| e | 47,6 | I27,5 | 79,9 |
| f | 39,5 | II9,5 | 80,0 |

Tabelle 16 zeigt, daß die Verbindungen VII a-f keine smektische Mesophase enthalten und der Temperaturenbereich der nematischen Phase $\Delta T_N$ bei den neuen Verbindungen VII a-f breiter als $\Delta T_N$ bei den bekannten Verbindungen ist.

Tabelle 17 gibt die Zusammensetzung und die Eigenschaften von flüssigkristallinen Materialien, welche neue Pyridinderivate der Formel VII enthalten, an.

Tabelle 17

| Beispiel Nr. | Zusammensetzung der erfindungsgemäßen flüssigkristallinen Materialien | Masse% | Temperaturbereich der nematischen Phase in °C |
|---|---|---|---|
| 1 | 2 | 3 | 4 |
| 53. | $C_5H_{11}$ | 90 | 11-47,6 |
| | $C_5H_{11}$ (VIIb) | 10 | |
| 54. | $C_3H_7$ | 17 | -30- 81,5 |
| | 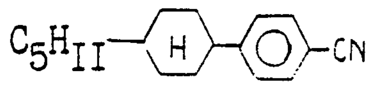 | 23 | |
| | 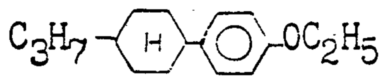 | 16 | |
| | 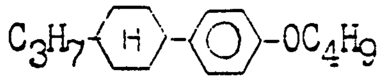 | 12 | |
| | 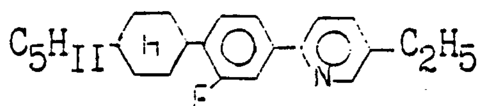 (VIIe) | 22 | |
| | 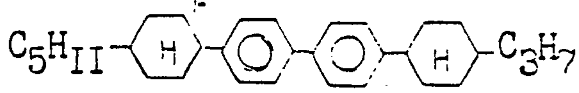 | 10 | |
| 55. | 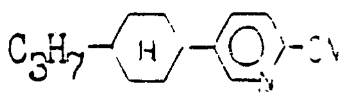 (II[illegible]) | 5 | -26-82,7 |
| | 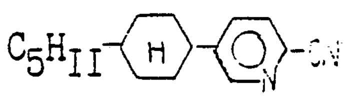 (IIa) | 5 | |
| | 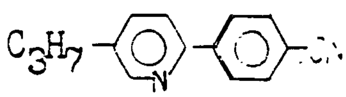 | 5 | |
| | 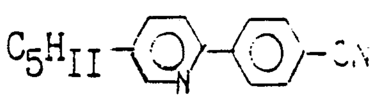 | 5 | |
| | 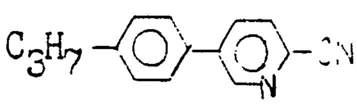 | 5 | |
| | 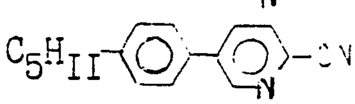 | 5 | |
| | 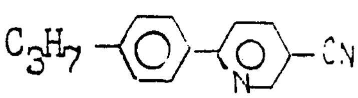 | 5 | |
| | 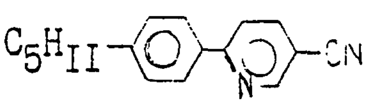 | 5 | |
| | 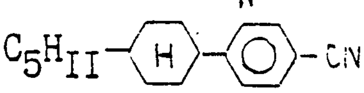 | 5 | |
| | 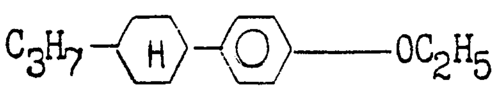 | 10 | |
| | 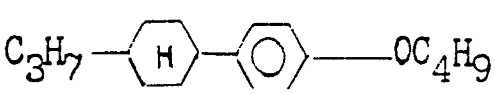 | 10 | |

Fortsetzung der Tabelle 17

| I | 2 | | 3 | 4 |
|---|---|---|---|---|
| 55. | 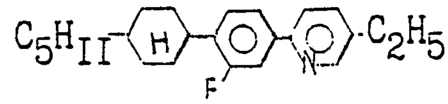 | (VIIb) | 9 | |
| | 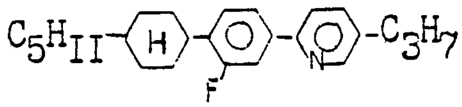 | (VIId) | 9 | |
| | 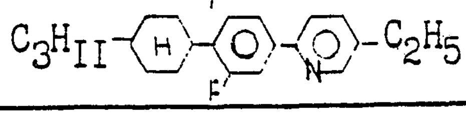 | (VIIa) | 9 | |
| 56. | 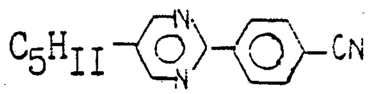 | | 10 | -29-76,7 |
| | 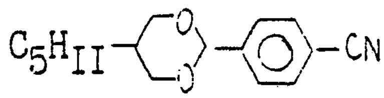 | | 10 | |
| | 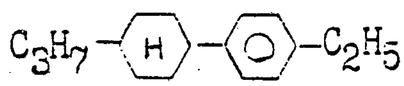 | | 15 | |
| | 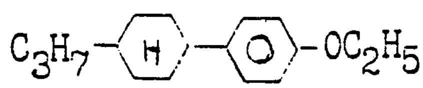 | | 5 | |
| | 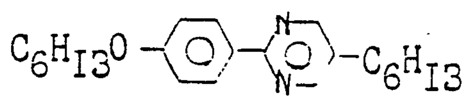 | | 5 | |
| | 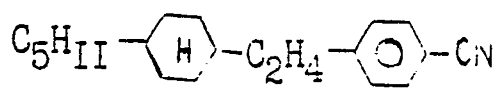 | | 5 | |
| | 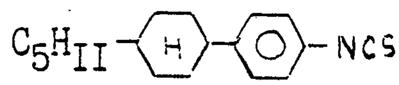 | | 5 | |
| | 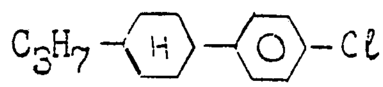 | | 5 | |
| | 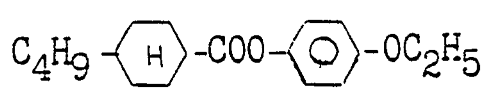 | | 5 | |
| | 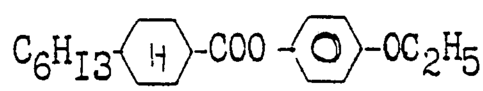 | | 5 | |
| | 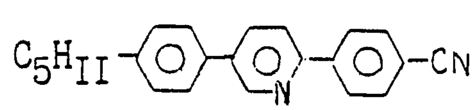 | | 5 | |
| | 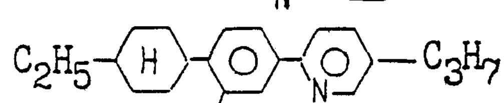 | (VIIe) | 10 | |
| | 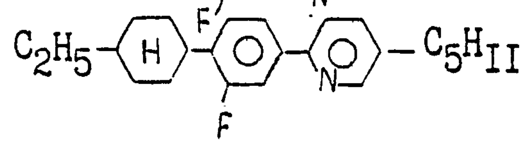 | (VIIf) | 10 | |

Fortsetzung der Tabelle 17

| I | 2 | 3 | 4 |
|---|---|---|---|
| 56. | 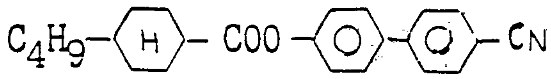 | 5 | |
| 57. | 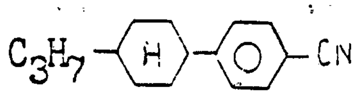 | I4 | -27-95,8 |
| | 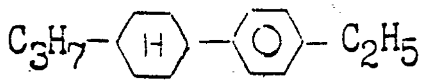 | I0 | |
| | 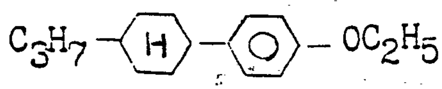 | I0 | |
| | 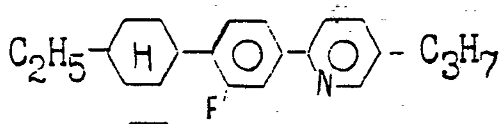 (VIIe) | 9 | |
| | 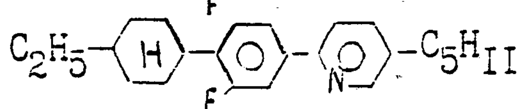 (VIIf) | 8 | |
| | 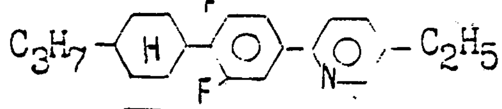 (VIIa) | 9 | |
| | 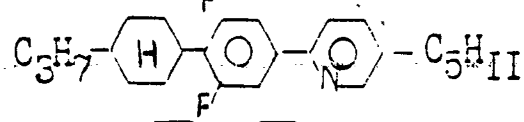 (VIIe) | 8 | |
| | 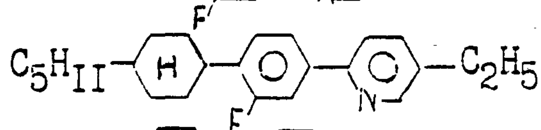 (VIIc) | 8 | |
| | 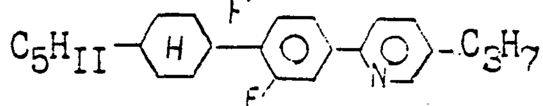 (VIId) | 8 | |
| | 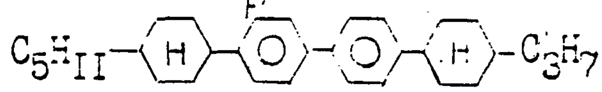 (IV) | I0 | |
| | 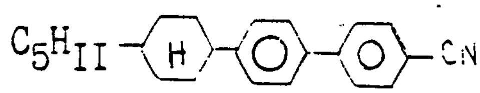 | 3 | |
| | 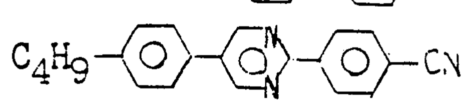 | 3 | |
| 58. | 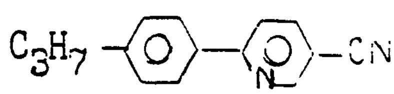 | I0 | -8-87,8 |
| | 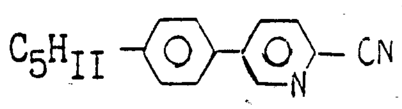 | I5 | |

Fortsetzung der Tabelle 17

| I | 2 | 3 | 4 |
|---|---|---|---|
| 58. | 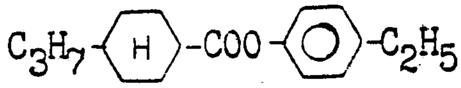 | 10 | |
| | 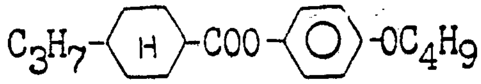 | 10 | |
| | 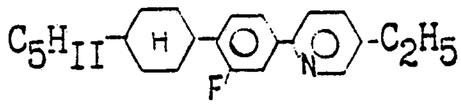 (VIIb) | 10 | |
| | 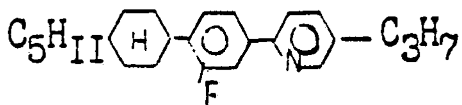 (VIId) | 9 | |
| | 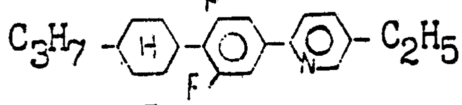 (VIIa) | 9 | |
| | 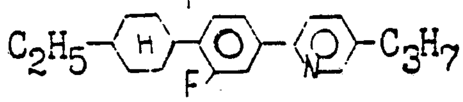 (VIIe) | 9 | |
| | 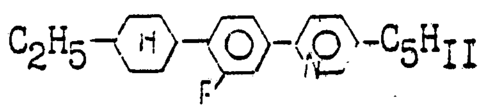 (II f) | 9 | |
| | 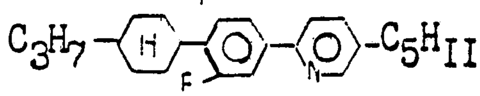 (VIIe) | 9 | |
| 59. | 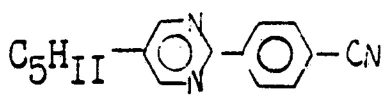 | 10 | -29-34,3 |
| | 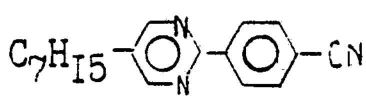 | 15 | |
| | 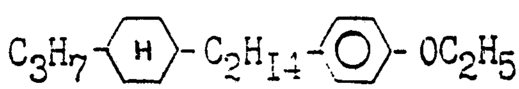 | 10 | |
| | 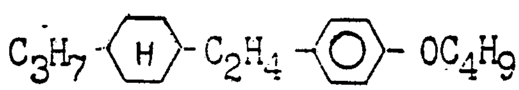 | 5 | |
| | 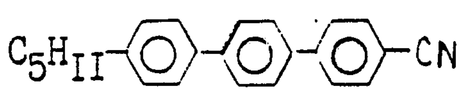 | 5 | |
| | 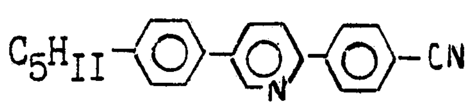 | 5 | |
| | 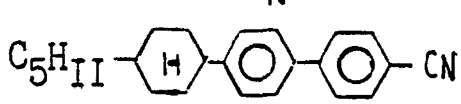 | 5 | |
| | 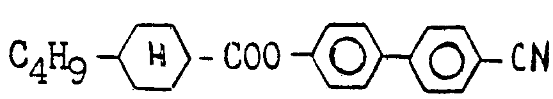 | 5 | |

Fortsetzung der Tabelle 17

| I | 2 | 3 | 4 |
|---|---|---|---|
| 59. | 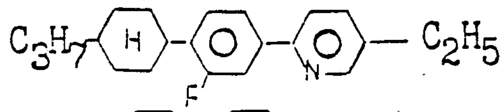 (VIIa) | 5 | |
| | 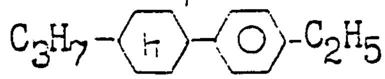 | I5 | |
| | 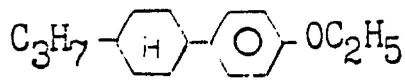 | I0 | |
| | 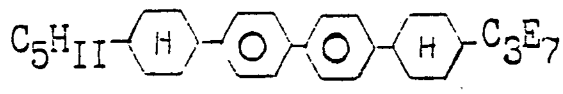 (IV) | I0 | |
| 60. | 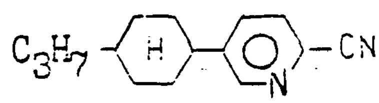 | I5 | -9-79,I |
| | 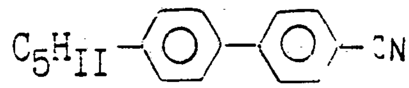 | 5 | |
| | 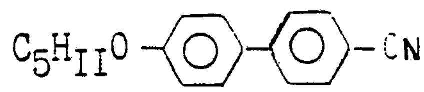 | 5 | |
| | 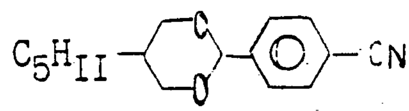 | I0 | |
| | 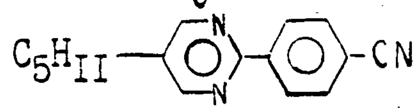 | I0 | |
| | 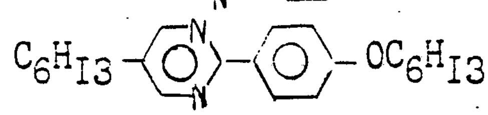 | I0 | |
| | 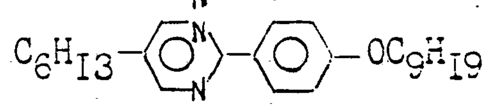 | I0 | |
| | 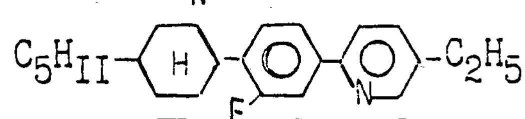 (VIIб) | 2 | |
| | 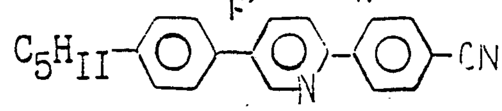 | I0 | |
| | 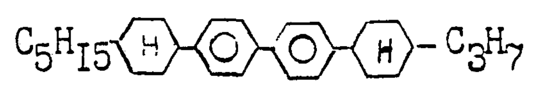 (IV) | I0 | |
| | 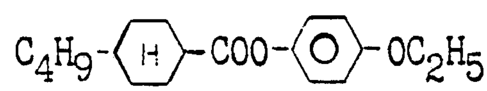 | I3 | |

Fortsetzung der Tabelle 17

| I | 2 | 3 | 4 |
|---|---|---|---|
| 61. | $C_3H_7$ 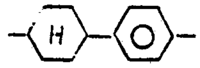 CN | 8 | -38÷ 84,7 |
| | $C_5H_{II}$ 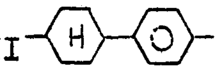 CN | II | |
| | $C_3H_7$ 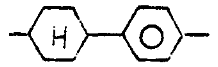 $OC_2H_5$ | IO | |
| | $C_3H_7$ 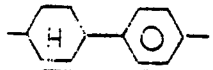 $C_2H_5$ | 25 | |
| | $C_3H_7$ 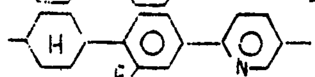 $C_2H_5$ (VIIa) | IO | |
| | $C_2H_5$ 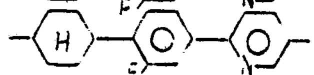 $C_3H_7$ (VIIe) | IO | |
| | $C_5H_{II}$ 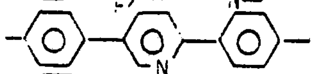 CN | 8 | |
| | $C_5H_{II}$ 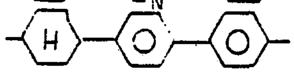 CN | 8 | |
| | $C_5H_{II}$ 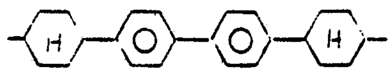 $C_3H_7$ (IV) | IO | |

Wie aus der Tabelle 17 zu ersehen ist, macht die Verwendung neuer Pyridinderivate der Formel VII als Komponenten der flüssigkristallinen Materialien möglich, flüssigkristalline Materialien mit geringen unteren Grenzen und hohen oberen Grenzen der Temperaturenbereiche der nematischen Phase, die zur Verwendung in äusseren elektrooptischen Einrichtungen geeignet sind, herzustellen.

Beispiel 62

Herstellung von 2-[4-(trans-4-Alkyl)-3-fluorphenyl]-- 5- [4-(trans-4-alkylzyklohexyl)phenyl]-pyridinen(X)

62.1 2-[4-(trans-4-Penthyl)-3-nitrophenyl]-5--[4-(trans-4-äthylzyklohexyl)phenyl]-pyridin (VIIIa).

Erhalten in 75%iger Ausbeute analog zur Verbindung Va.

$T_{C-N}$ =87,0 °C $T_{N-I}$ = 168 °C (aus Äthanol)

$^1$H-NMR-Spektrum ähnelt dem der Verbindung V.a.

Gefunden in %: C 77,81; H 9,21; $C_{30}H_{42}N_2O_2$.

Berechnet in %: C 77,88; H 9,15.

Analog zu V.a. werden andere Verbindungen der Formel VIII hergestellt.

62.2. 2-[4-(trans-4-Pentyl)-3-aminophenyl]-5[4-(trans--4-äthylzyklohexyl)phenyl] -pyridin (IXa)

Erhalten in 90%iger Ausbeute analog zu Verbindung VI.a.

$T_{C-S}$ = 98,1 °C; $T_{S-N}$ =135,0; $T_{N-I}$ = 212,4 °C (aus Alkohol)

Gefunden in %: C 83,36; H 10,19. $C_{30}H_{44}N_2$

Berechnet in %: C 83,28; H 10,25

Analog zu VI. a werden andere Verbindungen der Formel IX hergestellt.

62.3. 2-[4-(trans-4-Pentyl)-3-fluorphenyl] 5- [4-(trans-4-äthylzyklohexyl)phenyl]-pyridin (X.a).

Erhalten analog zu VII a. $T_{C-N}$ =104,6 °C; $T_{N-I}$ > 270,0 °C (aus Alkohol).

Gefunden in %: C 82,73; H 9,65. $C_{30}H_{42}NF$

Berechnet in %: C 82,71; H 9,72

Analog zu VII.a werden andere Verbindungen der Formel X hergestellt.

Beispiel 63

Herstellung von 2-(4-Alkyl-3-fluorphenyl)-5-(trans--4-alkylzyklohexyl)-pyridinen (XIV)

63.1. 2-(4-Pentyl-3-nitrophenyl)-5-(trans-4-äthylzyklohexyl)-pyridin (XIIa)

Man erhält analog zu V. a in 70%iger Ausbeute. $T_{C-N}$ = 30 °C, $T_{N-I}$ = 42 °C (aus Äthanol).

Gefunden in %: C 75,69; H 8,51; $C_{24}H_{32}N_2O_2$.

Berechnet in %: C 75,75; H 8,48

Analog zu V. a werden andere Verbindungen der Formel XII hergestellt.

63.2. 2-(4-Pentyl-3-aminophenyl)-5-(4-trans-4--äthylzyklohexyl)-pyridin (XIII a). Erhalten in 92%iger Ausbeute analog zu VI. a.

$T_{C-N}$ = 63,1 °C; $T_{N-I}$ = 120 °C (aus Äthanol)

Gefunden in %: C 82,43; H 9,56. $C_{24}H_{34}N_2$

Berechnet in %: C 82,23; H 9,78

Analog zu VI. a werden andere Verbindungen der Formel XIII hergestellt.

63.3. 2-(4-Pentyl-3-fluorphenyl)-5-(trans-4-äthylzyklohexyl)-pyridin (XIVa)

Erhalten analog zu VIIa. $T_{S-N}$ = 120 °C, $T_{N-I}$=127 °C.

Gefunden in %: C 81,46; H 9,23. $C_{24}H_{32}NF$.

Berechnet in %: C 81,54; H 9,12

Analog zu VIIa werden andere Verbindungen der Formel XIV hergestellt.

Beispiel 64. Herstellung von 2-(4-Alkyloxy-3-fluorphenyl)-5-(trans-4-alkylzyklohexyl)-pyridinen (XV)

64.1. 2-/4-Heptyloxy-3-fluorphenyl)-5-(trans-4-äthylzyklohexyl)-pyridin (XVa)

Erhalten analog zu Beispiel 33.4. Die Ausbeute beträgt 38 %. $T_{C-S}$ = 53 °C, $T_{S-N}$ = 122 °C, $T_{N-I}$ = 136 °C (aus Äthanol)

Gefunden in %: C 78,60; H 9,12. $C_{26}H_{36}NOF$.

Berechnet in %: C 78,55; H 9,13.

Analog zu Beispiel 33.4 werden andere Verbindungen der Formel XV hergestellt.

Beispiel 65.

Herstellung von 2- p-[2-(trans-4-Alkylzyklohexyl)äthyl]-3-fluorphenyl-5-alkylpyridinen (XVI)

65.1. Herstellung von 2-[4-/2-(trans-4-Butylzyklohexyl)-äthyl/-3-nitrophenyl]-5-äthylpyridin (64.1.a) analog zu V.a. $T_{Schmelz}$ = 75 °C (Äthanol)

Gefunden in %: C 76,25; H 8,53; $C_{25}H_{34}N_2O_2$

Berechnet in %: C 76,10; H 8 8,69

65.2. Herstellung von 2-[4-/2-(trans-4-Butyl-zyklohexyl)-äthyl]3-aminophenyl -5-äthylpyridin (65.2.a) analog zu VI.a

$T_{S-N}$ =97,0 °C, $T_{N-I}$ = 116,0 °C (aus Äthanol).

Gefunden in %: C 82,50; H 10,00. $C_{25}H_{36}N_2$

Berechnet in %: C 82,36; H 9,95.

65.3. Herstellung von 2-[4- /2-(trans-4-Butylzyklohexyl)-äthyl] -3-fluorphenyl -5-äthylpyridin (XVIa) analog zu VIIa. $T_{C-N}$ = 65 °C, $T_{N-I}$ = 115 °C

Gefunden in %: C 81,75; H 9,44. $C_{25}H_{34}NF$

Berechnet in %: C 81,70; H 9,32

Beispiel 66

Herstellung von 5-Alkyl-2-(4-isothiozyanophenyl)-pyridinen (XVII)

66.1. Herstellung von 5-Pentyl-2-(4-nitrophenyl)-pyridin (66.1.a)

Es wird analog zu 25.3.a-25.5.a aus 1-β-Alkyläthylenylpiperidin und 1-Akryloyl-4-nitrobenzol hergestellt.

$T_{Schmelz}$ = 48 °C (aus Äthanol)

$^1$H-NMR-Spektrum ($CCl_4$, $\delta$, ppm): Triplett 0,92 ($CH_3$), Multiplett 1,62 ($CH_2$), Triplett 2,6 ($CH_2Py$), Duplett 8,45 (J 2 Hz, $H_2$), verbr. Singulett 7,55 ($H_\beta$ $H_j$), verbr. Sing, 8,15 ($H_{2,6,3,5}$)

Analog zu 25.3.a-25.5.a sind andere Verbindungen 66.1.b-e hergestellt.

Tabelle 18

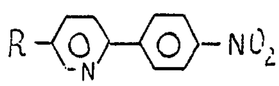

(66.1)

| Nr. | R | $T_{Schmelz}$ in °C | Ausbeute in % | Gefunden in % C | Gefunden in % H | Bruttoformel | Berechnet in % C | Berechnet in % H |
|---|---|---|---|---|---|---|---|---|
| a | $C_5H_{11}$ | 48 | 34 | 71,21 | 6,81 | $C_{16}H_{18}N_2O_2$ | 71,09 | 6,71 |
| b | $C_3H_7$ | 73 | 36 | 69,60 | 5,74 | $C_{14}H_{14}N_2O_2$ | 69,48 | 5,82 |
| c | $C_4H_9$ | 49 | 40 | 70,38 | 6,19 | $C_{15}H_{16}N_2O_2$ | 70,29 | 6,29 |
| d | $C_6H_{13}$ | 49 | 41 | 71,82 | 6,82 | $C_{17}H_{20}N_2O_2$ | 71,81 | 7,09 |
| e | $C_7H_{15}$ | 46 | 38 | 72,37 | 7,51 | $C_{18}H_{22}N_2O_2$ | 72,46 | 7,43 |

66.2. 2-(4-Aminophenyl)-5-pentylpyridin (66.2.a)

Ein Gemisch aus 30 ml Äthanol, 0,01 Mol 2-(4-Nitrophenyl)-5-pentylpyridin (66.1.a), 1 g Ni/Re, 2,5 ml Hydrazinhydrat und 0,1 g NaOH wird in einem mit Rückflußkühler versehenen Kolben gesiedet, bis die Ausgangsverbindung 66.1.a auf dem Chromatogramm verschwindet. Das Reaktionsgut wird abfiltriert und auf eine Temperatur von -10 °C abgekühlt. Man isoliert 0,0073 Mol 2-(4-Aminophenyl)-5-pentylpyridin (66.2.a) in 73%iger Ausbeute. Analog sind andere Verbindungen 66.2 b-e hergestellt, deren Elementaranalyse und Eigenschaften in der Tabelle 19 angegeben sind.

Tabelle 19

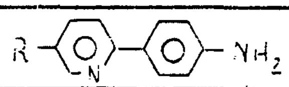

(66.2.a-e)

| Nr. | R | $T_{Schmelz}$ in °C | Ausbeute in % | Gefunden in % C | Gefunden in % H | Gefunden in % N |
|---|---|---|---|---|---|---|
| 1 | 2 | 3 | 4 | 5 | 6 | 7 |
| a | $C_5H_{II}$ | 60 | 73 | 79,98 | 8,50 | II,52 |

Fortsetzung der Tabelle 19

| 1 | 2 | 3 | 4 | 5 | 6 | 7 |
|---|---|---|---|---|---|---|
| b | $C_3H_7$ | II2 | 78 | 79,23 | 7,54 | I3,I7 |
| c | $C_4H_9$ | 93 | 76 | 79,64 | 8,0I | I2,35 |
| d | $C_6H_{I3}$ | 59 | 80 | 80,36 | 8,93 | I0,7I |
| e | $C_7H_{I5}$ | 55 | 74 | 80,4I | 9,22 | I0,37 |

Fortsetzung der Tabelle 19

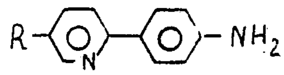

(66.2.a-e)

| Nr. | Bruttoformel | Berechnet in % | | |
|---|---|---|---|---|
| | | C | H | N |
| 1 | 8 | 9 | I0 | II |
| a | $C_{I6}H_{20}N_2$ | 79,96 | 8,39 | II,65 |
| b | $C_{I4}H_{I6}N_2$ | 79,20 | 7,60 | I3,20 |
| c | $C_{I5}H_{I8}N_2$ | 79,6I | 8,02 | I2,38 |
| d | $C_{I7}H_{22}N_2$ | 80,27 | 8,72 | II,0I |
| e | $C_{I8}H_{24}N_2$ | 80,55 | 9,0I | I0,44 |

66.3. 5-Pentyl-2-(4-isothiozyanophenyl)-pyridin (XVIIa)

Man löst 0,02 Mol 2-(4-Aminophenyl)-5-pentylpyridin 66.2.a in 10 ml absolutem Benzol auf, gibt 0,02 Mol $CS_2$, 3 ml $Et_3N$ zu und läßt für 5 Tage stehen. Der ausgefallene Niederschlag wird abfiltriert und mit absolutem Äther gewaschen, dann in einen Dreihalskolben, versehen mit einem Rührwerk, einem Thermometer und einem Tropftrichter mit Gegenstrom, eingebracht. Man bringt in den Kolben 20 ml absolutes Chloroform ein und tropft bei 0 °C 3 ml $Et_3N$ und dann 3 ml Äthylchlorformiat zu. Das Reaktionsgut wird innerhalb von 10 Minuten bei 0 °C und dann innerhalb von 1 Stunde bei Raumtemperatur umgerührt, die Chloroformschicht mit 1%iger Salzsäure und Wasser gewaschen, und nach der Trocknung über $Na_2SO_4$ durch $SiO_2$-Schicht L = 5/40 (h = 2 cm)

abfiltriert. Man dampft die Mutterlauge unter Vakuum ein, kristallisiert den Rückstand aus Hexan um. Man isoliert 0,0106 Mol 5-(Pentyl-2-(4-isothiozyanophenyl)-pyridin in 53%iger Ausbeute. Analog werden andere Verbindungen XVII b-e, deren Elementaranalyse und Eigenschaften in der Tabelle 20 angegeben sind, hergestellt.

Phasenübergangstemperaturen der bekannten Verbindungen 4-Alkyl-4'-isothiozyanobiphenyle und der neuen Verbindungen 5-Alkyl-2-(4-isothiozyanophenyl)-pyridine (XVII a-e) sind vergleichsweise in der Tabelle 21 gegenübergestellt. Die Tabelle 21 zeigt, daß die neuen Verbindungen bedeutend höhere Klärpunkte gegenüber den bekannten Verbindungen 4-Alkyl-4'-isothiozyanobiphenylen aufweisen. (MCLC, Letters, v.102, no.155, 1984. R.Dabrowski, J.Dziaduszek, T.Szczucinski. "4-(Trans-4' -n-alkylcyclohexyl)isothiocyanatobenzenes", A new class of lowmelting stable nematics")

Tabelle 20

5-Alkyl-2-(4-isothiozyanophenyl)-pyridine 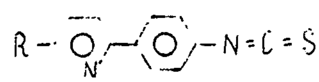

| Nr. | R | Ausbeute in % | $T_{C-S}$, in °C | $T_{S-I}$, in °C | Gefunden in % C | H | N |
|---|---|---|---|---|---|---|---|
| 1 | 2 | 3 | 4 | 5 | 6 | 7 | 8 |
| a | $C_5H_{II}$ | 53 | 34,0 | 98,5 | 72,48 | 6,39 | 10,00 |
| b | $C_3H_7$ | 67 | 64,0 | 99,0 | 71,0I | 5,36 | II,20 |
| c | $C_4H_9$ | 58 | 37,6 | I00,0 | 7I,59 | 6,03 | I0,60 |
| d | $C_6H_{I3}$ | 63 | 27,0 | 99,0 | 72,8I | 6,90 | 9,52 |
| e | $C_7H_{I5}$ | 70 | 25,0 | 99,0 | 73,46 | 7,29 | 9,I4 |

Fortsetzung der Tabelle 20

5-Alkyl-2-(4-isothiozyanophenyl)-pyridine 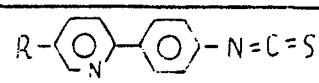

| Nr. | Bruttoformel | Berechnet in % C | H | N |
|---|---|---|---|---|
| 1 | 9 | I0 | II | I2 |
| a | $C_{I7}H_{I8}N_2S$ | 72,30 | 6,42 | 9,92 |

Fortsetzung der Tabelle 20

| I | 9 | 10 | 11 | 12 |
|---|---|---|---|---|
| b | $C_{I5}H_{I4}N_2S$ | 70,83 | 5,55 | 11,01 |
| c | $C_{I6}H_{I6}N_2S$ | 7I,6I | 6,0I | I0,44 |
| d | $C_{I8}H_{20}N_2S$ | 72,93 | 6,80 | 9,45 |
| e | $C_{I9}H_{22}N_2S$ | 73,5I | 7,I4 | 9,02 |

Tabelle 21

| 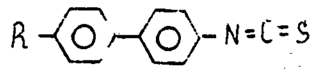 | | | | 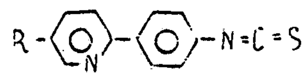 (XVII a-e) | | | |
|---|---|---|---|---|---|---|---|
| Nr. | R | $T_{C-S}$ | $T_{S-I}$ | Nr. | R | $T_{C-S}$ | $T_{S-I}$ |
| a | $C_5H_{II}$ | 53,0 | 74,5 | a | $C_5H_{II}$ | 34,0 | 98,5 |
| | | | | b | $C_3H_7$ | 64,0 | 99,0 |
| | | | | c | $C_4H_9$ | 37,6 | I00,0 |
| b | $C_6H_{I3}$ | | 77,0 | d | $C_6H_{I3}$ | 27,0 | 99,0 |
| c | $C_7H_{I5}$ | 56,0 | 73,0 | e | $C_7H_{I5}$ | 26,0 | 99,0 |

Tabelle 22 gibt die Zusammensetzung und Eigenschaften der flüssigkristallinen Materialien an, welche neue Derivate von 2,5-disubstituiertem Pyridin der Formel XVII enthalten.

Tabelle 22

| Beispiele Nr. | Zusammenseztungen der erfindungsgemässen flüssigkristallinen Materialien | Masse% | Bereich der nematischen Phase, °C |
|---|---|---|---|
| 1 | 2 | 3 | 4 |
| 67. | 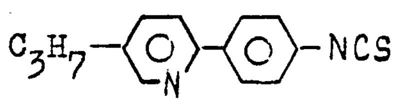 (XVIIb) | 10 | -17 - 18,3 |
| | 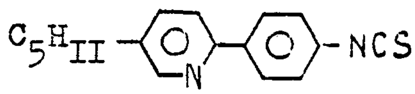 (XVIIa) | 15 | |
| | 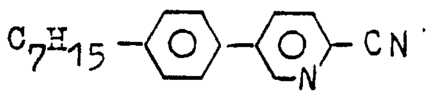 | 20 | |
| | 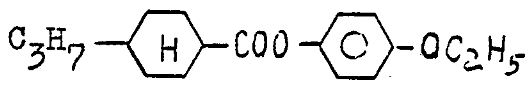 | 15 | |
| | 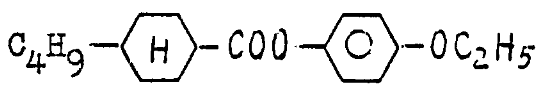 | 10 | |
| | 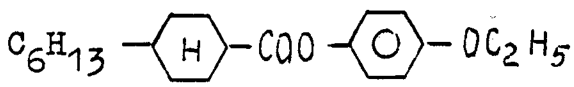 | 15 | |
| | 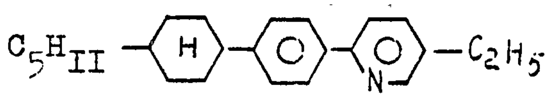 | 5 | |
| | 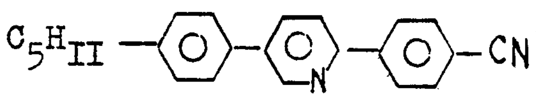 | 10 | |
| 68. | 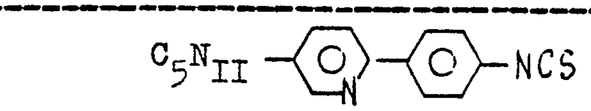 (XVIIa) | 7 | -18 - 92,1 |
| | 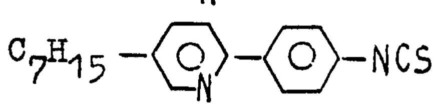 (XVIIe) | 6 | |

Fortsetzung der Tabelle 22

| I | 2 | 3 | 4 |
|---|---|---|---|
| 68. | 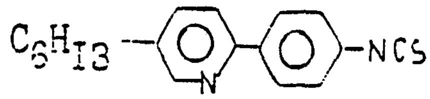 (XVIId) | 4 | |
| | 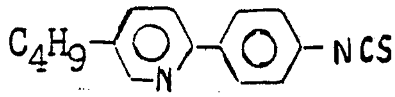 (XVIIc) | 4 | |
| | 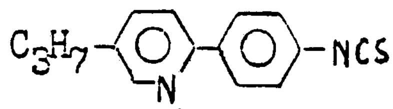 (XVIIb) | 4 | |
| | 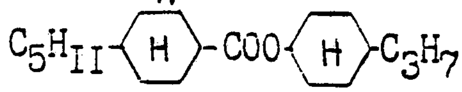 | I0 | |
| | 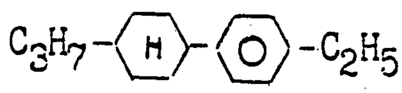 | I0 | |
| | 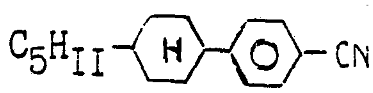 | I0 | |
| | 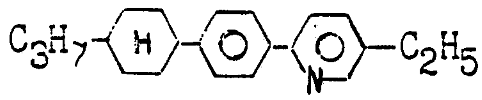 | 8 | |
| | 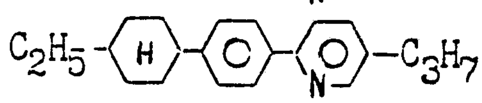 | 8 | |
| | 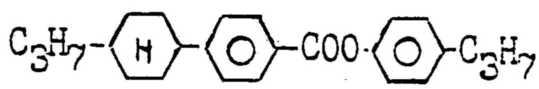 | 9 | |
| | 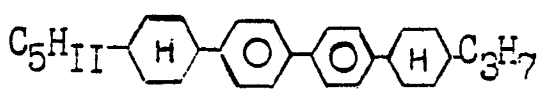 (IV) | I0 | |
| | 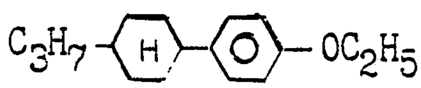 | I0 | |
| 69. | 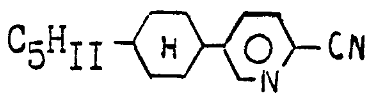 (II) | I0 | -I7-8I,6 |
| | 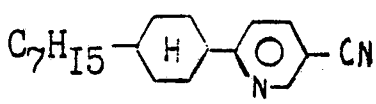 (XXII) | I5 | |
| | 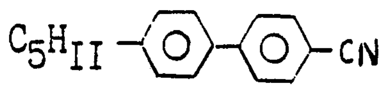 | I0 | |
| | 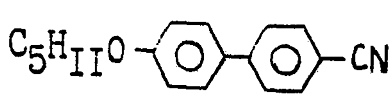 | I0 | |
| | 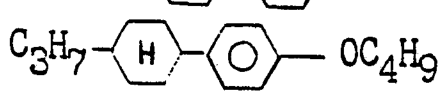 | I0 | |

Fortsetzung der Tabelle 22

| I | 2 | | 3 | 4 |
|---|---|---|---|---|
| 69. | 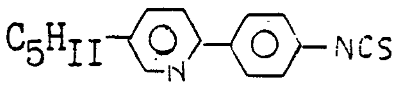 | (XVIIa) | 5 | |
| | 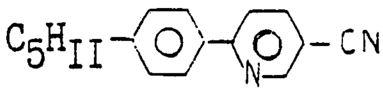 | | I0 | |
| | 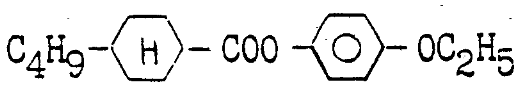 | | I0 | |
| | 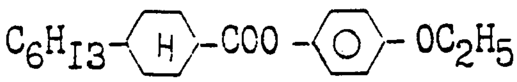 | | I0 | |
| | 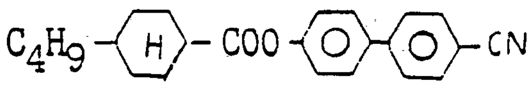 | | I0 | |
| 70. | 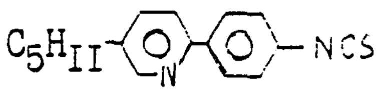 | (XVIIa) | 20 | -I7-79,4 |
| | 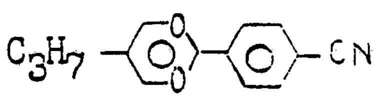 | | 30 | |
| | 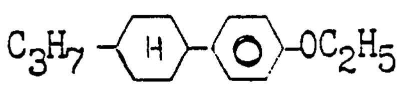 | | I0 | |
| | 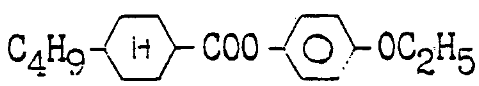 | | I0 | |
| | 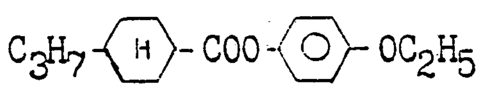 | | I0 | |
| | 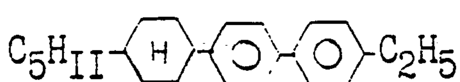 | | I0 | |
| | 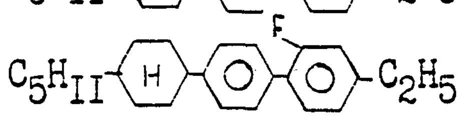 | | I0 | |
| 7I. | 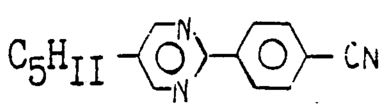 | | I0 | -23-78,I |
| | 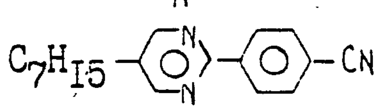 | | 20 | |
| | 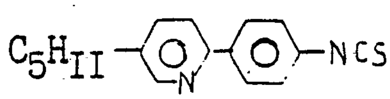 | (XVIIa) | I0 | |

Fortsetzung der Tabelle 22

| I | 2 | | 3 | 4 |
|---|---|---|---|---|
| 71. | 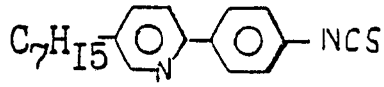 | (XVIIe) | 5 | |
| | 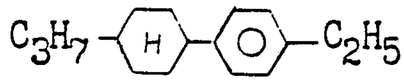 | | 15 | |
| | 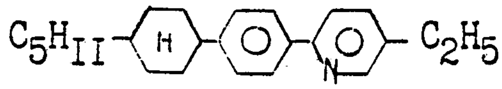 | | 8 | |
| | 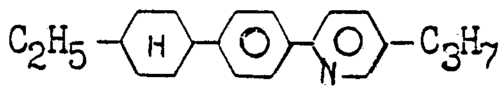 | | 8 | |
| | 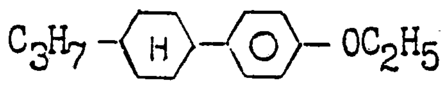 | | 10 | |
| | 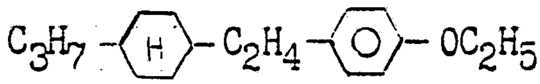 | | 10 | |
| | 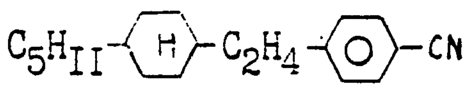 | | 4 | |
| 72. | 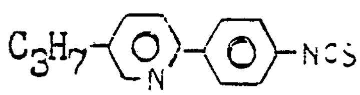 | (XVIIb) | 6 | -7-77,3 |
| | 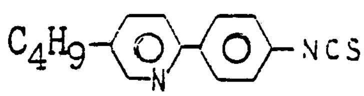 | (XVIIc) | 6 | |
| | 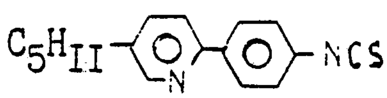 | (XVIIa) | 6 | |
| | 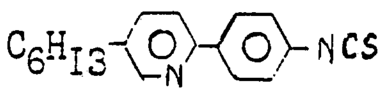 | (XVIId) | 6 | -7-77,3 |
| | 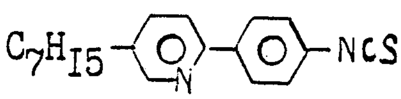 | (XVIIc) | 7 | |
| | 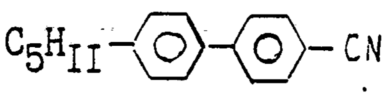 | | 20 | |
| | 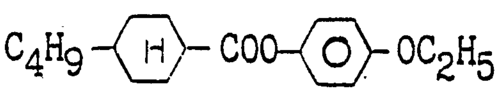 | | 10 | |
| | 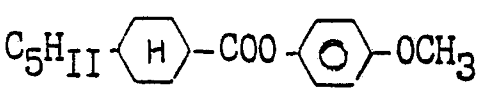 | | 20 | |
| | 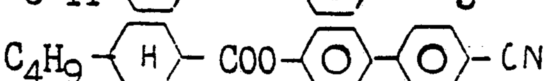 | | 10 | |

Fortsetzung der Tabelle 22

| I | 2 | | 3 | 4 |
|---|---|---|---|---|
| 73. | 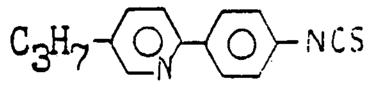 | (XVIIб) | 7 | -23-84,I |
| | 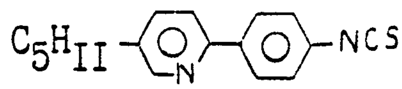 | (XVIIa) | 8 | |
| | 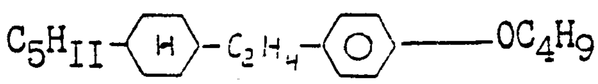 | | IO | |
| | 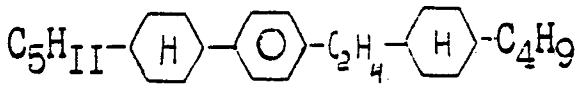 | | IO | |
| | 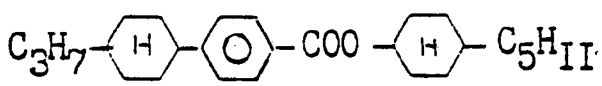 | | IO | |
| | 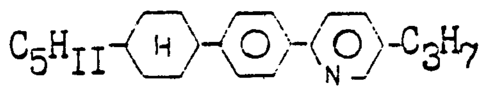 | | IO | |
| | 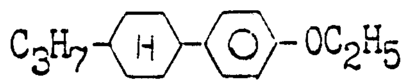 | | IO | |
| | 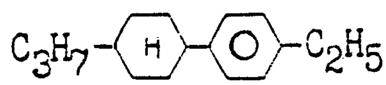 | | IO | |
| | 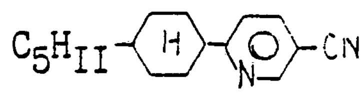 | (XXII) | I5 | |
| | 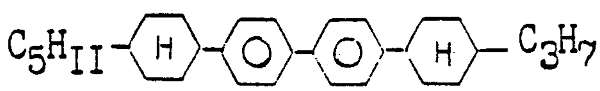 | (IV) | IO | |
| 74. | 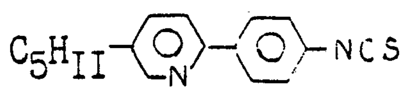 | (XVIIa) | 2 | -I9-87,5 |
| | 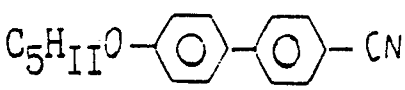 | | IO | |
| | 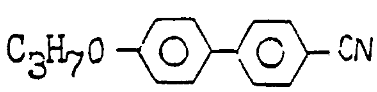 | | IO | |
| | 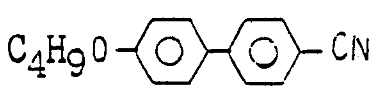 | | IO | |
| | 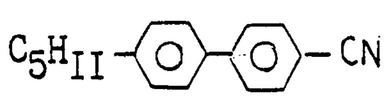 | | I5 | |
| | 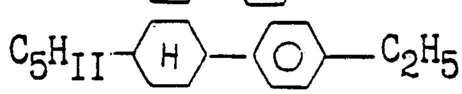 | | 20 | |

Fortsetzung der Tabelle 22

| I | 2 | | 3 | 4 |
|---|---|---|---|---|
| 74. | 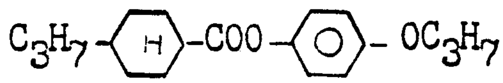 | | I0 | |
| | 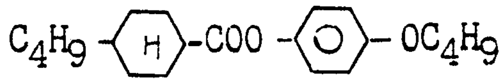 | | I0 | |
| | 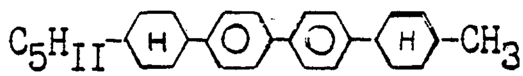 | (IV) | I0 | |
| | 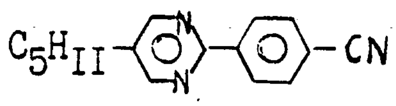 | | 3 | |
| 75. | 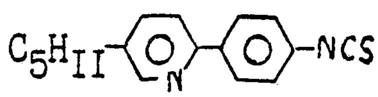 | (XVIIa) | 7 | -I5-89,4 |
| | 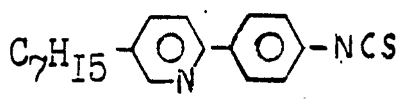 | (XVIIc) | 6 | |
| | 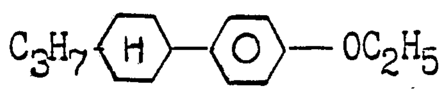 | | I0 | |
| | 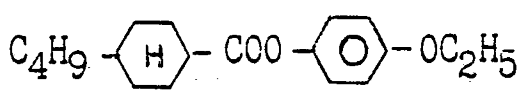 | | I0 | |
| | 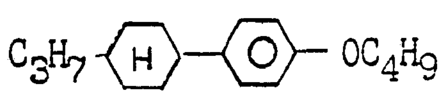 | | I0 | |
| | 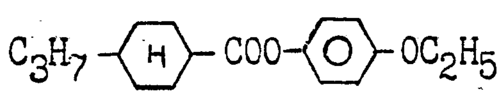 | | I0 | |
| | 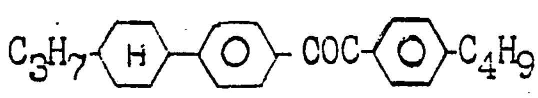 | | I0 | |
| | 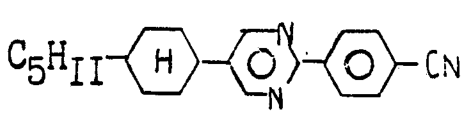 | | I0 | |
| | 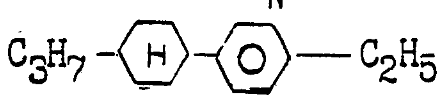 | | 20 | |
| | 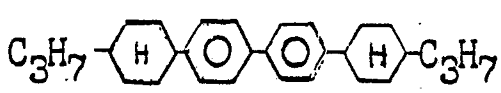 | (IV) | 7 | |

Aus der Tabelle 22 ist zu ersehen, daß die Verwendung der neuen Pyridinderivate der Formel XVII als Komponenten von flüssigkristallinen Materialien ermöglicht, flüssigkristalline Materialien mit einem breiten Temperaturenbereich der nematischen Phase herzustellen.

Beispiel 76

Herstellung von 2-[4-(4-Zyanophenyl)-phenyl] -5-alkylpyridinen (XVIII)

76.1. Herstellung von 2-[4-(4-Bromphenyl)-phenyl] -5-pentylpyridin

(76.1.a) Man erhält analog zu 25.3.a-25.5.a aus 1-β-Alkyläthylenylpiperidin und 4-Akryloyl-4'-bromphenyl.

Gefunden in %: (69,36; H 5,91. $C_{22}H_{22}NBr$

Berechnet in %: C 69,48; H 5,83.

76.2. Herstellung von 2-[4-(4-Zyanophenyl)-phenyl]-5-pentylpyridin (XVIIIa). Erhalten analog zu III.a unter Verwendung der Verbindung 76.1.a.

$T_{C-S}$ = 90,1 °C, $T_{S-N}$ = 162 °C, $T_{N-I}$ = 251 °C.

Gefunden in %: C 84,71; H 6,65. $C_{23}H_{22}N_2$.

Berechnet in %: C 84,63; H 6,79.

Analog zu IIIa werden andere Verbindungen der Formel XVIII hergestellt.

Beispiel 77

Herstellung von 5-Alkyl-2-[4-(2-E-zyanovinyl)-phenyl]-pyridinen (XIX)

77.1. 5-Propyl-2-[4-(2-E-karboxyvinyl)-phenyl]-pyridin-hydrochlorid (77.1.a)

In einen Kolben, versehen mit einem Rückflußkühler, einem Rührwerk und einem Gaseinlaßrohr, bringt man im Argonstrom 4,14 g (15 mMol) 2-(4-Bromphenyl)-5-propylpyridin, 0,183 g (0,6 mMol) $P(aCH_3C_6H_4)_3$, 0,0345 g (0,15 mMol) $Pd(OAc)_2$, 7,65 ml $Et_3N$ und 1,62 g (1,56 ml, 22,5 mMol) Akrylsäure ein. Der Kolben wird bis zum Sieden erhitzt und im Argonstrom innerhalb von 1 Stunde verdampft. Man löst das Reaktionsgut in 100 ml Wasser auf, filtriert durch ein Glasfilter und säuert das Filtrat mit 10%iger Salzsäure bis zum Erzielen eines pH-Wertes von etwa 6 an. Der ausgefallene Niederschlag wird abfiltriert, mit Wasser und

Azeton gewaschen, an der Luft getrocknet. Man isoliert 4 g (99 %) 77.1. a und leitet zur folgenden Stufe ohne Umkristallisation und Analyse.

77.2. 5-Propyl-2-[4-(2-E-karboxyvinyl)-phenyl]-pyridinamid (77.2.a)

Zu einer Mischung von 3,35 g (11,1 mMol) Verbindung 77.1.a in 10 ml Dichlormethan setzt man unter Umrühren 3,5 g (16,5 mMol) Phosphorpentachlorid zu und rührt noch innerhalb von 3 Stunden weiter um. Das Reaktionsgut wird langsam bei 0 °C in 30 ml Ammoniakflüssigkeit gegossen, innerhalb von 1 Stunde gerührt, und das Dichlormethan eingedampft. Der Niederschlag wird abfiltriert, mit Wasser und Azeton gewaschen. Man isoliert 2,3 g (79,5 %) Verbindung 77.2.a. mit $T_{Schmelz}$ = 240 bis 245 °C. Analog isoliert man die Verbindung 77.2.b. mit $T_{Schmelz}$ =238-241 °C, 77.2.c mit $T_{Schmelz}$ =235-240 °C, 77.2.d mit $T_{Schmelz}$ = = 230-234 °C, 77.2.e mit $T_{Schmelz}$ = 229-234 °C. Die Stoffe werden ohne Analyse zur folgenden Stufe geleitet.

77.3. 5-Propyl-2-[4-(2-E-zyanovinyl)-phenyl]-pyridin (XIXa)

Man siedet das Gemisch von 2,3 g (8,75 mMol) ungereinigtem Amid 77.2.a und 10 ml Phosphoroxidchlorid innerhalb von 2 Stunden, destilliert das überschüssige Phosphoroxidchlorid im Vakuum ab, fügt 50 ml Benzol dem Rückstand hinzu und neutralisiert mit der Ammoniakflüssigkeit unter Abkühlung. Die Benzolschicht wird mit Wasser gewaschen, über wasserfreiem Natriumsulfat getrocknet und durch $SiO_2$--Schicht L = 5/40 (h = 2 cm) abfiltriert. Man treibt Benzol ab, kristallisiert den Rückstand aus Alkohol um. Man isoliert 1,4 g (61 %) Verbindung XIX a.

$^1$H-NMR-Spektrum ($CCl_4$): Multiplett 0,85 (3 H, $CH_3$); Multiplett 1,3-1,6 (2 H, $CH_2$); Triplett 2,65 (2 H, J = = 6 Hz, $CH_2Ph$); Duplett 5,95 (1 H, J = 16,5 Hz, H );Duplett 7,45 (1 H, J = 16,5 Hz, $H_\beta$ ); Duplett 7,55 (2 H, J = 8 Hz, $H_{3,5}$); Dopellduplett 7,6 (1 H, $J_{\beta\gamma}$ = 8 Hz, $J_{\alpha\gamma}$ = 2 Hz, $H_\gamma$ ); Doppelduplett 7,7 (1 H, $J_{\beta\gamma}$ = 8 Hz, $J_{\beta\alpha}$ = 1 Hz, $H_\beta$ ); Duplett 8,05 (2 H, J = 8 Hz, $H_{26}$); Duplett 8,55 (1 H, J = 1 Hz, $H_\alpha$ ).

Analog werden andere Verbindungen der Formel XIX b-e hergestellt.

Die Phasenübergangstemperaturen und Elementaranalyse der Verbindungen der Formel XIX a-e sind in der Tabelle 23 dargestellt.

Die Phasenübergangstemperaturen der bekannten Verbindungen 4-Alkyl-4' -(2-E-zyanovinyl)-biphenyle und der neuen Verbindungen der Formel XIX sind vergleichsweise in der Tabelle 24 gegenübergestellt.

Tabelle 23

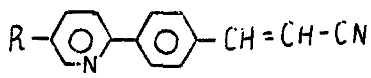 (XIX)

| Nr. | R | $T_{C-N}$ C-S, in °C | $T_{S-N}$, in °C | $T_{N-I}$, in °C | Gefunden in % C | Gefunden in % H |
|---|---|---|---|---|---|---|
| 1 | 2 | 3 | 4 | 5 | 6 | 7 |
| a | $C_3H_7$ | 84,2 | IIO,0 | I62,0 | 82,I3 | 6,49 |
| b | $C_4H_9$ | 73,5 | - | I50,2 | 82,54 | 7,IO |
| c | $C_5H_{II}$ | 65,7 | - | I55,I | 82,5I | 7,I8 |
| d | $C_6H_{I3}$ | 32,I | I23,I | I46,2 | 82,90 | 7,56 |
| e | $C_7H_{I5}$ | 48,0 | I33,0 | I43,0 | 82,9I | 8,02 |

Fortsetzung der Tabelle 23

| Nr. | Bruttoformel | Berechnet in % C | Berechnet in % H | Ausbeute in % |
|---|---|---|---|---|
| 1 | 8 | 9 | IO | II |
| a | $C_{I7}H_{I6}N_2$ | 82,22 | 6,50 | 6I |
| b | $C_{I8}H_{I8}N_2$ | 82,40 | 6,92 | 59 |
| c | $C_{I9}H_{20}N_2$ | 82,57 | 7,29 | 55 |
| d | $C_{20}H_{22}N_2$ | 82,7I | 7,64 | 57 |
| e | $C_{2I}H_{24}N_2$ | 82,85 | 7,95 | 53 |

Tabelle 24

R 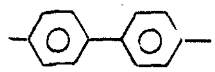 CH=CH-CN

| Nr | R | $T_{C-N}$, C-S, in °C | $T_{S-N}$, in °C | $T_{N-I}$, in °C | $\Delta T_N$, in °C |
|---|---|---|---|---|---|
| 1 | 2 | 3 | 4 | 5 | 6 |
| a | $C_3H_7$ | | | | |
| b | $C_4H_9$ | | | | |
| c | $C_5H_{II}$ | 53,0 | - | II2,0 | 59,0 |
| d | $C_6H_{I3}$ | | | | |
| e | $C_7H_{I5}$ | | | | |

Fortsetzung der Tabelle 24

R 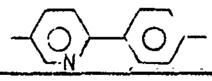 CH=CH-CN (XIX)

| Nr | R | $T_{C-N}$, C-S, in °C | $T_{S-N}$, in °C | $T_{N-I}$ in °C | $\Delta T_N$, in °C |
|---|---|---|---|---|---|
| 1 | 7 | 8 | 9 | 10 | 11 |
| a | $C_3H_7$ | 84,2 | II0,0 | I62,0 | 52,0 |
| b | $C_4H_9$ | 73,5 | - | I50,2 | 76,7 |
| c | $C_5H_{II}$ | 65,7 | - | I55,I | 89,4 |
| d | $C_6H_{I3}$ | 32,I | I23,I | I46,2 | 23,I |
| e | $C_7H_{I5}$ | 48,0 | I33,0 | I43,0 | I0,0 |

Aus der Tabelle 24 ist ersichtlich, daß Klärpunkte der neuen Verbindungen der Formel XIX (a-e) bedeutend höher als die der bekannten Verbindungen sind (Biuletyn Wojskowej Akademii technicznej, Rok XXX, Nr. 6 (346), 143, 1981. R.Dabrowski, E.Zytynski. "Wlasnosci mesomorficzne pochodnych 4-n-pentylobifenylu").

Die Tabelle 25 gibt die Zusammensetzung und die Eigenschaften der flüssigkristallinen Materialien an, welche die neuen Pyridinderivate der Formel XIX (a-e) enthalten.

Tabelle 25

| Nr. | Zusammensetzung des erfindungsgemäßen flüssigkristallinen Materials | Masse % | Bereich der nematischen Phase in $^{o}C$ |
|---|---|---|---|
| 1 | 2 | 3 | 4 |
| 78. | 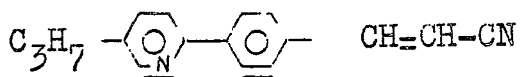 | IO | -I2-93 |
| | 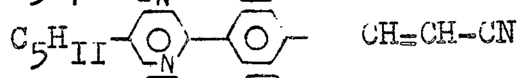 | I5 | |
| | 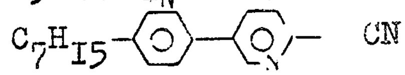 | 20 | |
| | 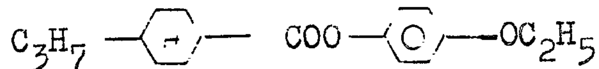 | I5 | |
| | 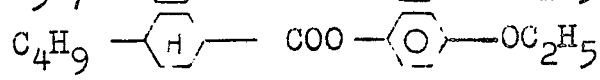 | IO | |
| | 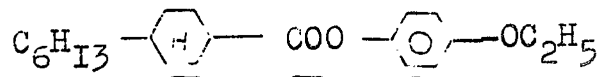 | I5 | |
| | 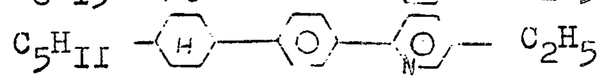 | 5 | |
| | 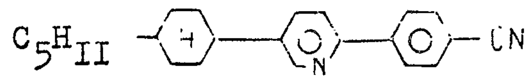 | (IIIc) IO | |
| 79. | 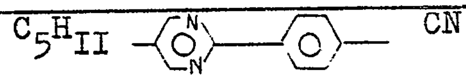 | IO | -I8-90 |
| | 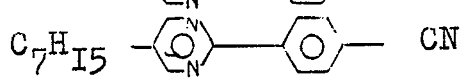 | 20 | |
| | 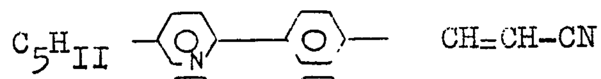 (XIXc) | IO | |
| | 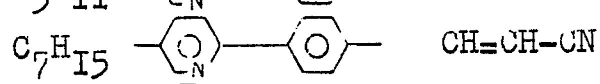 (XIXe) | 5 | |
| | 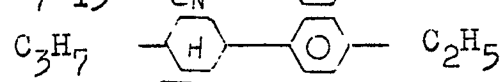 | I5 | |
| | 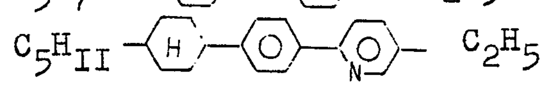 | 8 | |
| | 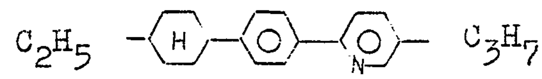 | 8 | |
| | 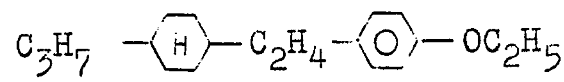 | IO | |
| | 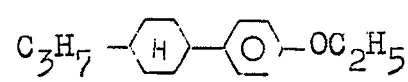 | IO | |
| | 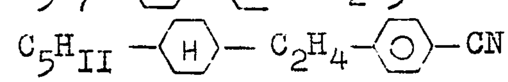 | 4 | |

Fortsetzung der Tabelle 25

| 1 | 2 | 3 | 4 |
|---|---|---|---|
| 80 | 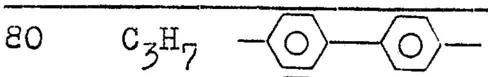 | 20 | -I9-73,6 |
| | 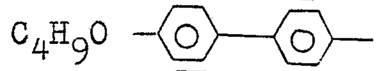 | I5 | |
| | 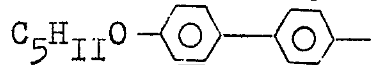 | 8 | |
| | 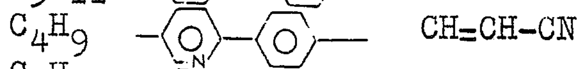 (XIXb) | 3 | |
| | 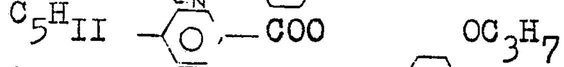 | IO | |
| | 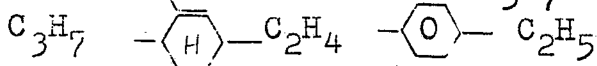 | IO | |
| | 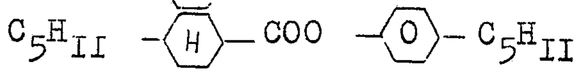 | IO | |
| | 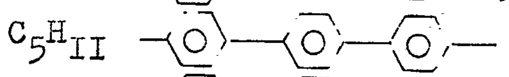 | 9 | |
| | 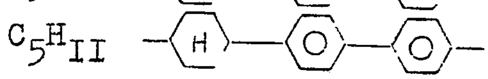 | IO | |
| | 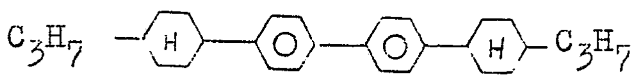 (IVb) | 5 | |
| 8I | 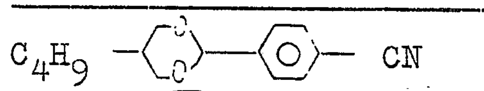 | I5 | -26-80,4 |
| | 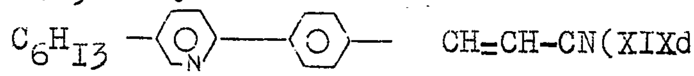 (XIXd) | 5 | |
| | 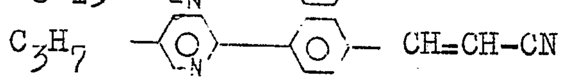 (XIXa) | 5 | |
| | 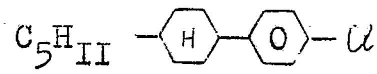 | IO | |
| | 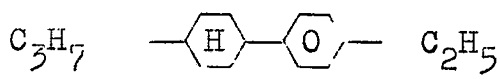 | IO | |
| | 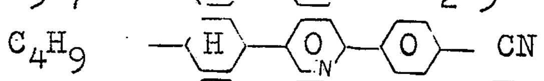 (IIIa) | 8 | |
| | 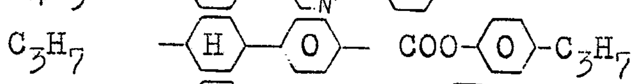 | 8 | |
| | 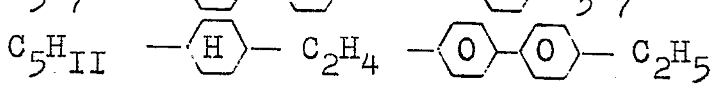 | 20 | |
| | 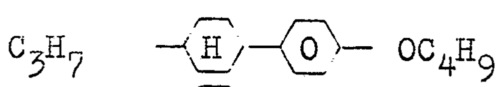 | 9 | |
| | 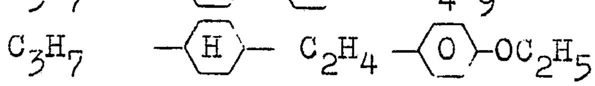 | IO | |
| 82 | 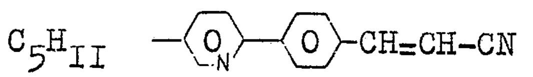 (XIXc) | IO | -I7-II8,2 |
| | 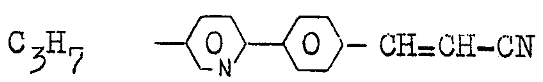 (XIXa) | 5 | |
| | 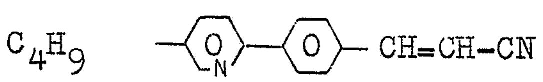 (XIXb) | 5 | |

Fortsetzung der Tabelle 25

| I | 2 | 3 | 4 |
|---|---|---|---|
| 82. | 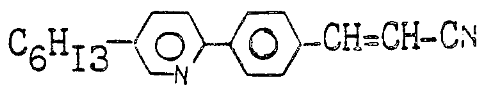 (XIXd) | 5 | |
| | 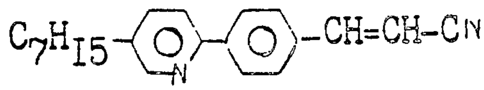 (XIXe) | I5 | |
| | 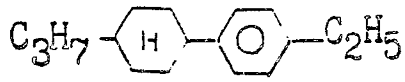 | I5 | |
| | 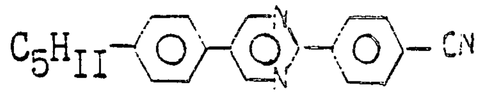 | 8 | |
| | 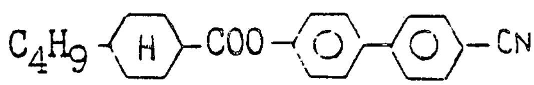 | I0 | |
| | 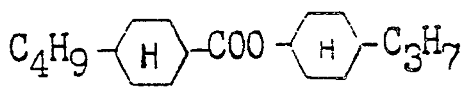 | I0 | |
| | 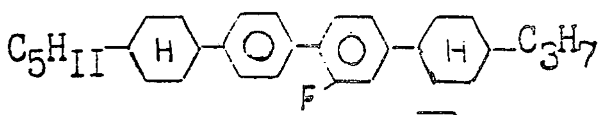 | 8 | |
| | 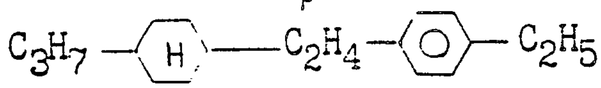 | 9 | |
| 83. | 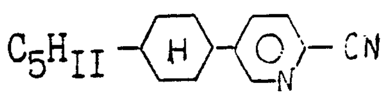 (IId) | 3 | -6-I23 |
| | 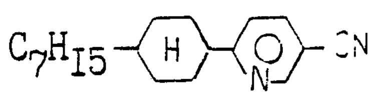 (IIe) | 2 | |
| | 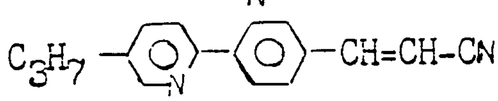 (XIXa) | I0 | |
| | 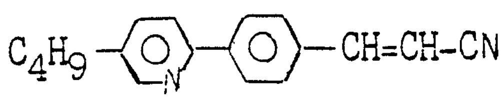 (XIXb) | I0 | |
| | 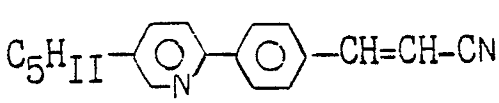 (XIXc) | I0 | |
| | 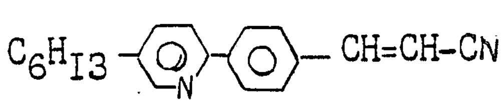 (XIXd) | I0 | |
| | 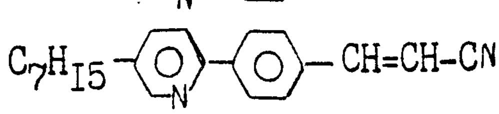 (XIXe) | I0 | |
| | 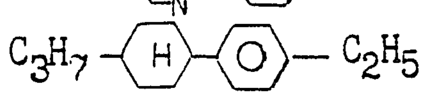 | I0 | |

Fortsetzung der Tabelle 25

| I | 2 | 3 | 4 |
|---|---|---|---|
| 83. | 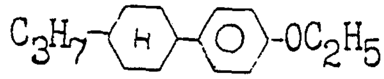 | 8 | |
| | 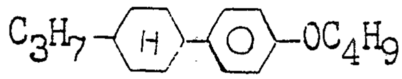 | 7 | |
| | 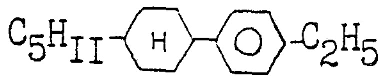 | 5 | |
| | 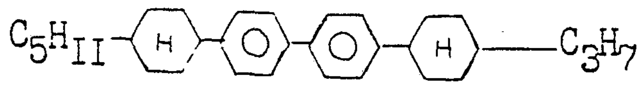 | 8 | |
| | 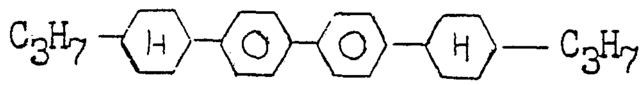 | 7 | |
| 84. | 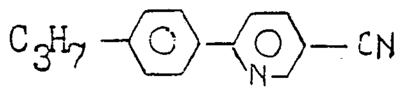 | 7 | -32-69,I |
| | 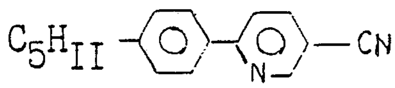 | 8 | |
| | 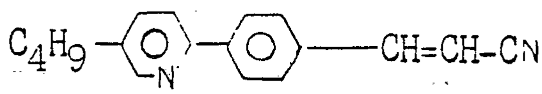 (XIXб) | I | |
| | 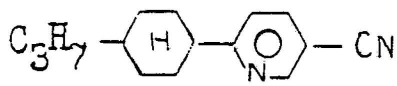 (XXП) | 3 | |
| | 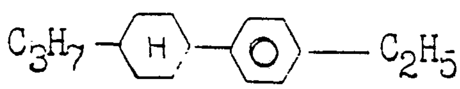 | 20 | |
| | 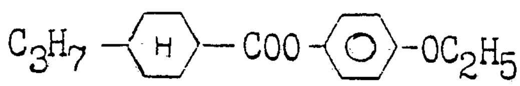 | 5 | |
| | 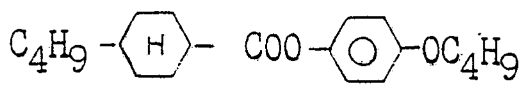 | 7 | |
| | 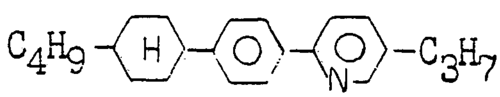 | 5 | |
| | 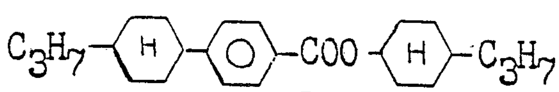 | 6 | |
| | 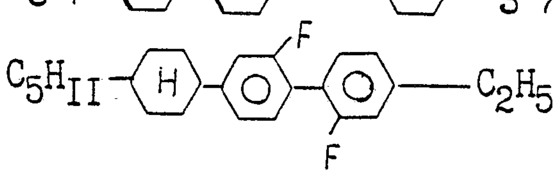 | I8 | |

Fortsetzung der Tabelle 25

| 1 | 2 | | 3 | 4 |
|---|---|---|---|---|
| 84 | $C_6H_{13}$ | 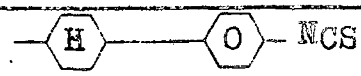 | IO | |
| | $C_5H_{11}$ | 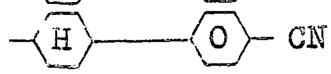 | IO | |

Die Tabelle 25 zeigt, daß die Verwendung der neuen Pyridinderivate der Formel XIX als Komponenten der flüssigkristallinen Materialien ermöglicht, flüssigkristalline Materialien mit einem breiten Temperaturenbereich der nematischen Phase und hohen Klärpunkten herzustellen.

Beispiel 85. Herstellung von 2- [4(2-E-Zyanovinyl)-phenyl]-5-(trans-4-alkylzyklohexyl)-pyridinen (XX)

85.1. 2- [4-(2-E-Karboxyvinyl)-phenyl] -5-(trans-4-propylzyklohexyl)-pyridin-hydrogenchlorid (85.1.a)

Die Verbindung 85.1.a ist analog zu 77.1.a aus 25.5.b erhalten und ohne Reinigung zur folgenden Stufe geleitet.

85.2. 2- [4-(2-E-Karboxyvinyl)-phenyl]5-(trans-4-propylzyklohexyl)-pyridin-amid.(85.2.a) ist analog zu 77.2.a erhalten und ohne Reinigung zur folgenden Stufe geleitet.

85.3. Herstellung von 2-[4-(2-E-Zyanovinyl)-phenyl]--5-(4-trans-propylzyklohexyl)-pyridin (XXa).

Erhalten analog zu XIX a. $T_{C-N}$= 133,0 °C, $T_{N-I}$ = = 318,4 °C (aus Äthanol)

Gefunden in %: C 83,64; H 7,81. $C_{23}H_{26}N_2$

Berechnet in %: C 83,59; H 7,93.

Analog zu XIX. a werden andere Verbindungen der Formel XX hergestellt.

Beispiel 86. Herstellung von 2-[4-(2-E-Zyanovinyl)-phenyl]-5-(4-alkylphenyl)-pyridinen (XXI)

86,1. Herstellung von 2-[4-(2-E-Zyanovinyl)-phenyl]--5-(4-amylphenyl)-pyridin (XXI.a). Erhalten analog zu XIX.a aus 2-(4-Bromphenyl)-5-(4-amylphenyl)-pyridin. $T_{C-S}$ = 136,1 °C, $T_{S-N}$ = 168,5 °C, $T_{N-I}$ = 310 °C.

Gefunden in %: C 85,31; H 6,73. $C_{25}H_{24}N_2$

Berechnet in %: C 85,19; H 6,86.

Analog zu XIX.a werden andere Verbindungen der For-

 0310676

mel XXI hergestellt.

Beispiel 87

Herstellung von 2-(tans-4-Alkylzyklohexyl)-5-zyanopyridinen (XXII)

87.1. trans-4-Hexyl-4-azetylzyklohexan (87.1.a) ist nach der allgemeinüberlichen Methodik aus trans-4-Hexylzyklohexankarbonsäurechlorid und magnesiumorganischem Diäthylmalonatderivat in 79%iger Ausbeute hergestellt. Der Siedepunkt beträgt 120 °C,4,Torr $n_D^{25}$ = 1,4590. Mit Hilfe der $^1$H-NMR von $^{13}$C wurde nachgewiesen, daß das trans--cis-Verhältnis bei 87.1a 7:1 beträgt.

Analog werden andere Verbindungen der Formel 87.1.b-e hergestellt.

| Substanzen | Ausbeute in % | Siedepunkt/Torr °C | $n_D^{25}$ |
|---|---|---|---|
| 87.1b | 74 | I10/5 | I,4588 |
| 87.1.c | 79 | I28/2 | I,4579 |
| 87.1.d | 88,5 | 86/2 | I,4556 |
| 87.1.e | 73 | I35/2 | I,4600 |

87.2. 3-Zyano-6-(trans-4-hexylzyklohexyl)-pyridin-2-on (87.2.a)

Zu 3,27 g (0,14 Mol) Natriumdraht in 200 ml absolutem Äther fügt man unter Umrühren und Sieden eine Mischung hinzu, die aus 29,6 g (0,14 Mol) Verbindung (87.1.a) und 12,7 g (0,17 Mol)Äthylformiat besteht. Der ausgefallene Niederschlag wird abfiltriert, mit 50 ml absolutem Äther gewaschen und im Vakuum getrocknet. Man isoliert 31,8 g (87,7%) Natriumsalz. Das erhaltene Salz in einer Menge von 31,8 g (0,12 Mol) und 10,5 g (0,12 Mol) Zyanazetamid bringt man in eine Lösung ein, die 500 ml Dioxan, 50 ml $H_2O$, 3,5 ml Essigsäure und 5 ml Piperidin enthält, und rührt bei Raumtemperatur innerhalb von 5 Stunden um. Das Gemisch siedet 12 Stunden lang, dann gießt man es in I l $H_2O$ und säuert mit Essigsäure an. Der Niederschlag wird abfiltriert, mit Wasser und Äthanol gewaschen, an der Luft getrocknet und ohne Umkristallisation zur folgenden Stufe geleitet. Man isoliert 20 g Verbindung 87.2.a in 56%iger Ausbeute. $T_{Schmelz}$ = 228 °C (aus Dioxan).

Analog werden andere Verbindungen der Formel 87.2.b-e hergestellt.

| Substanz | Ausbeute in % | Schmelzpunkt in °C (aus Dioxan) |
|---|---|---|
| 87.2.b | 74 | 233 |
| 87.2.c | 53 | 231 |
| 87.2.d | 51,7 | 236 |
| 87.2.e | ≈ 50 | 227 |

87.3. 2-Chlor-3-zyano-6-(trans-4-hexylzyklohexyl)-pyridin (87.3.a)

Eine Mischung aus 18,2 g (0,06 Mol) Verbindung 87.2.a und 25,4 g (0,13 Mol) $PhPOCl_2$ wird unter Umrühren bei einer zwischen 120 und 130 °C liegenden Temperatur innerhalb von 3 Stunden erhitzt. Man kühlt das Reaktionsgut auf Raumtemperatur ab und gießt in 200 g Eis-Wasser-Mischung aus, rührt 1 Stunde lang um und säuert mit wäßriger Ammoniaklösung bis zum Erzielen eines pH-Wertes von etwa 9 an. Der Niederschlag wird abfiltriert, mit Wasser gewaschen, getrocknet und abdestilliert. Man isoliert 14,8 g Verbindung 87.3.a in 75%iger Ausbeute. $T_{Siede}$ = 220 °C/ /2 Torr. $T_{Schmelz}$ = 61,9 °C (aus Hexan). Analog werden andere Verbindungen 87.3.b-e hergestellt.

| Substanz | Ausbeute in % | $T_{Siede}$ in °C/Torr | $T_{Schmelz}$ in °C |
|---|---|---|---|
| 87.3.b | 70 | 190/2 | 41,9 |
| 87.3.c | 71 | 193/2 | 71,3 |
| 87.3.d | 73 | 180/2 | 37,8 |
| 87.3.e | 75 | 225/2 | 53,5 |

87.4. 2-(trans-4-Hexylzyklohexyl)-5-zyanopyridin (XXIIa)

Eine Mischung aus 14,8 g (0,048 Mol) Verbindung 87.3.a, 1 g 10%igem Pd/C, 5,3 g wässerfreier Soda (0,05 Mol), 200 ml absolutem Äthylazetat wird bei Raumtemperatur und 1 atm Wasserstoffdruck hydriert, bis die berechnete Wasserstoffmenge (innerhalb von etwa 10 Stunden) aufgenommen wird. Das Reaktionsgut wird abfiltriert, das Lösungsmittel

im Vakuum abdestilliert und der Rückstand durch Chromatografieren in einer Säule mit Silikagel (Säulenhöhe 20 cm, Silikagel 40/100, Eluent Benzol-Hexan-Mischung bei einem Verhältnis von 1:2) gereinigt. Man isoliert 7 g Verbindung XXII.a in 54%iger Ausbeute. $T_{C-S}$ = 36,3 °C, $T_{S-N}$= = 41,5 °C, $T_{N-I}$ = 60,4 °C (aus Hexan). Analog werden andere Verbindungen der Formel XXII b-e hergestellt. Phasenübergangstemperaturen und Elementaranalyse der Verbindungen der Formel XXII b-f sind in der Tabelle 26 angegeben. Der Aufbau der Verbindungen wird durch 'H-NMR- und NMR-Spektren mit $^{13}C$ nachgewiesen. So weist das 'H-NMR-Spektrum ($CCl_4$) der Verbindungen der Formel XXII a ($\delta$, ppm) folgendes auf:
1,08-2,85 [23 H, Multiplett $CH_3$, $CH(CH_2)_9$]; 7,28 (1 H, Duplett, $J_{ortho}$ = 8 Hz, $H_3$); 7,92 (1 H Doppelduplett $J_{ortho}$ = 8 Hz, $H_4$); 8,8 (1 H, Singulett, $H_6$).

Tabelle 26

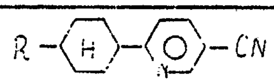 (XXVI)

| Nr. | R | $T_{C-N}$, C-S, in °C | $T_{S-N}$, in °C | $T_{N-I}$, in °C |
|---|---|---|---|---|
| 1 | 2 | 3 | 4 | 5 |
| b | $C_3H_7$ | 48,0 | - | 53,6 |
| c | $C_4H_9$ | 32,0 | - | 49,2 |
| d | $C_5H_{II}$ | 48,0 | - | 64,2 |
| e | $C_6H_{13}$ | 36,3 | 41,4 | 60,4 |
| f | $C_7H_{15}$ | 47,6 | 52,2 | 66,2 |

Fortsetzung der Tabelle 26

| Nr. | Gefunden in % | | Brutto-formel | Berechnet in % | |
|---|---|---|---|---|---|
| | C | H | | C | H |
| 1 | 6 | 7 | 8 | 9 | 10 |
| b | 78,96 | 8,75 | $C_{15}H_{20}N_2$ | 78,90 | 8,83 |
| c | 79,35 | 9,28 | $C_{16}H_{22}N_{22}$ | 79,29 | 9,15 |
| d | 79,51 | 9,46 | $C_{17}H_{24}N_2$ | 79,64 | 9,44 |
| e | 79,85 | 10,01 | $C_{18}H_{26}N_2$ | 79,95 | 9,69 |
| f | 80,15 | 9,69 | $C_{19}H_{28}N_2$ | 80,23 | 9,92 |

Zur Messung der elektrooptischen Parameter der bekannten und der neuen Verbindungen sind Mischungen dieser Stoffe zu verwenden, weil die einzelnen Verbindungen der bekannten Verbindungen und die Verbindungen der Formel XXII schmale nematische Bereiche aufweisen, welche zur Messung der elektrooptischen Parameter nicht ausreichen. Die Mischungen des dritten, fünften und sechsten Homologen liefern dabei einen breiten Temperaturenbereich der Mesophase und eignen sich also zu Vergleichszwecken. Es ist auch zu berücksichtigen, daß die Gesetzmäßigkeit der Änderungen von Eigenschaften bei Gliedern der homologen Reihe gewöhnlich mit dem ausreichenden Genauigkeitsgrad bekannt ist, d.h. nach den Eigenschaften der Mischung des dritten, fünften und sechsten Homologen kann man die Eigenschaften der Glieder der ganzen homologen Reihe beurteilen.

Es wurden deshalb folgende ternäre Mischungen hergestellt:

Mischung A: 30 Mol% 2-(trans-4-Propylzyklohexyl)-5-zyanopyridin, 40 Mol% 2-(trans-4-Pentylzyklohexyl)-5-zyanopyridin, 30 Mol% 2-(trans-4-Hexylzyklohexyl)-5-zyanopyridin

Mischung B: 30 Mol% 5-Propyl-2-(4-zyanophenyl)-pyridin. 40 Mol% 5-Pentyl-2-(4-zyanophenyl)-pyridin, 30 Mol% 5-Hexyl-2-(4-zyanophenyl)-pyridin

Mischung C: 30 Mol% 2-(4-Propylphenyl)-5-zyanopyridin, 40 Mol% 2-(4-Pentylphenyl)-5-zyanopyridin, 30 Mol% 2-(4-Hexylphenyl)-5-zyanopyridin.

Die Vergleichsdaten der Phasenübergangstemperaturen, Anisotropien der Dielektrizitätskonstante und elektrooptische Parameter von Mischungen A, B, C zeigt die Tabelle 27.

Tabelle 27. Eigenschaften der Mischungen von flüssigkristallinen Stoffen

| Mischung | $T_{C-N}$ in $^{o}C$ | $T_{N-I}$ in $^{o}C$ | $\gamma 1{,}25^{o}C$ P | $\Delta\varepsilon$ $\tau=0{,}95$ | $\Delta\varepsilon/\varepsilon_{\perp}$ $\tau=0{,}95$ |
|---|---|---|---|---|---|
| 1 | 2 | 3 | 4 | 5 | 6 |
| A | 11 | 57,3 | 1,40 | 3,3 | 0,44 |
| B | 7 | 40,4 | 1,92 | 17,1 | 1,70 |
| C | 15 | 62,7 | 3,50 | 7,3 | 0,85 |

Fortsetzung der Tabelle 27

| Mischung | Δn $\tau$=0,95 | $U_{Schwell}$ in V bei 25 °C | $U_{Sätt}$ in V bei 25°C | $U_{in}$ V | $\tau_{Aus}$ in ms bei 25°C | D $\mu$m |
|---|---|---|---|---|---|---|
| 1 | 7 | 8 | 9 | 10 | 11 | 12 |
| A | 0,104 | 2,50 | 3,45 | 5 | 43 | 10 |
| B | 0,180 | 1,12 | 1,58 | 5 | 57 | 10 |
| C | 0,207 | 1,90 | 2,63 | 5 | 60 | 10 |

Darin bedeuten:

$\tau = T_{Meß}$, °K/$T_{N-I}$ in °K

$\Delta\varepsilon = \varepsilon_{\parallel} = \varepsilon_{\perp}$ Anisotropie der Dielektrizitätskonstante

$\gamma_1$ Poise Koeffizient der Rotationsviskosität

Δn optische Anisotropie

$U_{Schwelle}$ in V Schwellespannung beim Twist-Effekt beim Durchlässigkeitsniveau von 0,9 (die Ausgangsdurchlässigkeit der Zelle beträgt 1,0)

$U_{Sätt}$ in V Sättigungsspannung beim Twist-Effekt beim Durchlässigkeitsniveau der Zelle von 0,1

U, in V Meßspannung

$\tau_{Aus}$ in ms Ausschaltzeit, d.h. die Zeit, die vom Abschalten der Spannung bis zum Erreichen des Durchlässigkeitsniveaus aus der Zelle von 0,9 dauert.

d in $\mu$m Dicke der Zelle

Die Tabelle 27 zeigt, daß der Koeffizient der Rotationsviskosität $\gamma_1$ = 1,40 P bei 25 °C für die Mischung der neuen Verbindungen (Mischung A) bedeutend kleiner als $\gamma_1$ für die Mischung B ( $\gamma_1$ = 1,92 P) und für die Mischung C ( $\gamma_1$= 3,50 P) ist.

In der Tabelle 28 sind Zusammensetzungen und Eigenschaften von flüssigkristallinen Materialien dargestellt, welche neue Pyridinderivate der Formel XXII enthalten.

Tabelle 28

| Nr. | Zusammensetzung des erfindungsgemäßen Materials | Masse% | $\gamma_1$ in P bei 25° | Bereich der nematischen Phase in °C |
|---|---|---|---|---|
| 1 | 2 | 3 | 4 | 5 |
| 88 | $C_3H_7$ 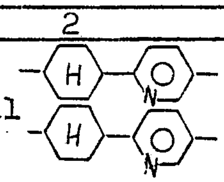 CN (XXII b)<br>$C_5H_{11}$ CN (XXII d) | 19<br>29 | 1,58 | -16-71,5 |

| 1 | 2 | 3 | 4 | 5 |
|---|---|---|---|---|
| | 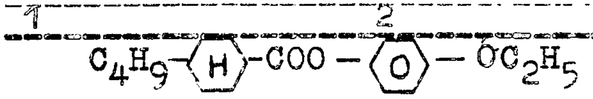 | 26 | | |
| | 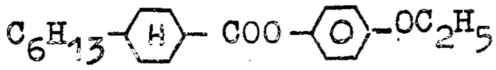 | 16 | | |
| | 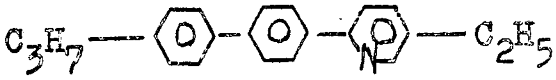 | 10 | | |
| 89. | 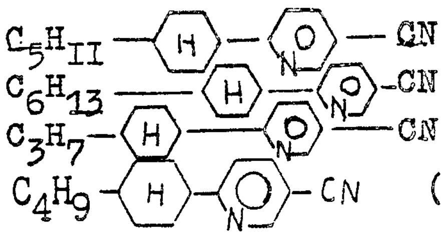 (XXII d) | 25 | 1,51 | -22÷61,4 |
| | (XXII a) | 15 | | |
| | (XXII b) | 15 | | |
| | (XXIIc) | I5 | | |
| | 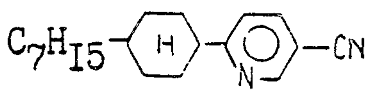 (XXIIe) | I5 | | |
| | 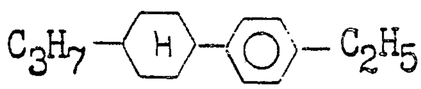 | I0 | | |
| | 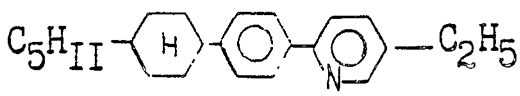 | I5 | | |
| 90. | 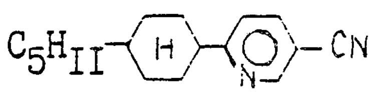 (XXIId) | I0 | I,83 | -I8-74,2 |
| | 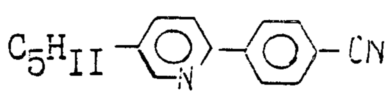 | 5 | | |
| | 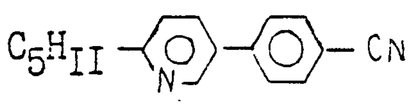 | 5 | | |
| | 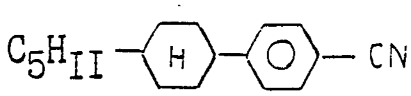 | 5 | | |
| | 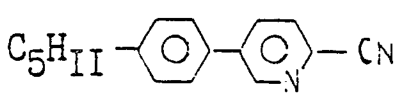 | 5 | | |
| | 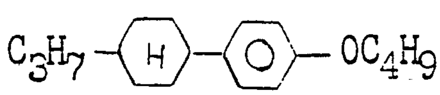 | I7 | | |
| | 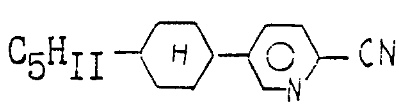 (II) | 5 | | |
| | 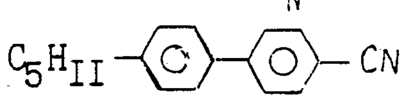 | 5 | | |
| | 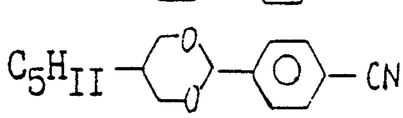 | 5 | | |
| | 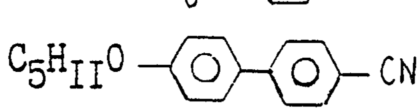 | 5 | | |
| | 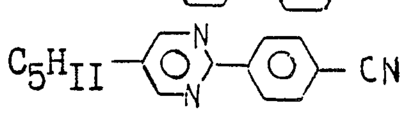 | 5 | | |

Fortsetzung der Tabelle 28

| I | 2 | 3 | 4 | 5 |
|---|---|---|---|---|
| 90. | 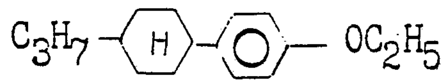 | I8 | | |
| | 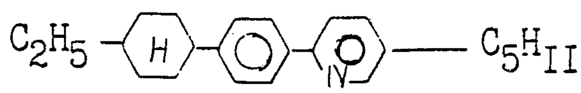 | I0 | | |
| 9I. | 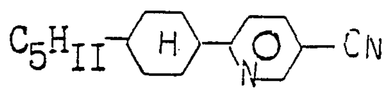 (XXΠd) | 20 | I,8I | -22-I03 |
| | 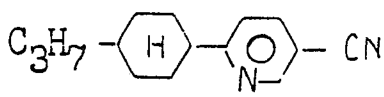 (XXΠβ) | I5 | | |
| | 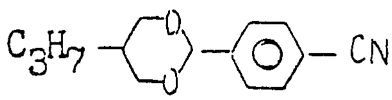 | I0 | | |
| | 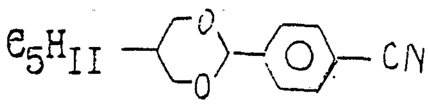 | I0 | | |
| | 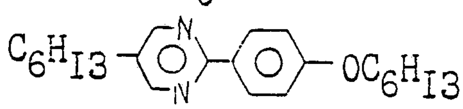 | 5 | | |
| | 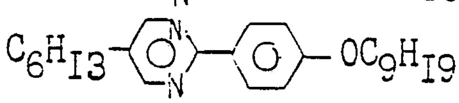 | 5 | | |
| | 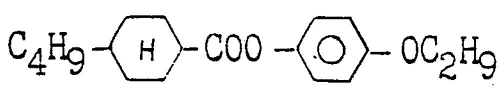 | I0 | | |
| | 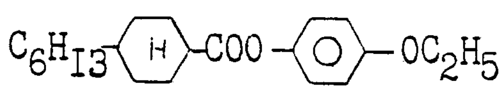 | 5 | | |
| | 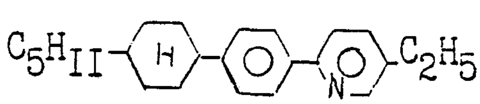 | 5 | | |
| | 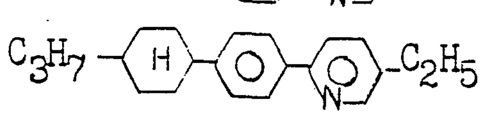 | 5 | | |
| | 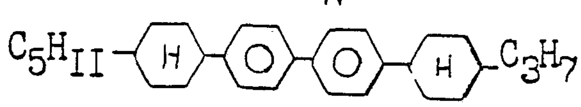 | I0 | | |
| 92. | 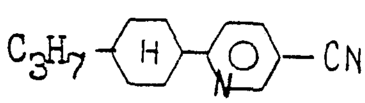 (XXΠβ) | I0 | I,73 | -I5-62,4 |
| | 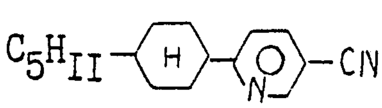 (XXΠ d) | I5 | | |

Fortsetzung der Tabelle 28

| I | 2 | | 3 | 4 | 5 |
|---|---|---|---|---|---|
| 92. | 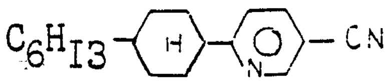 | (XXIIa) | I0 | | |
| | 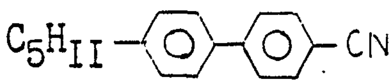 | | I5 | | |
| | 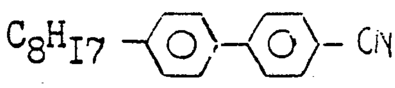 | | 6 | | |
| | 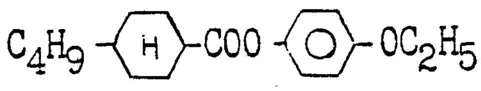 | | 25 | | |
| | 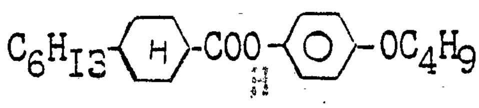 | | I0 | | |
| | 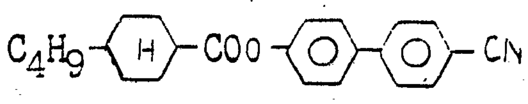 | | 9 | | |
| 93. | 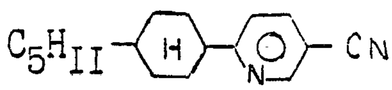 | (XXIId) | I7 | I,67 | -I5-90,7 |
| | 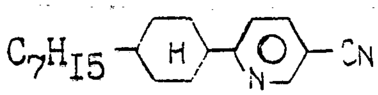 | (XXIIe) | 23 | | |
| | 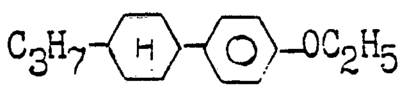 | | I6 | | |
| | 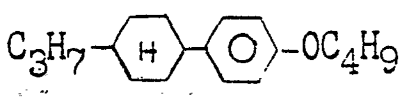 | | I2 | | |
| | 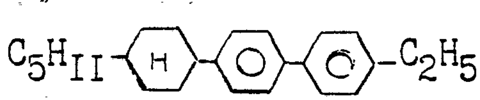 | | 22 | | |
| | 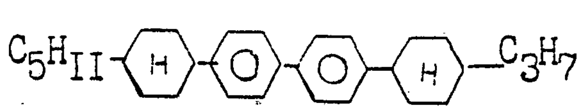 | | I0 | | |
| 94. | 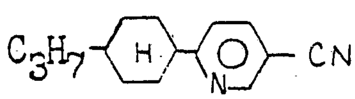 | (XXIIb) | 3 | 2,00 | -I8-80,8 |
| | 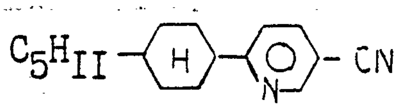 | (XXIId) | 4 | | |
| | 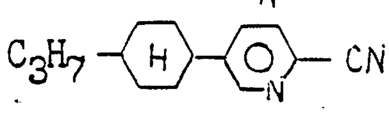 | (II) | I0 | | |

Fortsetzung der Tabelle 28

| I | 2 | | 3 | 4 | 5 |
|---|---|---|---|---|---|
| 94. | 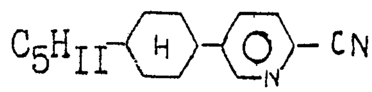 | (П) | I5 | | |
| | 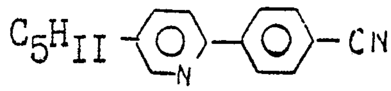 | | I0 | | |
| | 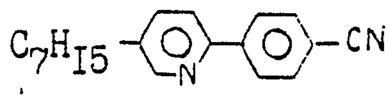 | | I5 | | |
| | 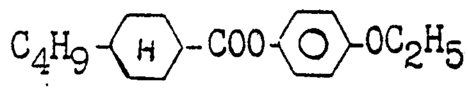 | | 20 | | |
| | 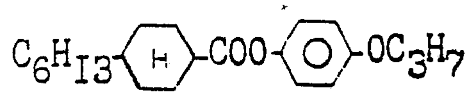 | | I3 | | |
| | 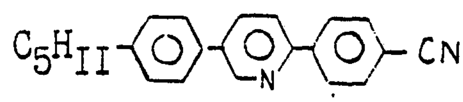 | | 5 | | |
| | 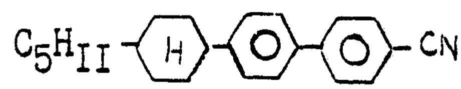 | | 5 | | |
| 95. | 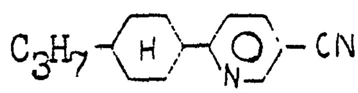 | (XXП*b*) | 20 | I,52 | -9-77 |
| | 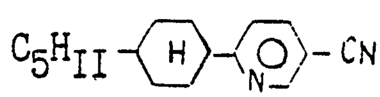 | (XXП*d*) | 20 | | |
| | 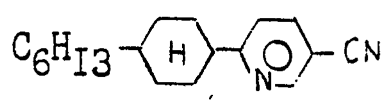 | (XXПa) | 20 | | |
| | 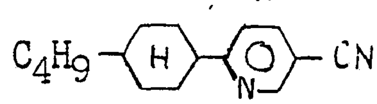 | | 20 | | |
| | 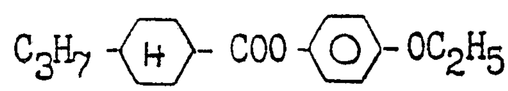 | | I0 | | |
| | 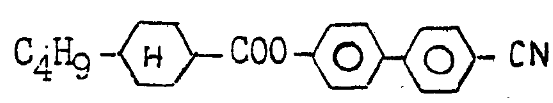 | | I0 | | |
| 96. | 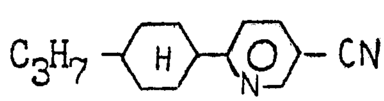 | | I0 | I,78 | -I6-87 |
| | 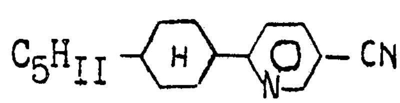 | | I5 | | |

Fortsetzung der Tabelle 28

| 1 | 2 | 3 | 4 | 5 |
|---|---|---|---|---|
| 96. | 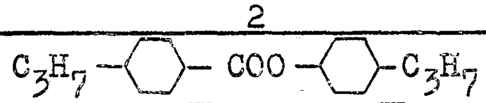 | 10 | | |
| | 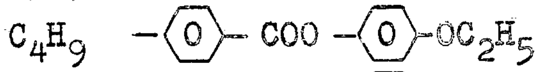 | 10 | | |
| | 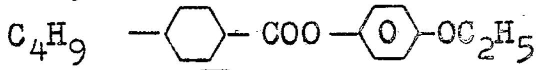 | 20 | | |
| | 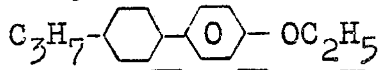 | 10 | | |
| | 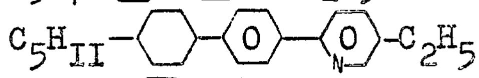 | 15 | | |
| | 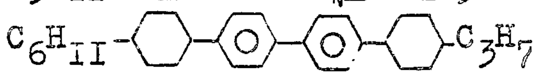 | 10 | | |

Aus der Tabelle 28 ist zu ersehen, daß die Verwendung der neuen Pyridinderivate der Formel XXII als Komponenten der flüssigkristallinen Materialien möglich macht, flüssigkristalline Materialien mit geringen Koeffizienten der Rotationsviskosität und breiten Temperaturenbereichen der nematischen Phase herzustellen, die sich zur Verwendung in schnellwirkenden elektrooptischen Einrichtungen eignen.

Beispiel 97. Herstellung von 2-(4-Alkoxy-3-fluorphenyl)-5-alkylpyridinen (XXIII)

97.1. 2-(4-Heptyloxy-3-fluorphenyl)-5-heptylpyridin (XXIII.a)

Erhalten durch Kondensation von Enamin mit entsprechendem Vinylketon unter anschließender Umsetzung von Pyran mit Hydroxylammoniumchlorid analog zu 33.4. Ausbeute 65%. $T_{C-S_A}$ = 28 °C, $T_{S_A-I}$ = 49,1 °C (aus Äthanol).

Gefunden in %: C 77,91; H 9,43. $C_{25}H_{36}NDF$.

Berechnet in %: C 77,88; H 9,41.

Analog werden andere Verbindungen der Formel XXIII hergestellt.

Industrielle Anwendbarkeit

Die erfindungsgemäßen Verbindungen weisen einen breiten Spektrumbereich der Eigenschaften auf, der es möglich macht, diese Verbindungen bei der Entwicklung von flüssigkeitsfallinen Materialien zu verwenden. Diese flüssigkristallinen Materialien können in verschiedenen elektrooptischen Einrichtungen mit statischer und dynamischer Arbeitsweise, beispielsweise in den Uhren, Mikrokalkulatoren, Fernseheabschirmungen, in den Einrichtungen mit der Sonnenbatteriespeisung sowie in den Einrichtungen der äußeren Anwendung u.a.m. ihre Anwendung finden.

0310676

# PATENTANSPRÜCHE

1. Flüssigkristalline Derivate von 2,5-disubstituiertem Pyridin der allgemeinen Formel 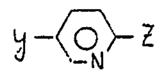

worin wenn Y 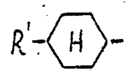 ist, Z für CN,

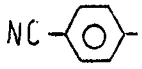, 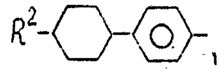, 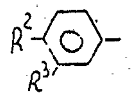, 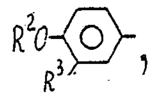, 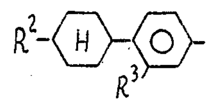, 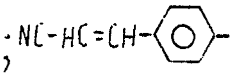

steht, wenn Y $R^1$, ist, Z für

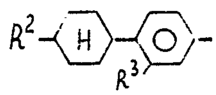, 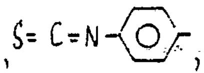, 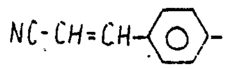, 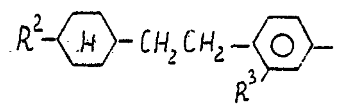

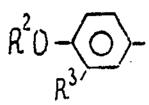, 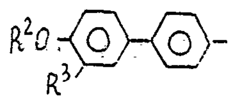, 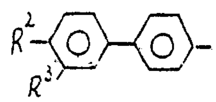, 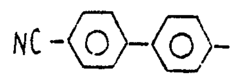

steht, wenn Y -CN ist, Z für 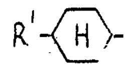 steht, wenn Y 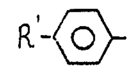 ist, Z für NC-CH= 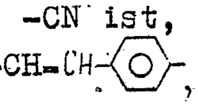, 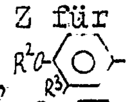 steht, wenn Y 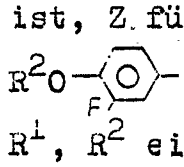 ist, Z für 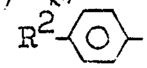 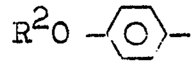 steht, $R^1$, $R^2$ eine Alkylgruppe mit 1 bis 12 Kohlenstoffatomen darstellen, $R^3$ für F, $NO_2$, $NH_2$ und CN steht.

2. Verbindung nach Anspruch 1 der Formel

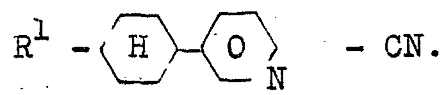

3. Verbindung nach Anspruch 1 der Formel

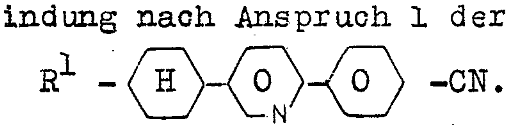

4. Verbindung nach Anspruch 1 der Formel

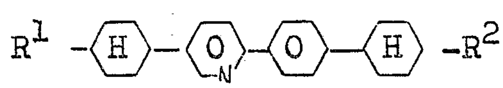

5. Verbindung nach Anspruch 1 der Formel

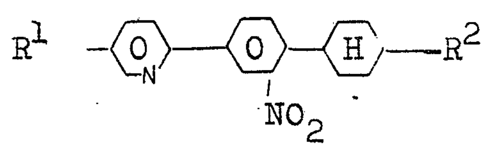

6. Verbindung nach Anspruch 1 der Formel

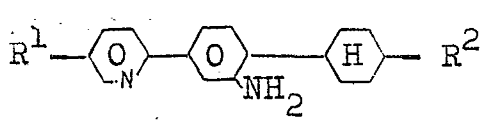

7. Verbindung nach Anspruch 1 der Formel

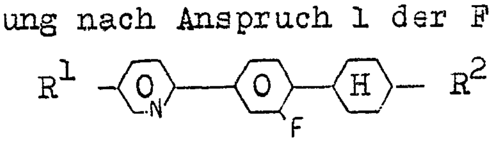

8. Verbindung nach Anspruch 1 der Formel

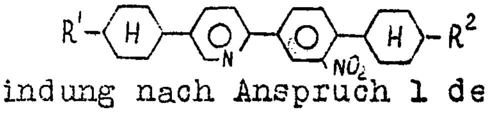

9. Verbindung nach Anspruch 1 der Formel

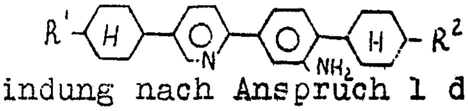

10. Verbindung nach Anspruch 1 der Formel

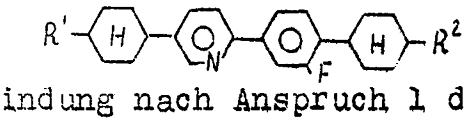

11. Verbindung nach Anspruch 1 der Formel

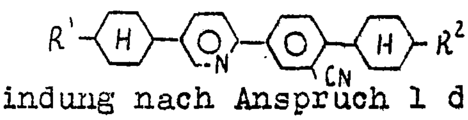

12. Verbindung nach Anspruch 1 der Formel

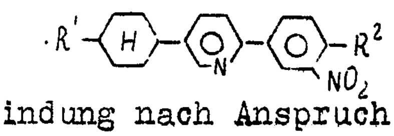

13. Verbindung nach Anspruch 1 der Formel

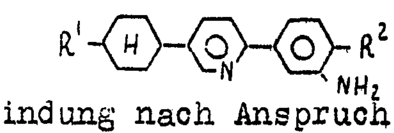

14. Verbindung nach Anspruch 1 der Formel

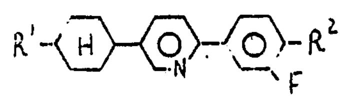

15. Verbindung nach Anspruch 1 der Formel

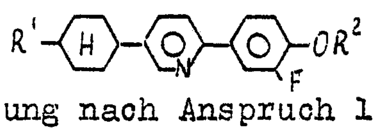

16. Verbindung nach Anspruch 1, der Formel

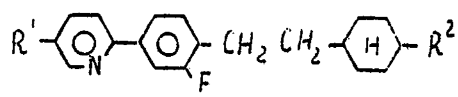

17. Verbindung nach Anspruch 1 der Formel

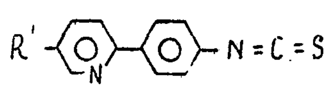

18. Verbindung nach Anspruch 1 der Formel

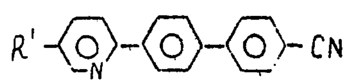

19. Verbindung nach Anspruch 1 der Formel

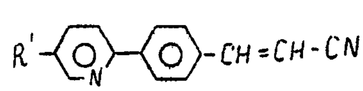

20. Verbindung nach Anspruch 1 der Formel

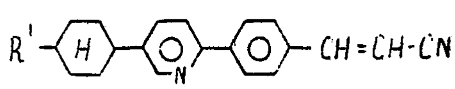

21. Verbindung nach Anspruch 1 der Formel

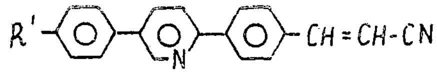

22. Verbindung nach Anspruch 1 der Formel

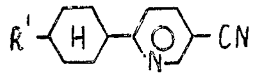

23. Verbindung nach Anspruch 1 der Formel

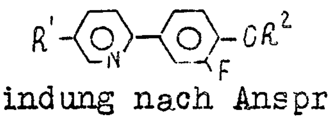

24. Verbindung nach Anspruch 1 der Formel

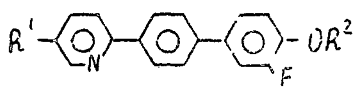

25. Verbindung nach Anspruch 1 der Formel

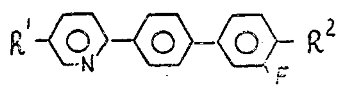

26. Verbindung nach Anspruch 1 der Formel

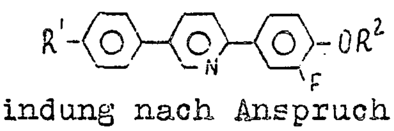

27. Verbindung nach Anspruch 1 der Formel

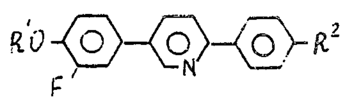

28. Verbindung nach Anspruch 1 der Formel

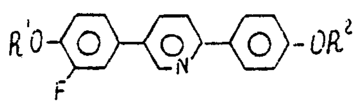

29. Flüssigkristallines Material, enthaltend zumindest zwei flüssigkristalline Komponenten, d a d u r c h g e k e n n z e i c h n e t, daß mindestens eine der genannten flüssigkristallinen Komponenten ein flüssigkristallines Derivat von 2,5-disubstituiertem Pyridin nach Anspruch 1 dient.

30. Flüssigkristallines Material nach Anspruch 1, d a d u r c h g e k e n n z e i c h n e t, daß es das genannte flüssigkristalline Derivat von 2,5-disubstituiertem Pyridin in einer Menge von 1 bis 75 Masse% enthält.

# INTERNATIONAL SEARCH REPORT

0310676

International Application No PCT

**I. CLASSIFICATION OF SUBJECT MATTER** (if several classification symbols apply, indicate all) [6]

According to International Patent Classification (IPC) or to both National Classification and IPC

IPC 4 C 07 D 213/57, 213/16, 213/24, C 09 K 19/34, 19/42

**II. FIELDS SEARCHED**

Minimum Documentation Searched [7]

| Classification System | Classification Symbols |
|---|---|
| IPC 4 | C 07 D 213/16, 213/24÷ 213/57, C 09 K 19/34, 19/42 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | EP, A2, 0240320, (CHISSO CORPORATION), 7 October 1987(07.10.87), see the abstract | 1,10,14,15 |
| X,P | EP, A2, 0240320, (CHISSO CORPORATION), 7 October 1987(07.10.87), see the abstract | 4 |
| X | DE, A1, 3600052, (MERCK PATENT GmbH), 9 July 1987(09.07.87), see column 1, lines 1-37 | 1,2,3,11,18 |
| X | US, A, 4684477, (CHISSO CORPORATION), 4 August 1987(04.08.87), see the abstract | 1,22 |
| A | EP, A2, 0164721, (CHISSO CORPORATION), 18 December 1985(18.12.85), see claim 6 | 29,30 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 23 May 1988(23.05.88) | 1 July 1988(01.07.88) |
| International Searching Authority | Signature of Authorized Officer |
| ISA/SU | |